(19) Europäisches Patentamt
European Patent Office
Office européen des brevets

(11) EP 4 342 878 A1

(12) EUROPEAN PATENT APPLICATION
published in accordance with Art. 153(4) EPC

(43) Date of publication:
**27.03.2024 Bulletin 2024/13**

(21) Application number: **22804016.8**

(22) Date of filing: **18.05.2022**

(51) International Patent Classification (IPC):
***C07C 211/29*** (2006.01) ***A61K 31/44*** (2006.01)
***A61P 35/00*** (2006.01)

(52) Cooperative Patent Classification (CPC):
**A61K 31/44; A61P 35/00; C07C 211/29**

(86) International application number:
**PCT/CN2022/093688**

(87) International publication number:
**WO 2022/242696 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2021 CN 202110560615**
**30.07.2021 CN 202110875453**

(71) Applicant: **Cytosinlab Therapeutics Co., Ltd.**
**Shanghai 201203 (CN)**

(72) Inventors:
- **FU, Xingnian**
**Shanghai 201203 (CN)**
- **WU, Haiping**
**Shanghai 201203 (CN)**
- **WANG, Meng**
**Shanghai 201203 (CN)**
- **MI, Yuan**
**Shanghai 201203 (CN)**
- **SHI, Hui**
**Shanghai 201203 (CN)**
- **GUO, Jiannan**
**Shanghai 201203 (CN)**

(74) Representative: **Lavoix**
**Bayerstraße 83**
**80335 München (DE)**

# (54) ARGININE METHYLTRANSFERASE INHIBITOR AND USE THEREOF

(57) Provided are an arginine methyltransferase inhibitor and a use thereof. Specifically, provided are a compound that can be used as an I-type PRMT inhibitor, a preparation method therefor, and a use thereof in the treatment of related diseases. The structure of said compound is as represented by formula (I).

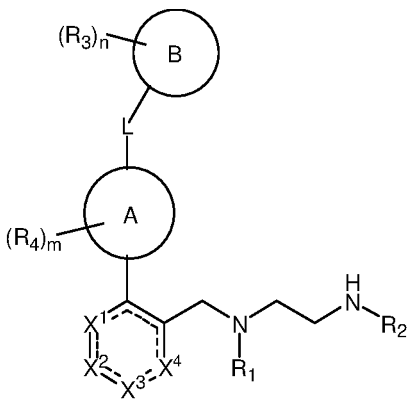

I

EP 4 342 878 A1

## Description

### Technical field

The invention relates to the field of pharmaceutical chemistry and medicine, in particular to a class of type I PRMT inhibitor compound, preparation method therefor and its application in the field of disease treatment.

### Background

Protein arginine methylation is a highly-abundant post-translational modification that exists widely in the cytoplasm and nucleus. The protein arginine methyltransferases (PRMTs) family is involved in the process of protein arginine methylation, which mainly uses S-adenosylmethionine (SAM) as a methyl donor to methylate the nitrogen atom of the protein arginine side chain to generate S-adenosyl-L-homocysteine and methylated arginine. The PRMTs family contains 9 kinds of PRMT isoforms. According to the type of catalytic reaction, PRMTs can be divided into type I (PRMT1\PRMT2\PRMT3\PRMT4\PRMT6\PRMT8), type II (PRMT5\PRMT9) and type III (PRMT7). Type I PRMT is responsible for asymmetric dimethylated arginine (ADMA), type II PRMT is responsible for symmetric dimethylated arginine (SDMA), and type III PRMT is responsible for monomethylated arginine (MMA).

At present, many documents have confirmed that the abnormal expression of type I PRMT is closely related to the occurrence and development of various diseases. For example, PRMT1 has been found to play an oncogenic role in leukemia, lung cancer, liver cancer, gastric cancer, colon cancer, breast cancer, pancreatic cancer, head and neck cancer, prostate cancer, bladder cancer, ect. In malignant glioma, PRMT2 was found to be over-expressed, and this was closely related to poor prognosis. In 70% of acute myeloid leukemia (AML) patients, at least a two-fold over-expression of PRMT4 was observed. PRMT6 was found to be over-expressed in 52.6% of gastric cancer cells, and its expression had a significant positive correlation with the modification level of its substrate. Meanwhile, type I PRMT is mainly responsible for catalyzing the asymmetric dimethylation of arginine, and the changed ADMA levels in vivo are closely related to cardiovascular diseases, diabetes, renal failure, asthma and chronic non-obstructive diseases. Therefore, it can be said that the abnormal expression of type I PRMT is related to the occurrence and development of various diseases. In summary, it is of great significance to develop new PRMT inhibitor molecules.

### SUMMARY

The purpose of the present invention is to provide novel PRMT inhibitor molecules.

The first aspect of the present invention provides a compound represented by the following formula I, or a pharmaceutically acceptable salt or deuterated compound thereof:

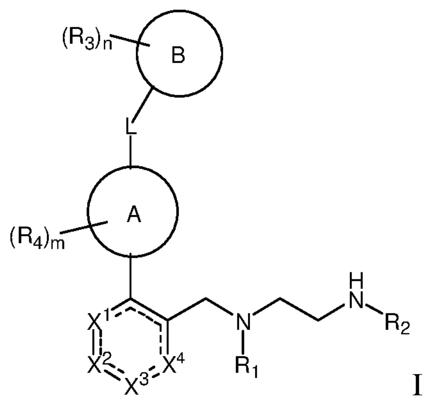

I

$X^1$, $X^2$, $X^3$ and $X^4$ are each independently selected from the group consisting of: CR, NR and N; the dash line is a chemical bond or absent;

ring A is selected from the group consisting of substituted or unsubstituted saturated or partially unsaturated (non-aromatic) 4-8 membered carbocycle, and substituted or unsubstituted saturated or partially unsaturated (non-aromatic) 4-8 membered heterocycle;

ring B is selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_6$-$C_{10}$aryl, substituted or unsubstituted 5-12 membered heteroaromatic ring, substituted or unsubstituted 3-12 membered heterocycle containing 1-3 heteroatoms selected from oxygen, sulfur, and nitrogen, sub-

stituted or unsubstituted -$C_1$-$C_6$alkyl-6-10 membered aryl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$ amide, substituted or unsubstituted $C_1$-$C_4$ alkyl-$S(O)_2$,and substituted or unsubstituted $C_1$-$C_4$alkyl-SO-;

m and n are each independently selected from the group consisting of 0, 1, 2, 3, 4, 5 and 6;

L is selected from the group consisting of chemical bonds, -O-, -$(CHR^6)_p$-, -$CHR^6$-O-, -$CHR^6$-C(O)-, carbonyl, S, -NH-, -NHC(O)-, -$NHS(O)_2$-, -NHC(O)NH-, -NHC(S)NH-, -COO-, -O-$S(O)_2$-, -COO-$CH_2$-, -C(O)$CH_2$-, and -$S(O)_2$-, or L is absent;

p is selected from the group consisting of 1, 2 and 3;

$R^1$ and $R^2$ are each independently selected from the group consisting of H, substituted or unsubstituted $C_1$-$C_6$ alkyl, and substituted or unsubstituted $C_3$-$C_6$cycloalkyl;

$R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_2$-$C_6$alkenyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-12 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$amide, substituted or unsubstituted $C_1$-$C_4$alkyl-$S(O)_2$-, and substituted or unsubstituted $C_1$-$C_4$alkyl-SO-;

R is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-12 membered heteroaryl, substituted or unsubstituted 4-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$amide, substituted or unsubstituted $C_1$-$C_4$alkyl-$S(O)_2$-, and substituted or unsubstituted $C_1$-$C_4$alkyl-SO-;

or two R located on adjacent ring atoms together with the ring atoms to which they are attached form a substituted or unsubstituted 5-11 membered carbocycle or heterocycle, and the ring is partially unsaturated or saturated; preferably, the carbocycle or heterocycle is a 5-9 membered carbocycle or heterocycle, more preferably a 5-7 membered carbocycle or heterocycle (the carbocycle or heterocycle is saturated, partially unsaturated or aromatic);

$R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_2$-$C_6$alkenyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-12 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$ amide, substituted or unsubstituted $C_1$-$C_4$alkyl-$S(O)_2$-, and substituted or unsubstituted $C_1$-$C_4$alkyl-SO-;

or two $R^4$ on the same or adjacent ring atoms together with the ring atoms to which they attached form a substituted or unsubstituted 3-11 membered carbocycle or heterocycle, and the ring is a partially unsaturated ring, saturated ring or aromatic ring;

$R^6$ is a group selected from the group consisting of H, halogen, cyano, amino, nitro, hydroxyl, thiol, aldehyde group, carboxyl, acyl, sulfonyl, substituted or unsubstituted $C_1$-$C_6$alkyl, substituted or unsubstituted $C_1$-$C_6$alkoxy, and substituted or unsubstituted $C_1$-$C_6$alkylamino;

unless otherwise specified, in the above formulas, the "substituted" means that the hydrogen atom on the corresponding group is substituted by one or more substituents selected from the group consisting of: deuterium, tritium, halogen, hydroxyl, carboxyl, thiol, benzyl , oxo (=O), $C_1$-$C_{12}$alkoxycarbonyl, $C_1$-$C_6$aldehyde group, amino, $C_1$-$C_6$ amide, nitro, cyano, unsubstituted or halogenated $C_1$-$C_6$alkyl, $C_2$-$C_{10}$alkenyl, $C_1$-$C_6$alkoxy, $C_1$-$C_6$alkyl-amino, $C_1$-$C_6$alkyl-sulfonamide, $C_1$-$C_6$alkyl-ureido, $C_1$-$C_6$alkyl-S-, $C_6$-$C_{10}$aryl, five- or six-membered heteroaryl, five- or six-membered non-aromatic heterocyclyl, five- or six-membered non-aromatic heterocyclyl-($CH_2$)-, -O-($C_6$-$C_{10}$aryl), -O-(five or six membered heteroaryl), -NH-($C_6$-$C_{10}$aryl), -NH-(five-membered or six-membered heteroaryl), $C_1$-$C_{12}$alkylaminocarbonyl, unsubstituted or halogenated $C_2$-$C_{10}$acyl, sulfonyl (-$SO_2$-OH), sulfonamide (-SO2-NH2 ), phosphoryl (-$PO_3$-OH), unsubstituted or halogenated $C_1$-$C_4$alkyl-$S(O)_2$-, unsubstituted or halogenated $C_1$-$C_4$alkyl-SO-,

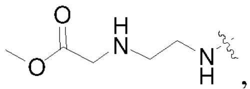, 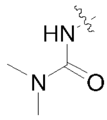,

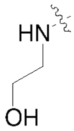, 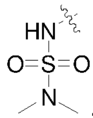, 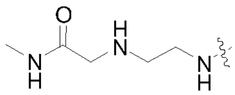, 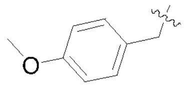,

and

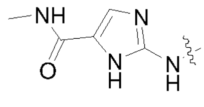;

in each formula, the heterocycle or heteroaromatic ring has 1-3 heteroatoms selected from the group consisting of N, S and O; each aryl, heteroaryl, and heterocyclyl can be independently substituted by 1-3 substituents selected from the group consisting of deuterium, tritium, halogen, hydroxyl, carboxyl, thiol, $C_1$-$C_6$alkyl, and $C_1$-$C_6$alkoxy.

In another preferred embodiment, the aromatic ring, aromatic-ring or aromatic system includes conventional tautomers of aromatic rings, such as pyridone, benzimidazole, benzopyrazole and the like.

In another preferred embodiment, at least one of $X^1$, $X^2$ $X^3$ and $X^4$ is N.

In another preferred embodiment, the ring

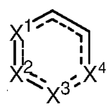

is an aromatic ring.

In another preferred embodiment, two R located on adjacent ring atoms together with the ring atoms to which they are attached form a substituted or unsubstituted 5-11 membered carbocycle or heterocycle, this ring and the ring

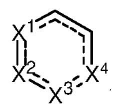

together form an aromatic ring system.

In another preferred embodiment, when ring B is H, said m is 2, 3, 4, 5 or 6; and at least two $R^4$ on the same or adjacent ring atoms together with the ring atoms to which they are attached form a substituted or unsubstituted 5-11 membered carbocycle or heterocycle (the ring is a partially unsaturated ring, saturated ring or aromatic ring).

In another preferred embodiment, L is selected from the group consisting of: chemical bond, -O-, $-(CHR^6)_p-$, $-CHR^6$-O-, $-CHR^6$-C(O)-, carbonyl, S, -NH-, -NHC(O)-, -COO-, $-COO-CH_2-$, $-C(O)CH_2-$, and $-S(O)_2-$, or L is absent.

In another preferred embodiment, $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-10 membered heteroaryl, substituted or unsubstituted 5-7 membered heterocycle containing 1-3 heteroatoms selected from oxygen, sulfur and nitrogen, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_6$-$C_{10}$aryloxy, substituted or unsubstituted $C_1$-$C_6$amide, substituted or unsubstituted $-C_1$-$C_6$alkyl-phenyl, substituted or unsubstituted $C_1$-$C_4$alkyl-$S(O)_2$-, and substituted or unsubstituted $C_1$-$C_4$alkyl-SO-; and/or

R is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted

or unsubstituted $C_1$-$C_6$alkylamino, substituted or unsubstituted $C_6$-$C_{10}$aryl, substituted or unsubstituted 5-10 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_6$-$C_{10}$aryloxy, substituted or unsubstituted $C_1$-$C_6$ amide, substituted or unsubstituted -$C_1$-$C_6$alkyl-phenyl, substituted or unsubstituted $C_1$-$C_4$alkyl-S(O)$_2$-, and substituted or unsubstituted $C_1$-$C_4$alkyl-SO-; or two R located on adjacent ring atoms together with the carbon atoms to which they attached form a substituted or unsubstituted 5-9 membered carbocycle or heterocycle, and the ring is partially unsaturated, saturated or aromatic; and/or

In another preferred embodiment, $R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-7 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_8$carbocycle, substituted or unsubstituted $C_2$-$C_6$acyl, substituted or unsubstituted $C_2$-$C_{10}$ester group, substituted or unsubstituted $C_6$-$C_{10}$aryloxy, substituted or unsubstituted $C_1$-$C_6$ amide, substituted or unsubstituted -$C_1$-$C_6$alkyl-phenyl, substituted or unsubstituted $C_1$-$C_4$alkyl-S(O)$_2$-, and substituted or unsubstituted $C_1$-$C_4$alkyl-SO-; or two $R^4$ located on adjacent ring atoms together with the carbon atoms to which they are attached form a substituted or unsubstituted 5-9 membered carbocycle or heterocycle, and the ring is partially unsaturated or saturated.

In another preferred embodiment, ring B is selected from the group consisting of: H, or substituted or unsubstituted phenyl, substituted or unsubstituted 5-10 membered heteroaromatic ring, substituted or unsubstituted 3-12 membered heterocycle containing 1-3 heteroatoms selected from oxygen, sulfur and nitrogen, and substituted or unsubstituted $C_3$ -$C_{12}$carbocycle.

In another preferred embodiment, $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-10 membered heteroaryl, substituted or unsubstituted 5-7 membered heterocycle containing 1-3 heteroatoms selected from oxygen, sulfur and nitrogen, substituted or unsubstituted $C_2$-$C_6$acyl, substituted or unsubstituted $C_2$-$C_6$ester group, substituted or unsubstituted $C_1$-$C_6$ amide, substituted or unsubstituted $C_1$-$C_4$alkyl-S(O)$_2$-, and substituted or unsubstituted $C_1$-$C_4$alkyl-SO-; and/or

R is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_1$-$C_6$ amide, substituted or unsubstituted $C_1$-$C_4$alkyl-S(O)$_2$-, and substituted or unsubstituted $C_1$-$C_4$alkyl-SO-; or two R located on adjacent ring atoms together with the ring atoms to which they are attached form a substituted or unsubstituted 5-7 membered carbocycle or heterocycle; and/or

$R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_3$-$C_8$carbocycle, substituted or unsubstituted $C_2$-$C_6$acyl, substituted or unsubstituted $C_2$-$C_{10}$ester group, substituted or unsubstituted $C_1$-$C_6$ amide, substituted or unsubstituted $C_1$-$C_4$alkyl-S(O)$_2$-, and substituted or unsubstituted $C_1$-$C_4$alkyl-SO-; or two $R^4$ on adjacent ring atoms together with the carbon atoms to which they are attached form a substituted or unsubstituted 5-9 membered carbocycle or heterocycle, and the ring is partially unsaturated or saturated.

In another preferred example, ring

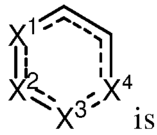 is 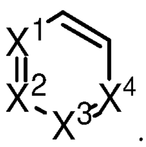.

In another preferred embodiment, ring

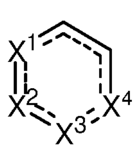

is selected from the group consisting of:

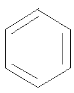, 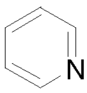, 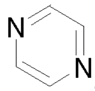, 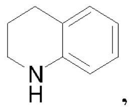, 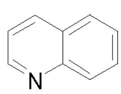, 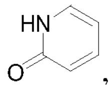, 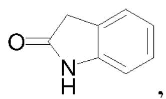, 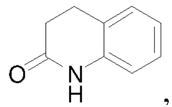, 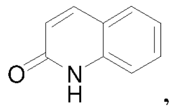,

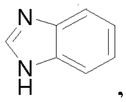, 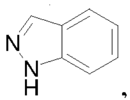, 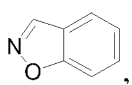, 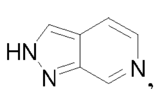, 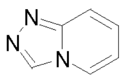, 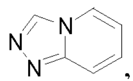, 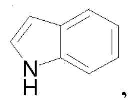, 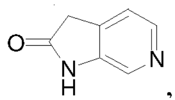,

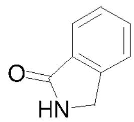, 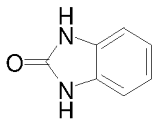, 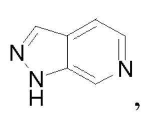, 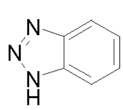,

and

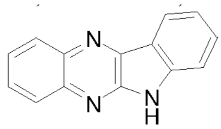.

In another preferred embodiment, ring B is selected from the group consisting of substituted or unsubstituted $C_6$-$C_{10}$aryl, substituted or unsubstituted 5-12 membered heteroaryl, substituted or unsubstituted 4-12 membered heterocycle containing 1-3 heteroatoms selected from oxygen, sulfur and nitrogen, and substituted or unsubstituted $C_3$-$C_{12}$carbocycle.

In another preferred embodiment, ring A is selected from the group consisting of:

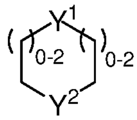,

wherein, $Y^1$ and $Y^2$ are each independently selected from the group consisting of: $CHR^6$ and $NR^6$; 0-2 means that the number of carbon atoms can be 0, 1 or 2; and the L-B structure can be located at $Y^1$ , $Y^2$ or other ring atoms (preferably at $Y^1$ , $Y^2$);

or ring A together with two $R^4$form a group selected from the group consisting of:

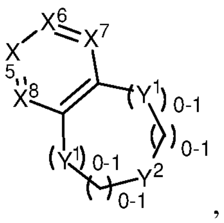,

wherein, $Y^1$ and $Y^2$ are each independently selected from the group consisting of: $CHR^6$, O and $NR^6$; $X^5$, $X^6$, $X^7$ and $X^8$ are each independently selected from the group consisting of: $CR^6$ and N.

In another preferred embodiment, ring A is a substituted or unsubstituted group selected from the group consisting of:

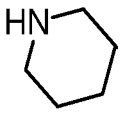, 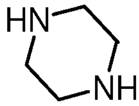, 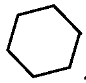, 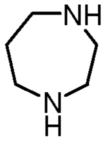, 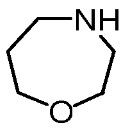

and

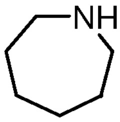

(wherein, when the connection site connected to other structural fragments or substituents is NH, the hydrogen atom on NH is lost to form the connection site);

or ring A together with two $R^4$ form a substituted or unsubstituted group selected from the group consisting of:

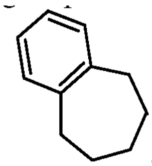, 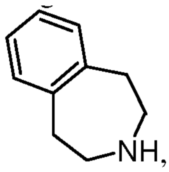, 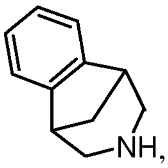, 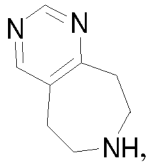, 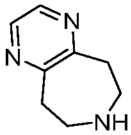, 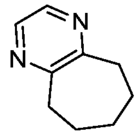, 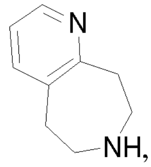,

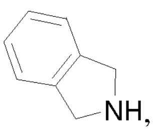, 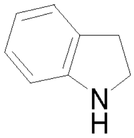, 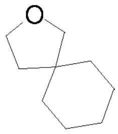, 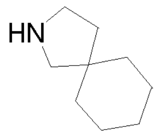, 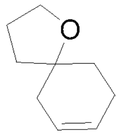, 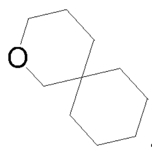, 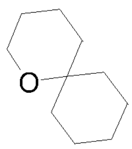, 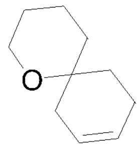,

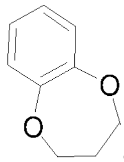, 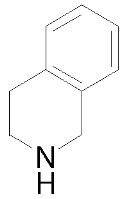, 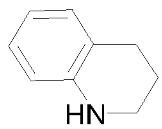

and

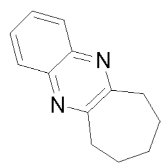

wherein, when the connection site with other structural fragments or substituents is NH, the hydrogen atom on NH is lost to form the connection site).

In another preferred embodiment, the substituents on ring A are groups selected from the group consisting of: H, halogen, cyano, amino, nitro, carbonyl, hydroxyl, thiol, aldehyde group, carboxyl, sulfonyl, substituted or unsubstituted $C_1$-$C_6$alkyl, substituted or unsubstituted $C_1$-$C_6$alkylamino, substituted or unsubstituted $C_3$-$C_8$carbocycle, substituted or unsubstituted $C_2$-$C_6$acyl, substituted or unsubstituted $C_2$-$C_{10}$ester group, substituted or unsubstituted $C_1$-$C_6$ amide, and substituted or unsubstituted $C_1$-$C_4$alkyl-$S(O)_2$-.

In another preferred embodiment, the compound has a structure shown in the following formula II:

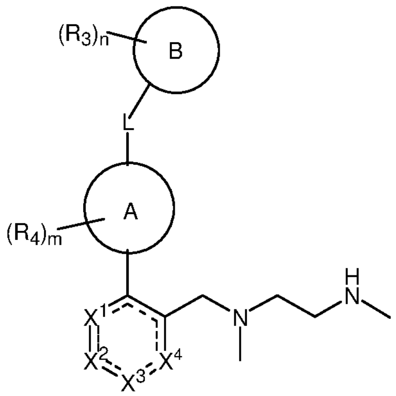

II

In another preferred embodiment, the compound has a structure shown in the following formula III:

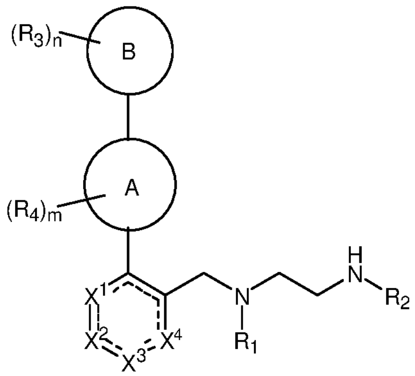

III

wherein,

ring A is selected from the group consisting of substituted or unsubstituted saturated or partially unsaturated (non-aromatic) 4-8 membered carbocycle, and substituted or unsubstituted saturated or partially unsaturated (non-aromatic) 4-8 membered heterocycle;

ring B is selected from the group consisting of substituted or unsubstituted $C_6$-$C_{10}$aryl, substituted or unsubstituted 5-12 membered heteroaryl, substituted or unsubstituted 3-12 membered heterocycle containing 1-3 heteroatoms selected from oxygen, sulfur and nitrogen, and substituted or unsubstituted $C_3$-$C_{12}$heterocycle;

preferably, $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_1$-$C_4$alkyl-$S(O)_2$-, and substituted or unsubstituted $C_1$-$C_4$alkyl-SO-;

preferably, $R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-7 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_1$-$C_4$alkyl-$S(O)_2$-, and substituted or unsubstituted $C_1$-$C_4$alkyl-SO-.

In another preferred embodiment, the compound has a structure shown in the following formula IV:

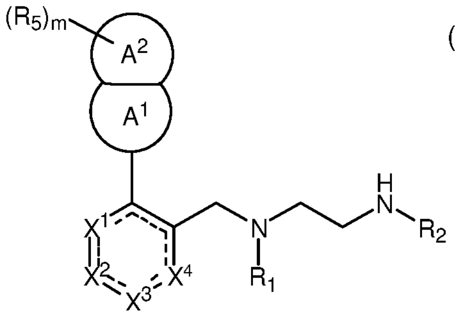

IV

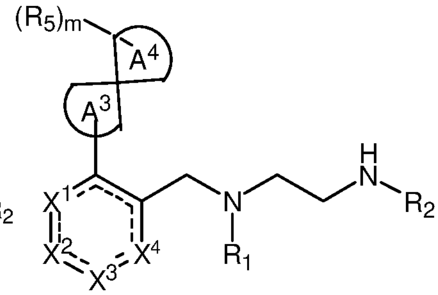

IVA

wherein,

ring $A^1$ is selected from the group consisting of substituted or unsubstituted saturated or partially unsaturated (non-aromatic) 5-8 membered carbocycle, and substituted or unsubstituted saturated or partially unsaturated (non-aromatic) 5-8 membered heterocycle;

ring $A^2$ is selected from the group consisting of substituted or unsubstituted $C_6$-$C_{10}$aryl, substituted or unsubstituted 5-12 membered heteroaryl, substituted or unsubstituted 5-7 membered heterocycle containing 1-3 heteroatoms selected from oxygen, sulfur and nitrogen, and substituted or unsubstituted $C_3$-$C_{12}$carbocycle; and ring $A^2$is fused with ring $A^1$;

ring $A^3$and ring $A^4$are each independently selected from the group consisting of substituted or unsubstituted saturated or partially unsaturated (non-aromatic) 5-8 membered carbocycle, and substituted or unsubstituted saturated or partially unsaturated (non-aromatic) 3-8 membered heterocycle;

m is 0, 1, 2, 3 or 4; $R^5$is selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_6$-$C_{10}$aryloxy or heteroaryloxy,substituted or unsubstituted $C_6$-$C_{10}$arylamino or heteroarylamino, substituted or unsubstituted $C_1$-$C_6$ amide, substituted or unsubstituted -$C_1$-$C_6$alkyl-phenyl, substituted or unsubstituted $C_1$-$C_4$alkyl-$S(O)_2$-, and substituted or unsubstituted $C_1$-$C_4$alkyl-SO-; and $R^5$ can be located on ring $A^1$, ring $A^2$, ring $A^3$or ring $A^4$.

In another preferred embodiment, the compound has a structure shown in the following formula V or VI:

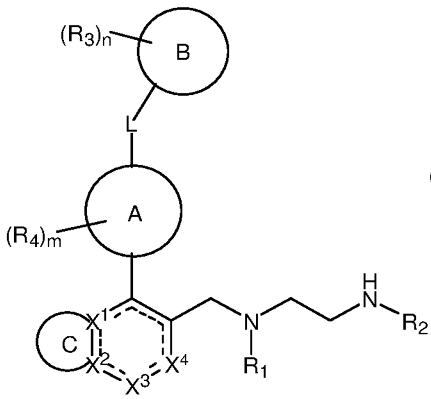

V

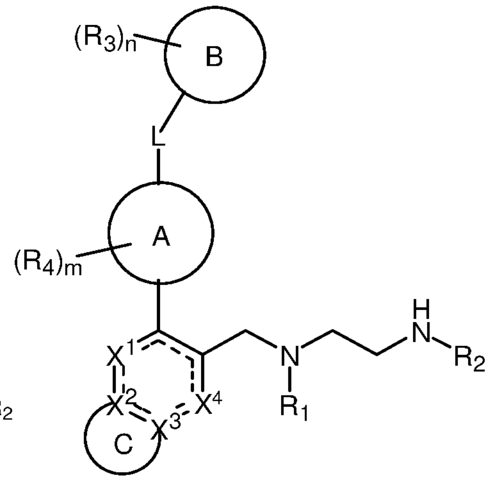

VI

wherein, ring C is substituted or unsubstituted phenyl, substituted or unsubstituted 5-7 membered heterocyclyl, or substituted or unsubstituted 5-6 membered heteroaryl;

ring A is selected from the group consisting of substituted or unsubstituted saturated or partially unsaturated (non-aromatic) 5-6 membered carbocycle, and substituted or unsubstituted saturated or partially unsaturated (non-aromatic) 4-8 membered heterocycle;

ring B is selected from the group consisting of substituted or unsubstituted $C_6$-$C_{10}$aryl, substituted or unsubstituted 5-12 membered heteroaryl, substituted or unsubstituted 4-12 membered heterocycle containing 1-3 heteroatoms selected from oxygen, sulfur and nitrogen, and substituted or unsubstituted $C_3$-$C_{12}$carbocycle;

preferably, $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_1$-$C_4$alkyl-$S(O)_2$-, and substituted or unsubstituted $C_1$-$C_4$alkyl-SO-;

preferably, $R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-7 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_1$-$C_4$alkyl-$S(O)_2$-, and substituted or unsubstituted $C_1$-$C_4$alkyl-SO-.

In another preferred embodiment, ring C is selected from the group consisting of benzene ring, 5-7 membered heteroaromatic ring, and 5-7 membered saturated or partially unsaturated heterocycle.

In another preferred embodiment, ring A is selected from the group consisting of substituted or unsubstituted saturated or partially unsaturated (non-aromatic) 5-6 membered carbocycle, and substituted or unsubstituted saturated or partially unsaturated (non-aromatic) 5-7 membered heterocycle.

In another preferred embodiment, ring B is selected from the group consisting of substituted or unsubstituted $C_6$-$C_{10}$aryl, substituted or unsubstituted 5-7 membered heteroaryl, substituted or unsubstituted 4-12 membered heterocycle containing 1-3 heteroatoms selected from oxygen, sulfur and nitrogen, and substituted or unsubstituted $C_3$-$C_{12}$carbocycle.

In another preferred embodiment, the compound of Formula I has a structure as shown in the following formula:

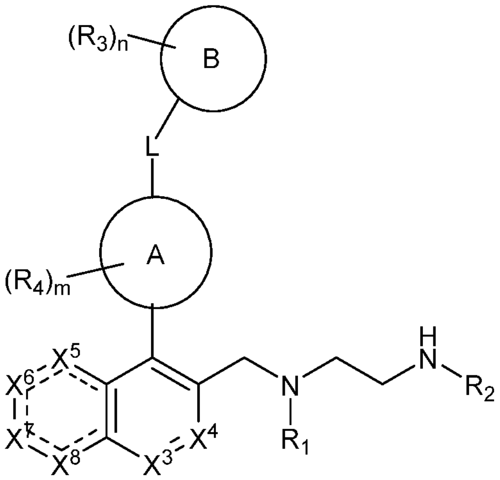

VA

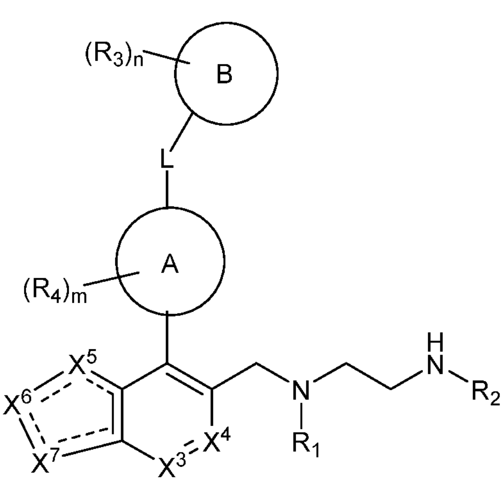

VB

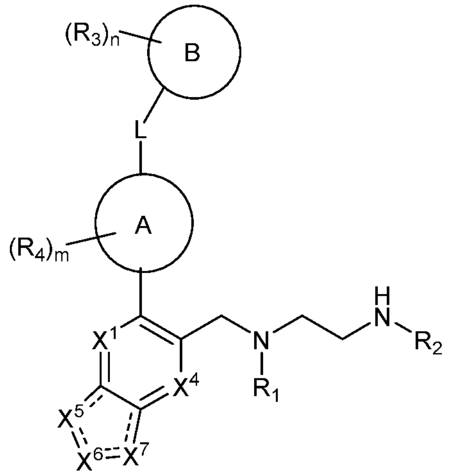

VIA

$X^5$, $X^6$, $X^7$, and $X^8$ are each independently selected from the group consisting of: $C(R)_2$, NR, CR and N; the dash lines are chemical bonds or absent.

In the second aspect of the present invention, provided is a pharmaceutical composition, comprising a therapeutically effective amount of the compound of formula I as described in the first aspect of the present invention, or a pharmaceutically acceptable salt thereof, and one or more pharmaceutically acceptable carriers, excipients, adjuvants, accessories and/or diluents.

In the third aspect of the present invention, provided is a use of a compound of formula I as described in the first aspect of the present invention, or a pharmaceutically acceptable salt thereof, in the preparation of a pharmaceutical composition for the treatment or prevention of diseases associated with PRMT; preferably the PRMT is type I PRMT.

In another preferred embodiment, the disease is selected from the group consisting of tumor, cardiovascular disease, neurodegenerative disease, malaria, AIDS, gout, diabetes, renal failure, chronic lung disease, oculopharyngeal muscular dystrophy, cocaine addiction, pulmonary hypertension, amyotrophic lateral sclerosis, and alcoholic cirrhosis.

In another preferred embodiment, the tumor is selected from any one of brain cancer, glioblastoma, leukemia, lymphoma, Bannayan-Zonana syndrome, Cowden disease, Lhermitte-Duclos disease, breast cancer, Wilms tumor, Ewing sarcoma, rhabdomyosarcoma, ependymoma, medulloblastoma, colon cancer, stomach cancer, bladder cancer, head and neck cancer, kidney cancer, lung cancer, liver cancer, melanoma, ovarian cancer, pancreatic cancer, prostate cancer, sarcoma, osteosarcoma, giant cell tumor of bone, and thyroid cancer.

It should be understood that within the scope of the present invention, the above-mentioned technical features of the present invention and the technical features specifically described in the following (such as embodiments) can be combined with each other to form a new or preferred technical solution. Limited by space, it will not be repeated here.

**Detailed Description of the Invention**

Through extensive and in-depth research, the present inventors unexpectedly discovered a class of compounds with PRMT inhibitory effect for the first time. On this basis, the present invention is completed.

**Terms**

As used herein, the halogen is F, Cl, Br or I.

In the present invention, unless otherwise specified, the terms used herein have the ordinary meaning known to those skilled in the art. In the present invention, all chemical formulas are intended to cover any possible optical or geometric isomers (such as R-form, S-form or racemate, or cis-trans isomers of alkenes, etc.) unless otherwise specified.

In the present invention, the term "$C_1$-$C_6$ alkyl" refers to a straight or branched alkyl having 1 to 6 carbon atoms, and including without limitation methyl, ethyl, propyl, isopropyl, butyl, isobutyl, sec-butyl, tert-butyl, pentyl, and hexyl, etc.; preferably, ethyl, propyl, isopropyl, butyl, isobutyl, sec-butyl, and tert-butyl.

In the present invention, the term "$C_1$-$C_6$alkoxy" refers to a straight or branched alkoxy with 1 to 6 carbon atoms, including without limitation methoxy, ethoxy, propoxy, isopropoxy and butoxy, etc.

In the present invention, the term "$C_2$-$C_6$ alkenyl" refers to a straight or branched alkenyl with 2 to 6 carbon atoms containing a double bond, including without limitation ethenyl, propenyl, butenyl, isobutenyl, pentenyl and hexenyl

etc.

In the present invention, the term "$C_2$-$C_6$alkynyl" refers to a straight or branched alkynyl group with 2 to 6 carbon atoms containing a triple bond, including without limitation ethynyl, propynyl, butynyl, isobutynyl, pentynyl and hexynyl, etc.

In the present invention, the term "C3-C10 cycloalkyl" refers to a cyclic alkyl having 3 to 10 carbon atoms on the ring, including without limitation cyclopropyl, cyclobutyl, cyclopentyl, cyclohexyl, cycloheptyl, cyclooctyl and cyclodecyl, etc. The terms "$C_3$-$C_8$cycloalkyl", "C3-C7 cycloalkyl", and "$C_3$-$C_6$cycloalkyl" have similar meanings.

In the present invention, the term "C3-C10 cycloalkenyl" refers to a cyclic alkenyl having 3 to 10 carbon atoms on the ring, including without limitation cyclopropenyl, cyclobutenyl, cyclopentenyl, cyclohexenyl, cycloheptenyl, cyclooctenyl and cyclodecylene, etc. The term "C3-C7 cycloalkenyl" has a similar meaning.

In the present invention, the term "C1-C12 alkoxycarbonyl" refers to an alkoxycarbonyl having 1 to 12 carbon atoms in the alkyl chain, including without limitation methoxycarbonyl, ethoxycarbonyl, propoxycarbonyl, isopropoxycarbonyl, tert-butoxycarbonyl, benzyloxycarbonyl, and the like.

In the present invention, the term "C1-C12 alkylaminocarbonyl" refers to an alkylaminocarbonyl group having 1 to 12 carbon atoms in the alkyl chain, including without limitation methylaminocarbonyl, ethylaminocarbonyl, propylaminocarbonyl, isopropylaminocarbonyl, tert-butylaminocarbonyl, benzylaminocarbonyl, dimethylaminocarbonyl, and the like.

In the present invention, the term "C5-C9 furanosyl" refers to a furanosyl group with 5 to 9 carbon atoms, wherein the position-1 of the glycosyl is connected to the main chain, including without limitation ribofuranosyl, deoxyribofuranosyl, galactofuranosyl, etc.

In the present invention, the term "C5-C9 pyranosyl" refers to a pyranosyl group having 5 to 9 carbon atoms, wherein the position-1 of the glycosyl is connected to the main chain, including without limitation the glucopyranosyl, glucuronic acidglucopyranosyl, rhamnopyranosyl, galactopyranosyl, mannopyranosyl, xylopyranosyl, etc.

In the present invention, the term "aromatic ring" or "aryl" has the same meaning, preferably "aryl" is "C6-C12 aryl" or "$C_6$-$C_{10}$aryl. The term "C6-C12 aryl" refers to an aromatic ring group having 6 to 12 carbon atoms without heteroatoms in the ring, such as phenyl, naphthyl and the like. The term "$C_6$-$C_{10}$aryl has a similar meaning.

In the present invention, the term "heteroaromatic ring" or "heteroaryl" has the same meaning and refers to a heteroaromatic group containing one to more heteroatoms. The heteroatoms herein include oxygen, sulfur and nitrogen. For example, furyl, thienyl, pyridyl, pyrazolyl, pyrrolyl, N-alkylpyrrolyl, pyrimidinyl, pyrazinyl, imidazolyl, tetrazolyl and the like. The heteroaromatic ring may be fused to an aryl, heterocyclyl or cycloalkyl ring, wherein the ring bonded to the parent structure is a heteroaryl ring. Heteroaryl can be optionally substituted or unsubstituted.

In the present invention, the term "3-12 membered heterocyclyl" refers to a saturated or unsaturated 3-12 membered cyclicgroup containing 1 to 3 heteroatoms selected from oxygen, sulfur and nitrogen on the ring, such as dioxolo, etc. The term "3-7 membered heterocyclyl" has a similar meaning.

In the present invention, the term "substituted" means that one or more hydrogen atoms on a specific group are replaced by a specific substituent. The specific substituents are the corresponding substituents described above, or the substituents appearedin each example. Unless otherwise specified, a substituted group may have a substituent selected from a specific group at any substitutable position, and the substituents may be the same or different at each position. A cyclic substituent, such as a heterocycloalkyl, can be attached to another ring, such as a cycloalkyl, to form a spirobicyclic ring system, eg, two rings sharea common carbon atom. Those skilled in the art will understand that combinations of substituents contemplated by thepresent invention are those that are stable or chemically feasible. The substituents are for example (but not limited to): C1-8 alkyl, C2-8 alkenyl, C2-8 alkynyl, C3-8 cycloalkyl, 3- to 12-membered heterocyclyl, aryl, heteroaryl, halogen, hydroxyl, carboxyl (-COOH), C1-8 aldehyde group, C2-10 acyl, C2-10 ester group, C1-C12 alkoxycarbonyl, amino, alkoxy, C1-10 sulfonyl, etc.

**PRMT inhibitor compounds**

The present invention provides a class of compounds with PRMT inhibitory activity, or pharmaceutically acceptable salts or deuterated compounds thereof:

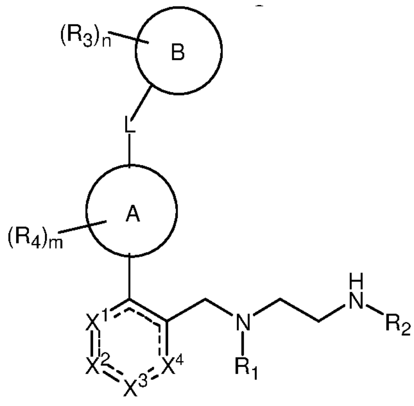

I

wherein, the definition of each group is as described above. In the present invention, preferred compounds have the structure shown in any one of the example compounds P001-P448.

**Pharmaceutical composition and mode of application**

Since the compound of the present invention has excellent activity of inhibiting type I PRMT, the compound of the present invention and its various crystal forms, pharmaceutically acceptable inorganic or organic salts, hydrates or solvates, and pharmaceutical compositions containing the compound of the present invention asthe main active ingredient can be used to treat, prevent and alleviaterelated diseases caused by abnormal activity or expression of PRMT.

The pharmaceutical composition of the present invention comprises the compound of the present invention or a pharmacologically acceptable salt thereof within a safe and effective amount range, and a pharmaceutically acceptable excipient or carrier. Wherein, "safe and effective amount" refers to: the amount of the compound is sufficient to obviously improve the condition without causing severe side effects. Usually, the pharmaceutical composition contains 1-2000 mg of the compound of the present invention per dose, more preferably, 5-500 mg of the compound of the present invention per dose. Preferably, the "one dose" is a capsule or tablet.

"Pharmaceutically acceptable carrier" refers to one or more compatible solid or liquid fillers or gel substances, which are suitable for human use and must have sufficient purity and low toxicity. "Compatibility" herein refers to the ability of components of a composition to blend with each other and with the compounds of the invention without significantly reducing the efficacy of the compounds. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, Magnesium stearate), calcium sulfate, vegetable oil (such as soybean oil, sesame oil, peanut oil, olive oil, etc.), polyols (such as propylene glycol, glycerin, mannitol, sorbitol), emulsifiers (e.g. Tween) etc.®), wetting agents (such as sodium dodecyl sulfate), colorants, flavoring agents, stabilizers, antioxidants, preservatives, pyrogen-free water, etc.

The mode of administration of the compounds or pharmaceutical compositions of the present invention are not particularly limited, and representative mode of administration include, but are not limited to, oral, intratumoral, rectal, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) filler or compatibilizer, such as starch, lactose, sucrose, glucose, mannitol and silicic acid; (b) adhesives, such as hydroxymethyl cellulose, alginate, gelatin, polyvinylpyrrolidone, sucrose and gum arabic; (c) moisturizing agents, such as glycerol; (d) disintegrating agents, such as agar, calcium carbonate, potato starch or cassava starch, algic acid, some complex silicates, and sodium carbonate; (e) retarding solvent, such as paraffin; (f) absorption accelerators, such as quaternary amine compounds; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. In capsules, tablets and pills, dosage forms may also contain buffers.

Solid dosage forms such as tablets, sugar pills, capsules and granules may be prepared using coating and shell materials such as casing and other materials well known in the art. They may comprise an opacifying agent, and the release of the active compound in such a composition may be released in a delayed manner in a part of the digestive tract. Examples of embedding components that can be employed are polymeric substances and wax substances. If necessary, the active compound may also form a microcapsule form with one or more of the excipients described above.

Liquid dosage forms for oral administration include pharmaceutically acceptable emulsions, solutions, suspensions, syrups or tinctures. In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or mixtures thereof.

In addition to these inert diluents, the composition may also contain auxiliaries such as wetting agents, emulsifiers, suspending agents, sweeteners, flavoring agents and spices.

In addition to the active compound, the suspension may comprise suspending agents, such as ethoxylatedisooctadecanol, polyoxyethylene sorbitol and dehydrated sorbitol esters, microcrystalline cellulose, methanolic aluminum, agar, and any mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

Dosage forms of the compound of the invention for topical administration include ointments, powder, patches, propellants and inhalants. The active ingredient is mixed under sterile conditions with a physiologically acceptable carrier and any preservatives, buffers or propellants as may be required.

The compound of the present invention can be administered alone or in combination with other pharmaceutically acceptable compounds. In some preferred embodiments, the compounds of the present invention can be administered together with other small molecular compounds to form PROTACs, or with other macromolecular compounds such as monoclonal antibodies to form ADCs.

When using the pharmaceutical composition, a safe and effective amount of the compound of the present invention is administered to mammals in need of treatment (such as humans), where the dosage at the time of administration is the pharmaceutically considered effective dose, which is typically 1 to 2,000 mg per day, more preferably 5 to 500mg per day for a 60 kg body weight human. Of course, the specific dosage should also consider the route of administration, the patient's health conditionand other factors, which are within the range of skilled doctors.

The present invention is further described below in conjunction with specific embodiments. It is to be understood that these examples are intended to illustrate the invention only and not to limit the scope of the invention. The experimental methods in the following examples that do not specify specific conditions are usually based on conventional conditions or conditions recommended by the manufacturer. Unless otherwise specified, percentages and portions are calculated by weight.

General synthesis methods:

**Intermediate Synthesis 1: Synthesis of 2-(2,3-difluoro-6,9-dihydro-5H-benzo[7]cyclopenten-7-yl) -4,4,5,5-tetramethyl-1,3,2-dioxaborolane (intermediate A):**

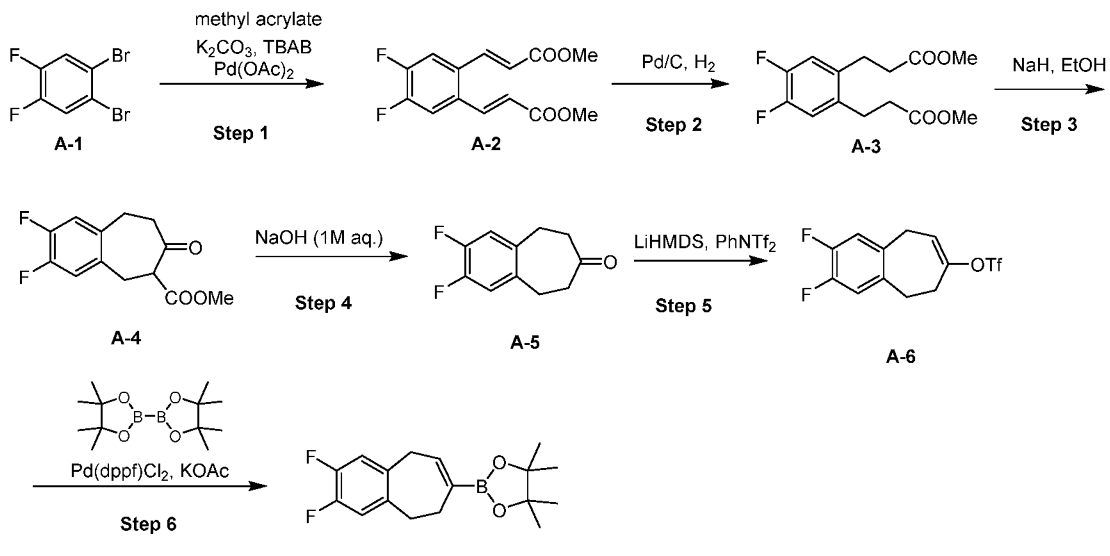


Intermediate A

**Step 1:** To a solution of 1,2-dibromo-4,5-difluorobenzene (A-1) (2.0 g, 7.36 mmol) and methyl acrylate (1.90 g, 22.1 mmol, 1.99 mL) in dimethylformamide (10 mL) was added tetrabutylammonium bromide (2.37 g, 7.36 mmol), and potassium carbonate (2.54 g, 18.4 mmol). After the reaction solution was replaced with nitrogen for 1 minute, palladium acetate (33.03 mg, 147 mmol), and the reaction solution was stirred at 80 °C for 16 hours. The reaction solution was then filtered, and the filter cake was washed 3 times with ethyl acetate (50 mL).The organic layer was washed with water (100 mL), dried with magnesium sulfate, filtered, and the filtrate was concentrated to dryness under reduced pressure, then purified by column chromatography (silica, 15% ethyl acetate in petroleum ether) to give the compound (2E,2'E)-dimethyl 3,3'-(4,5-difluoro-1,2-phenylene)diacrylate (A-2) (6.0 g, 21.26 mmol, 96.3% yield), as a white solid. $^1$H NMR (400 MHz, $CDCl_3$) δ (ppm) 7.93 (d, 2H), 7.39 (t, 2H), 6.31 (d, 2H), 3.85 (s, 6H)

**Step 2:** To a solution of (2E,2'E)-dimethyl 3,3'-(4,5-difluoro-1,2-phenylene)diacrylate (A-2) (6.0 g, 21.3 mmol) in methanol (150 mL) was added palladium/carbon (1.0 g, 10%), the reaction solution was degassed under reduced pressure and replaced with hydrogen for multipletimes, then stirred undera hydrogen balloon atmosphere for 16 hours.The reaction solution was then filtered with diatomite pad and the filter cake was washed with ethyl acetate (50 mL) three times.The filtrate was concentrated to dryness under reduced pressure, then purified by column chromatography (silica, 15% ethyl acetate in petroleum ether) to give the compound dimethyl 3,3'-(4,5-difluoro-1,2-phenylene)dipropionate (A-3) (5.8 g, 20.26 mmol, 95.31% yield), as a colorless oil. $^1$H NMR (400 MHz, $CDCl_3$) δ(ppm) 6.98 (t, 2H), 3.71 (s, 6H), 2.94 (t, 4H), 2.61 (t, 4H);

**Step 3:** A stirred solution of dimethyl 3,3'-(4,5-difluoro-1,2-phenylene)dipropionate (A-3) (1.0 g, 3.49 mmol) in xylene (5 mL) was slowly added at 95 °C to a suspension of NaH (209.59 mg, 5.24 mmol, 60% purity) in xylene (5 mL), with one drop of anhydrous ethanol being added forevery 1 mL of the solution of 3,3'-(4,5-difluoro-1,2-phenylene)dipropionate (A-3) in xylene. After addition, the temperature of the reaction was raised to 115 °C and the reaction was continued at this temperature for 1.5 hours. Six reactions were set up in parallel. The completion of the reaction and the generation of the target product were detected by LC-MS.The 6 reactions were combined, quenched with ice water (30 mL) and 1M hydrochloric acid aqueous solution (30 mL), and extracted 3 times with ethyl acetate (40 mL).The organic layer was dried with magnesium sulfate, filtered, and the filtrate was concentrated to dryness under reduced pressure to give a crude product of methyl 2,3-difluoro-7-oxo-6,7,8,9-tetrahydro-5H-benzo[7]cyclopentene-6-carboxylate (**A-4**) (5.3 g) as a yellow oil, which was used directly in the next step without purification. LCMS: (ESI) m/z=254.7 $[M+H]^+$, 268.8 $[M+14]^+$;

**Step 4:** To a solution of methyl 2,3-difluoro-7-oxo-6,7,8,9-tetrahydro-5H-benzo[7]cyclopentene-6-carboxylate (**A-4**) (5.3 g, 20.85 mmol) in ethanol (60 ml) was added sodium hydroxide (1 M aqueous solution, 62.5 ml), and the reaction solution was reacted for 2 hours at 90 °C. The completion of the reaction and the generation of the target product were detected by LC-MS. The reaction solution was then concentrated to dryness under reduced pressure, diluted with ethyl acetate (30 mL), and washed with water (30 mL).The organic layer was dried with anhydrous magnesium sulfate, filtered and the filtrate was concentrated to dryness under reduced pressure, then purified by column chromatography (silica, 12% ethyl acetate in petroleum ether) to give compound 2,3-difluoro-8,9-dihydro-5H-benzo[7]cyclopenten-7(6H)-one (A-5) (2.6 g, 13.2 mmol, with a two-step yield of 78.9%), as a yellow solid. $^1$H NMR (400 MHz, $CDCl_3$) δ(ppm) 7.06 (t, 2H), 2.78-2.95 (m, 4H), 2.53-2.69 (m, 4H),

**Step 5:** To a solution of 2,3-difluoro-8,9-dihydro-5H-benzo[7]cyclopenten-7(6H)-one (**A-5**) (1.0 g, 5.10 mmol) in tetrahydrofuran (10 ml) was added lithium bis(trimethylsilyl)amide (1 M tetrahydrofuran solution, 5.61 ml) at -70 °C, then reacted at -70 °C for 1 h under nitrogen protection. The reaction solution was added with N-phenylbis(trifluoromethanesulfonyl)imide (2.00 g, 5.61 mmol) at -70 °C and then slowly warmed to room temperature for 15 h. The completion of the reaction was confirmedby TLC detection (petroleum ether:ethyl acetate=8:1, Rf =0.8).The reaction solution was then quenched with water (25 mL) and extracted three times with ethyl acetate (15 mL).The organic layer was dried with magnesium sulfate, filtered and the filtrate was concentrated to dryness under reduced pressure, then purified by column chromatography (silica, 5% ethyl acetate in petroleum ether) to give compound 2,3-difluoro-6,9-dihydro-5H-benzo[7]cyclopenten-7-yl trifluoromethanesulfonate (**A-6**) (1.27 g, 3.87 mmol, 75.91% yield), as a colorless oil. $^1$H NMR (400 MHz, $CDCl_3$) δ(ppm) 6.87-7.09 (m, 2H), 5.89-5.97 (m, 1H), 3.35-3.49 (m, 2H), 2.89-3.02 (m, 2H), 2.55-2.75 (m, 2H);

**Step 6:** To a solution of 2,3-difluoro-6,9-dihydro-5H-benzo[7]cyclopenten-7-yl trifluoromethanesulfonate (**A-6**) (1.27 g, 3.87 mmol) and bis(pinacolato)diboron(1.08 g, 4.26 mmol) in dioxane (20 mL) was added potassium acetate (1.14 g, 11.61 mmol) and 1,1-bis(diphenylphosphino)ferrocene palladium chloride (283.09 mg, 386.89 μmol), and the reaction solution was replaced with nitrogen for 1 min, then reacted at 100 °Cunderstirring for 16 hours under nitrogen protection. The complete consumption of the raw material and the generation of new spots were detected by TLC (petroleum ether:ethyl acetate=8:1, Rf =0.8). The reaction solution was then cooled to room temperature, diluted with water (20 mL) and extracted with ethyl acetate (20 mL) three times.The organic layer was dried with magnesium sulfate, filtered and the filtrate was concentrated to dryness under reduced pressure, then purified by column chromatography (silica, 3% ethyl acetate in petroleum ether) to give compound 2-(2,3-difluoro-6,9-dihydro-5H-benzo[7]cyclopenten-7-yl)-4,4,5,5-tetramethyl-1,3,2-dioxabo rolane (**Intermediate A**) (880 mg, 2.87 mmol,

74.3% yield), as a yellow solid. $^1H$ NMR: (400 MHz, $CDCl_3$) δ(ppm) 6.95 (m, 1H), 6.86 (m, 1H), 6.54-6.69 (m, 1H), 3.45-3.58 (m, 2H), 2.87-3.01 (m, 2H), 2.29-2.54 (m, 2H), 1.25 (s, 12H)

**Intermediate Synthesis 2: Synthesis of 4-(4,4,5,5-tetramethyl-1,3,2-dioxaborolan-2-yl)-1-(m-tolyl)-1,2,3,6-tetrahydropyridine (Intermediate B):**

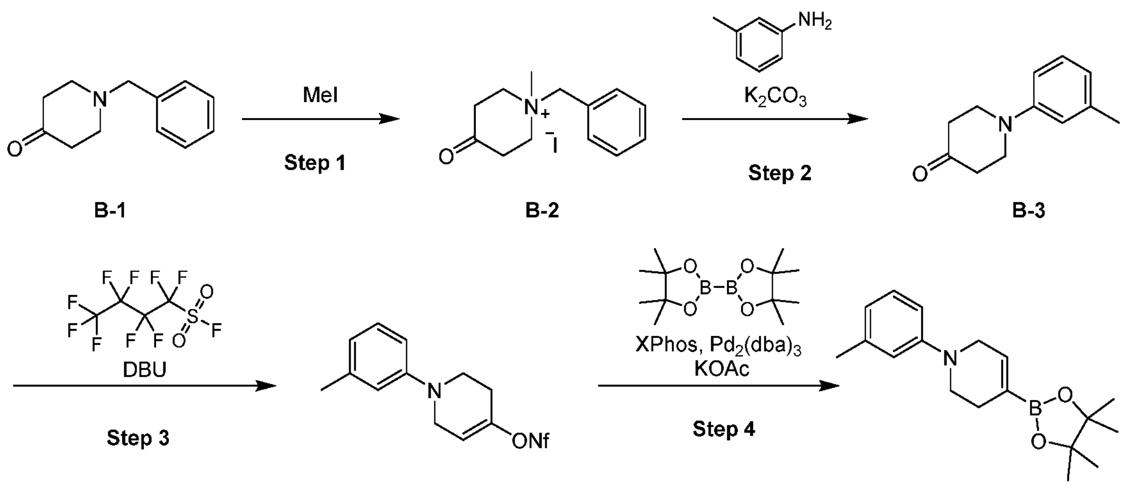


B-1 B-2 B-3 B-4 Intermediate B

__Step 1:__ To a solution of 1-benzylpiperidin-4-one (B-1) (100 g, 528 mmol, 1.0 equiv.) in acetone (700 mL) was added iodomethane (90.0 g, 634 mmol, 39.5 mL, 1.2 equiv.), and the reaction solution was stirred at 20 °C for 16 hours, with white solid precipitated. The reaction solution was then filtered, and the filter cake was washed twice with dry acetone (200 mL), collected and concentrated to dryness under reduced pressure to give 1-benzyl-1-methyl-4-oxopiperidin-1-iodide (B-2) (297 g, 897 mmol, 84.86% yield), as an off-white solid, which was used directly in the next step without purification.

__Step 2:__ A turbid solution of 1-benzyl-1-methyl-4-oxopiperidin-1-iodide (B-2) (162 g, 489 mmol, 1.3 equiv.) in ethanol (400 mL) and water (200 mL) was added in batches into arefluxd (90 °C) 3-methylaniline (40 g, 373 mmol, 1.0 equiv.) and potassium carbonate (7.74 g, 56.0 mmol, 0.15 equiv.) in ethanol (500 mL), and the reaction solution was stirred at 90 °C for another 40 minutes.The completion of the reaction and the generation of new spots were detected by TLC (petroleum ether: ethyl acetate = 4:1, Rf = 0.5).The reaction solution was then diluted with water (300 mL) and extracted three times with dichloromethane (200 mL).The organic layer was dried with magnesium sulfate, filtered, and the filtrate was concentrated to dryness under reduced pressure, then purified by column chromatography (silica, 12% ethyl acetate in petroleum ether) to give compound 1-(m-tolyl)piperidin-4-one (B-3) (59 g, 312 mmol, 83.5% yield) in a yellow syrup form;$^1H$ NMR (400 MHz, $CDCl_3$) δ(ppm) 7.21 (t, 1H), 6.79-6.89 (m, 2H), 6.75 (d, 1H), 3.61 (t, 4H), 2.59 (t, 4H), 2.35 (s, 3H)

__Step 3:__ To a solution of 1-(m-tolyl)piperidin-4-one (B-3) (90 g, 475 mmol, 1 equiv.) and 1,1,2,2,3,3,4,4,4-nonafluorobutane-1-sulfonyl fluoride (216 g, 715 mmol, 1.5 equiv.) in tetrahydrofuran (1200 mL) was added dropwise DBU (217 g, 1.43 mol, 215 mL, 3 equiv.) at 0 °C, and the reaction solution was stirred at 25 °C for 3 hours.The completion of the reaction was detected by LCMS. The reaction solution was then diluted with water (1 liter), adjusted to pH 3-4 with 10 % phosphoric acid, and extracted with methyl tert-butyl ether (1 liter) for 3 times.The organic layer was dried with magnesium sulfate, filtered, and the filtrate was concentrated to dryness under reduced pressure to obtain a roughproduct. The rough product was dispersed in petroleum ether (1.5 L) and stirred at 20 °C for 1 hour, filtered, and the filtrate was concentrated to dryness under reduced pressure to obtain a crude product of 1-(m-tolyl)-1,2,3,6-tetrahydropyridin-4-yl 1,1,2,2,3,3,4,4,4-nonafluorobutane-1-sulfonate (B-4) (175 g), as a yellow oil, which was used directly in the next step without purification. LCMS: (ESI) m/z=472.1 $[M+H]^+$;

__Step 4:__ Two reactions were set up in parallel. To a solution of 1-(m-tolyl)-1,2,3,6-tetrahydropyridin-4-yl 1,1,2,2,3,3,4,4,4-nonafluorobutane-1-sulfonate (**B-4**) (70 g, 148 mmol, 1.0 eq.), and 4,4,4',4',5,5,5',5'-octamethyl-2,2'-bis(1,3,2-dioxaborolan) (42.0 g. 165.mmol, 1.11 equiv.) in 1,4-dioxane (1000 mL) was added potassium acetate (44 g, 448 mmol), then the reaction solution was degassed under reduced pressure and replaced with nitrogen for multipletimes, and 2-dicyclohexylphosphino-2,4,6-triisopropylbiphenyl (4.3 g, 9.02 mmol, 0.06 equiv.) and tris(dibenzylideneacetone)dipalladium (4.10 g, 4.47 mmol, 0.03 equiv.) were added, then the reaction solution was stirred at

90 °C under nitrogen protection for 16 hours.The completion of the reaction was detected by LCMS. The reaction solution was then cooled to room temperature, filtered, the filter cake was washed with petroleum ether (500 mL) 3 times, and the filtrate was concentrated to dryness under reduced pressure, then purified by column chromatography (silica, 5% ethyl acetate in petroleum ether) to give a crude product, which was beatwith n-pentane (150 mL) to give 4-(4,4,5,5-tetramethyl-1,3,2-dioxaborolan-2-yl)-1-(m-tolyl)-1,2,3,6-tetrahydropyridine (Intermediate B) as a yellow solid (32 g, 101.60 mmol, 34.21% yield, 95% purity). LCMS: (ESI) m/z=300.2 $[M+H]^+$; $^1H$ NMR (400 MHz, $CDCl_3$) δ(ppm)7.10-7.22 (m, 1H), 6.70-6.81 (m, 2H), 6.56-6.69 (m, 2H), 3.76-3.83 (m, 2H), 3.33 (t, 2H), 2.38-2.45 (m, 2H), 2.38-2.46 (m, 1H), 2.34 (s, 3H), 1.30 (s, 12H)

**Intermediate Synthesis 3: Synthesis of 2,3,4,5-tetrahydro-1H-benzo[d]azepine (Intermediate C).**

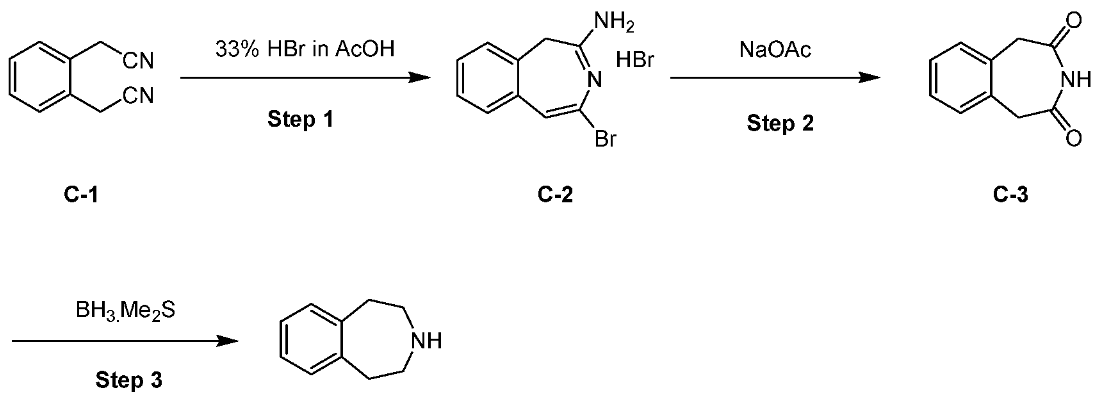


C-1 C-2 C-3

Intermediate C

**Step 1:**To a solution of 2,2'-(1,2-phenylene)diacetonitrile(**C-1**)(50 g, 320.14 mmol)in acetic acid(60 mL) was slowly added hydrogen bromide(196.23 g, 800.34 mmol, 33%hydrogen bromide in acetic acid)for 1 hour at 25°C.The reaction solution continued stirring for 1 hour.The completion of the reaction was detected by TLC.The suspended reaction solution was then filtered and washed with isopropyl ether to obtain a solid compound, which was dried under reduced pressure to give 4-bromo-1H-benzo[d]azepin-2-amine (**C-2**) as a bright yellow solid.The bright yellow solid crude product was used directly in the next step without purification.

**Step 2:**Asolution of 4-bromo-1H-benzo[d]azepin-2-amine (C-2) (88 g, 276.72 mmol, hydrobromidehydrochloride) in water (600 mL) was heated to 85 °C, and sodium acetate (29.51 g, 359.74 mmol) was added step by step.The reaction washeated to 95 °C and stirred for 6 hours.The completion of the reaction was detected by TLC. The reaction solution was then cooled to room temperature, filtered to obtaina solid compound, and the solid compound was washed with water to give the crude productof 1H-benzo[d]azepine-2,4(3H,5H)-dione (**C-3**) (47 g), as a pink solid.This crude product was used directly in the next step without purification.

**Step 3:** To a solution of 1H-benzo[d]azepine-2,4(3H,5H)-dione (**C-3**) (47 g, 268.29 mmol) in toluene (50 mL) was added slowly dropwise di-methylsulfide borane solution(10 M, 80.49 mL)under nitrogen atmosphere.The pink reaction solution was heated to 110 °C and stirred for 6 hours. TLC (Rf = 0.40) detection indicated complete reaction of the raw material.The reaction solution was then cooled to room temperature, methanol (115 mL) was added slowly dropwise, and the reaction solution was heated to 100 °Cafter addition and stirred for 1 hour. The reaction solution was then cooled to 20 °C, 4M hydrochloric acid-dioxane was added, the pH of the reaction solution was adjusted to 4-5, and the reaction was stirred at 20 °C for 2 hours. The reaction suspension was then filtered to give 2,3,4,5-tetrahydro-1H-benzo[d]azepine (**Intermediate C**) (26 g, 141 mmol, 52.7% yield, hydrochloride) as a pink solid. $^1H$ NMR (400 MHz, DMSO-$d_6$) δ(ppm)9.50 (br, 2H), 7.14-7.24 (m, 4H), 3.10-3.20 (m, 8H);

**Intermediate Synthesis 4: Synthesis of 7-(trifluoromethyl)-2,3,4,5-tetrahydro-1H-benzo[d]azepine (Intermediate D):**

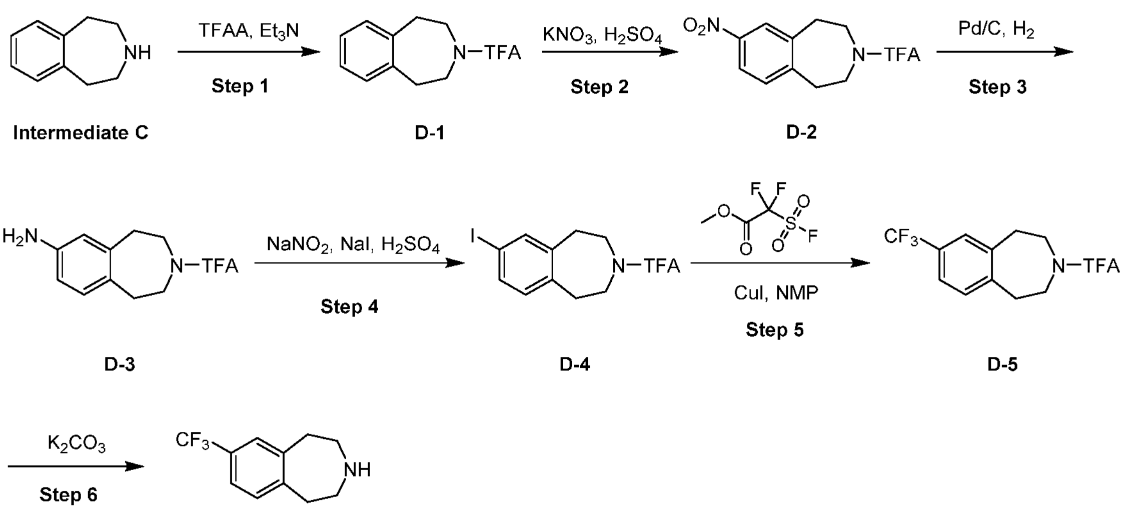


Intermediate C D-1 D-2

D-3 D-4 D-5

Intermediate D

**Step 1:** To a solution of 2,3,4,5-tetrahydro-1H-benzo[d]azepine (Intermediate C) (11 g free base, 74.72 mmol) in dichloromethane (220 mL) was added triethylamine (9.07 g, 89.7 mmol) at 25 °C under nitrogen protection, and trifluoromethanesulfonic anhydride (18.8 g, 89.7 mmol) was added dropwise at 0 °C and the reaction solution was stirred at 0 °C for 1 hour.The completion of the reaction was detected by LCMS.The reaction solution was then washed once with saturated sodium bicarbonate aqueous solution (200 mL), twice with 1 M hydrochloric acid aqueous solution (200 mL), and once with saturated salt water (200 mL)successively.The organic phase was dried with magnesium sulfate and filtered.The filtrate was concentrated to dryness under reduced pressure, and then purified by column chromatography (silica, 13% ethyl acetate in petroleum ether) to give 1-(4,5-dihydro-1H-benzo[d]azepin-3(2H)-yl)-2,2,2-trifluoroethanone (**D-1**) (16 g, 65.8 mmol, 88.0% yield) as a white solid. LCMS: (ESI) m/z= 244.1 $[M+H]^+$; $^1H$ NMR (400MHz, $CDCl_3$) δ(ppm) 7.15-7.24 (m, 4H), 3.77-3.83 (m, 2H), 3.70-3.75 (m, 2H), 2.98-3.04 (m, 4H);

**Step 2:**Two reactions were set up in parallel.1-(4,5-dihydro-1H-benzo[d]azepin-3(2H)-yl)-2,2,2-trifluoroethanone (D-1) (8.0 g, 32.9 mmol) was added to concentrated sulfuric acid (32 mL) at 0 °C and the mixture was stirred for 10 min to obtain a grayish yellow solution. Potassium nitrate (2.66 g, 26.3 mmol) was added in batches over 20 min at 0 °C, then the reaction solution was stirred at 0 °C for 30 minutes. The complete consumption of the raw material was detected by TLC. The reaction solution was then slowly poured into stirred ice water (50 mL) and extracted three times with ethyl acetate (50 mL).The organic layer was washed once with saturated salt water(50 mL), dried with magnesium sulfate, filtered, and the filtrate was concentrated to dryness under reduced pressure, then purified by column chromatography (silica, 15% ethyl acetate in petroleum ether) to give 2,2,2-trifluoro-1-(7-nitro-4,5-dihydro-1H-benzo[d] azepin-3(2H)-yl)ethanone (**D-2**) (8.6 g, 29.8 mmol, 45.2% yield, 99.7% purity)as a white solid . LCMS: (ESI) m/z=288.9 $[M+H]^+$; $^1H$ NMR (400 MHz, $CDCl_3$) δ(ppm) 8.05-8.10 (m, 2H), 7.32-7.39 (m, 1H), 3.80-3.87 (m, 2H), 3.77 (m, 2H), 3.09-3.17 (m, 4H)

**Step 3:** To a solution of 2,2,2-trifluoro-1-(7-nitro-4,5-dihydro-1H-benzo[d] azepin-3(2H)-yl)ethanone (**D-2**) (4.3 g, 14.9 mmol) in methanol (130 ml) was added 10% wet palladium carbon (794 mg, 746 μ mol) under nitrogen protection, the suspension was replaced three times with a hydrogen balloon and the reaction was stirred at 25 °C for 16 hours under hydrogen atmosphere.The completion of the reaction was detected by LCMS.The reaction solution was then filtered, the filtrate was concentrated under reduced pressure to dryness, andthen purified by column chromatography (silica, 30% ethyl acetate in petroleum ether) to obtain 1- (7-amino-4,5-dihydro-1H-benzo[d]azepin-3 (2H) - yl) -2,2,2-trifluoroethanone (**D-3**) as a bright yellow solid (6.6 g, 25.5 mmol, 85.4% yield, 99.7% purity). LCMS: (ESI) m/z=259.0 $[M+H]^+$;$^1H$ NMR (400 MHz, $CDCl_3$) δ (ppm)6.94 (dd, 1H), 6.47-6.55 (m, 2H), 3.71-3.78 (m, 2H), 3.57-3.70 (m, 4H), 2.83-2.94 (m, 4H)

**Step 4:** To a solution of 1-(7-amino-4,5-dihydro-1H-benzo[d]azepin-3(2H)-yl)-2,2,2-trifluoroethanone (**D-3**) (4.5 g, 17.4 mmol) in a mixed solution of water (58 ml) and sulfuric acid (9.57 g, 97.6 mmol.) was added dropwise an aqueous solution (29 mL) of $NaNO_2$ (1.56 g, 22.6 mmol) at 0 °C, and then stirred for 10 minutes.Then an aqueous solution of NaI (3.92 g, 26.1 mmol) in sulfuric acid (1M per liter, 5.8 mL) was added to the above solution. The reaction was naturally warmed to 25 °C and stirred for 16 hours. The complete consumption of the raw material was detected by TLC. The reaction solution was then extracted three times with dichloromethane (100 mL). The organic layer was washed twice with half-saturated sodium thiosulfate aqueous solution (100 mL), dried with magnesium sulfate, filtered, and the filtrate was concentrated to dryness under reduced pressure, and then purified by column

chromatography (silica, 4.3% ethyl acetate in petroleum ether) to give 2,2,2-trifluoro-1-(7-iodo-4,5-dihydro-1H-benzo[d]azepin-3(2H)-yl)ethanone (**D-4**) as a white solid ( 4.7 g, 12.7 mmol, 73.1% yield). $^{1}$H NMR (400 MHz, $CDCl_3$) δ(ppm) 7.46-7.57 (m, 2H), 6.86-6.94 (m, 1H), 3.72-3.83 (m, 2H), 3.62-3.72 (m, 2H), 2.93 (m, 4H); $^{19}$F NMR (376 MHz, $CDCl_3$) δ(ppm) -68.07

**Step 5:** To a solution of 2,2,2-trifluoro-1-(7-iodo-4,5-dihydro-1H-benzo[d]azepin-3(2H)-yl)ethanone (**D-4**) (250 mg, 677 μmol) in dimethylformamide (2 mL) was added methyl 2,2-difluoro-2-(fluorosulfonyl)acetate (651 mg, 3.39 mmol), cuprous iodide (26 mg, 135 μmol), and N-methylpyrrolidone (806 mg, 8.13 mmol) in turn at 25 °C under nitrogen atmosphere. The reaction was heated to 80 °C and stirred for 12 hours.The completion of the reaction was detected by LCMS. The reaction solution was then diluted with water (10 mL) and filtered. The filtrate was extracted twice with ethyl acetate (10 mL).The organic layer was dried with magnesium sulfate, filtered, concentrated to dryness under reduced pressure, and then purified by column chromatography (silica, 4% ethyl acetate in petroleum ether) to give 2,2,2-trifluoro-1-(7-(trifluoromethyl)-4,5-dihydro-1H-benzo[d]azepin-3(2H)-yl)ethanone (**D-5**) as a yellow solid (180 mg, 578 μmol, 85.4% yield).

**Step 6:** To a solution of 2,2,2-trifluoro-1-(7-(trifluoromethyl)-4,5-dihydro-1H-benzo[d]azepin-3(2H)-yl)ethanone (**D-5**) (120 mg, 386 μmol) in methanol (3 mL) was added potassium carbonate (160 mg, 1.16 mmol) all at once, and the reaction was heated to 50 °C and stirred for 12 hours. The complete consumption of the raw material and the generation of target product were detected by LCMS. The reaction solution was then concentrated to dryness, added with water (5 mL) and extracted three times with ethyl acetate (5 mL). The organic layer was dried with magnesium sulfate, filtered, and concentrated to dryness under reduced pressure to give a crude product of 7-(trifluoromethyl)-2,3,4,5-tetrahydro-1H-benzo[d]azepine (**Intermediate D**) (80 mg) in a yellow syrup form, which was used directly in the next step without further purification. LCMS: (ESI) m/z=215.9 $[M+H]^+$;

**Intermediate Synthesis 5: Synthesis of 7,8-difluoro-2,3,4,5-tetrahydro-1H-benzo[d] azepine Intermediate E):**

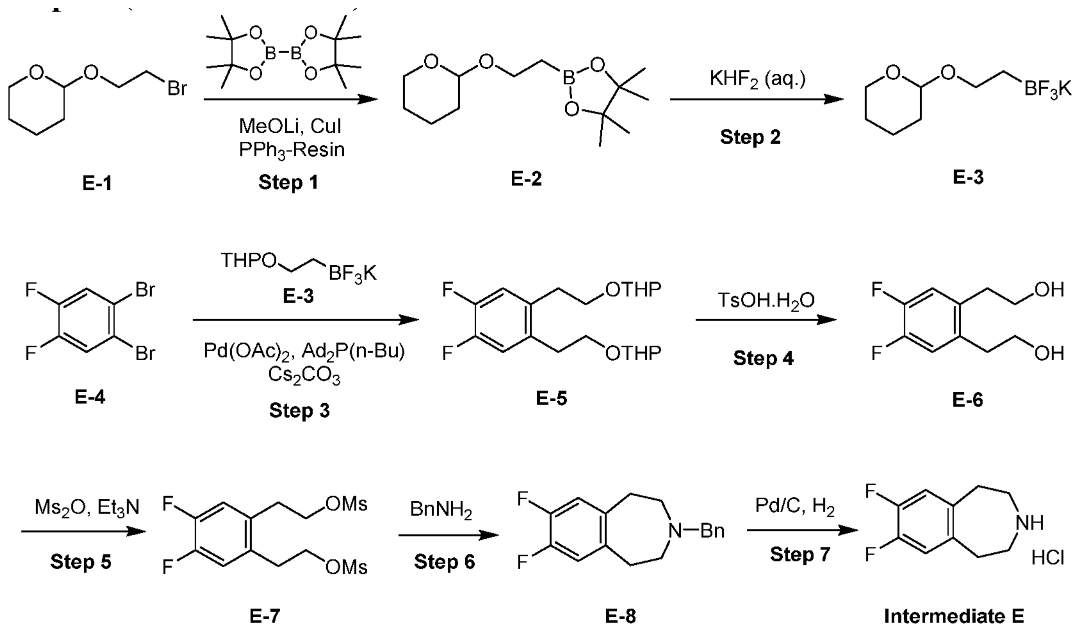


**Step 1:** To a solution of 2-(2-bromoethoxy)tetrahydro-2H-pyran (**E-1**) (18 g, 86.09 mmol) and 4,4,4',4',5,5,5',5'-octamethyl-2,2'-bis(1,3,2-dioxaborolane) (26.2 g, 103 mmol) in dimethylformamide (350 ml) was added lithium methoxide(6.54 g, 172.18 mmol, 2.0 eq), cuprous iodide (1.64 g, 8.61 mmol) and triphenylphosphine resin (2.25 g, ~3 mmol/g, 8.61 mmol), and the reaction solution was stirred at 25 °C for 12 hours. After the reaction was detected as finished, it was diluted with dichloromethane (200 mL), and filtered with diatomite.The filter cake was washed twice with dichloromethane (100 mL), and the filtrates were combined and concentrated to dryness under reduced pressure, then poured into saturated ammonium chloride solution and extracted with tert-butyl methyl ether (200 mL) three times.The resulting organic phase was washed respectively with water (300 mL) and saturated salt water(300 mL), dried with anhydrous sodium sulfate, and filtered.The filtrate was concentrated to dryness under reduced pressure to give a crude product of 4,4,5,5-tetramethyl-2-(2-(((tetrahydro-2H-pyran-2-yl)oxy)ethyl)-1,3,2-dioxaborolane (**E-2**) (19.0 g), as a colorless oil, and the crude product was used directly in the next step without further

purification. $^1$H NMR (400 MHz, $CDCl_3$) δ(ppm) 4.62 (t, 1H), 3.83-3.94 (m, 2H), 3.46-3.59 (m, 2H), 1.77-1.91 (m, 1H), 1.65-1.74 (m, 1H), 1.45-1.61 (m, 4H), 1.25 (s, 12H), 1.19 (t, 2H).

**Step 2:** To a solution of 4,4,5,5-tetramethyl-2-(2-(((tetrahydro-2H-pyran-2-yl)oxy)ethyl)-1,3,2-dioxaborolane (**E-2**) (19 g, 74.18 mmol) in tetrahydrofuran (540 ml) was added a solution ofpotassium bifluoride (17.38 g, 222.53 mmol, 7.33 ml) in water (60 ml), and stirred at 25 °C for 2 hours.The reaction solution was then concentrated to dryness under reduced pressure and lyophilized to obtain a white solid. The solid was washed 4 times with dry acetone (50 mL), filtered and the filtrate was concentrated to dryness under reduced pressure. The resulting solid was purified by beating with tert-butyl methyl ether (100 mL), collected by filtration and dried to give potassium (2-(((tetrahydro-2H-pyran-2-yl)oxy)ethyl)trifluoroborate (**E-3**) (13.5 g, 57.18 mmol, 77.09% yield),as a white solid. $^1$H NMR (400 MHz, DMSO-$d_6$) δ(ppm) 4.44 (dd, 1H), 3.72 (ddd, 1H), 3.58 (ddd, 1H), 3.33-3.39 (m, 1H), 3.23 (ddd, 1H), 1.64-1.77 (m, 1H), 1.50-1.60 (m, 1H), 1.29-1.49 (m, 4H), 0.20-0.45 (m, 2H).

**Step 3:**To a solution of 1,2-dibromo-4,5-difluorobenzene (E-4)(2.0 g, 7.36 mmol) and potassium (2-(((tetrahydro-2H-pyran-2-yl)oxy)ethyl)trifluoroborate (**E-3**)(3.99 g, 16.92 mmol) in water(10 mL) anddioxane(50 mL) was added Bis (1-adamantyl) - butyl phosphine(527.49 mg, 1.47mmol),cesium carbonate(14.38 g, 44.14 mmol) and palladium acetate(495.45 mg, 2.21 mmol), andthen replaced with nitrogen for 5minutes.The reaction was stirred at 100°C for 16 hours. TLC (petroleum ether: ethyl acetate = 8:1) detection indicatedcomplete consumption of the raw materials and the generation of new spots.The reaction solution was filtered with diatomite, then diluted with water (30 mL) and extracted with ethyl acetate (50 mL) three times.The organic phases were combined and washed with salt water (100 mL), dried with anhydrous magnesium sulfate, filtered and the filtrate was concentrated to dryness under reduced pressure,and then purified by column chromatography (silica, 10% ethyl acetate in petroleum ether) to afford 2,2'-((((4,5-difluoro-1,2-phenylene)bis(ethane-2,1-diyl))bis(oxy))bis(tetrahydro-2H-pyran) (**E-5**) (1.25 g, 3.37 mmol, 45.87% yield), as a yellow oil.$^1$H NMR (400 MHz, $CDCl_3$) δ(ppm)7.03 (t, 1H), 6.97-7.09 (m, 1H), 4.56-4.60 (m, 2H), 3.91 (td, 2H), 3.68-3.77 (m, 2H), 3.57 (td, 2H), 3.42-3.52 (m, 2H), 2.91 (t, 4H), 1.75-1.87 (m, 2H), 1.65-1.74 (m, 2H), 1.45-1.63 (m, 8H).

**Step 4:** To a solution of 2,2'-((((4,5-difluoro-1,2-phenylene)bis(ethane-2,1-diyl))bis(oxy))bis(tetrahydro-2H-pyran) (**E-5**) (1.25 g, 3.37 mmol) in methanol (15 ml) was added p-toluenesulfonic acid monohydrate (801.08 mg, 4.21 mmol, 1.2 equiv).The reaction solution was stirred at 25 °C for 16 hours.The complete consumption of the raw material and the generation of new spots were detected by TLC (petroleum ether: ethyl acetate = 10:1). Solvent was then removed by concentration under reduced pressure, dichloromethane (20 mL) and saturated sodium bicarbonate solution (20 mL) were added, and the aqueous phase was extracted five times with dichloromethane (20 mL). The organic layer was washed with saturated salt water(50 mL), dried with anhydrous magnesium sulfate, and filtered. The filtrate was concentrated to dryness under reduced pressure to give 2,2'-(4,5-difluoro-1,2-phenylene)diethanol (**E-6**) (700 mg, 3.46 mmol, 98.65% yield), as a yellow solid.The crude product was used directly in the next step without purification. $^1$H NMR (400 MHz, $CDCl_3$) δ(ppm)7.03 (t, 2H), 3.86 (t, 4H), 2.88 (t,4H), 1.97 (br, 2H)

**Step 5:** To a solution of 2,2'-(4,5-difluoro-1,2-phenylene)diethanol (**E-6**) (700 mg, 3.46 mmol) and triethylamine (1.75 g, 17.31 mmol) in dichloromethane (15 mL) was added methanesulfonic anhydride (1.51 g, 8.65 mmol)at °C, and the reaction solution was stirred at 0-25 °C for 1 hour. The complete consumption of the raw material and the generation of new spots were detected by TLC (petroleum ether: ethyl acetate = 10:1). The reaction was then quenched by dropwise addition of saturated sodium bicarbonate (20 mL), and extracted three times with dichloromethane (20 mL). The combined organic phase was dried, andfiltered. The filtrate was concentrated to dryness under reduced pressure to give a crude product of (4,5-difluoro-1,2-phenylene)bis(ethane-2,1-diyl) dimethanesulfonate (**E-7**) (1.32 g), as a yellow solid, which was used directly in the next step without further purification. $^1$H NMR(400 MHz, $CDCl_3$) δ(ppm) 7.07 (t, 2H), 4.39 (t, 4H), 3.08 (t, 4H), 2.98 (s, 6H)

**Step 6:** To a solution of (4,5-difluoro-1,2-phenylene)bis(ethane-2,1-diyl) dimethanesulfonate (**E-7**) (1.22 g, 3.40 mmol) in 1,2-dichloroethane (20 mL) was added benzylamine (3.65 g, 34.04 mmol), and the reaction solution was stirred at 50 °C for 16 hrs. LCMS showed completion of reaction.Then the reaction solution was added with saturated sodium bicarbonate (15 mL), and extracted twice with dichloromethane (15 mL).The organic phases were combined, dried with anhydrous magnesium sulfate, and filtered. The filtrate was concentrated to dryness under reduced pressure, and then purified by column chromatography (silica, 5% ethyl acetate in petroleum ether) to giveN-benzyl-7,8-difluoro-2,3,4,5-tetrahydro-1H-benzo[d]azepine (**E-8**) (600 mg, 1.98 mmol, 58.04% yield, 90% purity) as a light yellow liquid. LCMS: (ESI) m/z= 273.8 $[M+H]^+$.

**Step 7:** To a solution of N-benzyl-7,8-difluoro-2,3,4,5-tetrahydro-1H-benzo[d]azepine (**E-8**) (600 mg, 2.20 mmol) in hydrochloric acid/methanol (1 ml) in methanol (15 ml) was added 10% palladium/carbon (233.62 mg, 219.52 μmol).The reaction was replaced several times with hydrogen under vacuum, and then stirred under a hydrogen balloon (15 Psi) at 50 °C for 16 hours. LCMS showed the completion of reaction. The reaction solution was concentrated under reduced pressure to remove solvent, to give 7,8-difluoro-2,3,4,5-tetrahydro-1H-benzo[d]azepine (**Intermediate E**) (400 mg hydrochloride crude product) as a white solid, which was used directly in the next step without further purification. LCMS: (ESI) m/z=184.0 $[M+H]^+$, $^1$H NMR (400 MHz, DMSO-$d_6$) δ(ppm)8.83 (br s, 1H),

7.31 (t, 2H), 2.98-3.16 (m, 8H)

**Intermediate Synthesis 6: Synthesis of 2-(6,9-dihydro-5H-benzo[7]cyclopenten-7-yl)-4,4,5,5-tetramethyl-1,3,2-dioxaborolane (Intermediate F):**

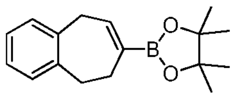

Intermediate F

Intermediate F can be synthetized by referring to the synthesis of Intermediate A. $^1$HNMR (400 MHz, $CHCl_3$) δ (ppm)7.15-7.18 (m, 2H), 7.04-7.09 (m, 2H), 6.62-6.73 (m, 1H), 3.55-3.62 (m, 2H), 3.01-3.07 (m, 2H), 2.46-2.53 (m, 2H), 1.21-1.27 (m, 12H)

**Example 1: Synthesis of (N1-((2-(4,5-dihydro-1H-benzo[d] azepin-3(2H)-yl)-6-methylpyridin-3-yl)methyl)-N1,N2-dimethylethane-1,2-diamine) (Compound P110):**

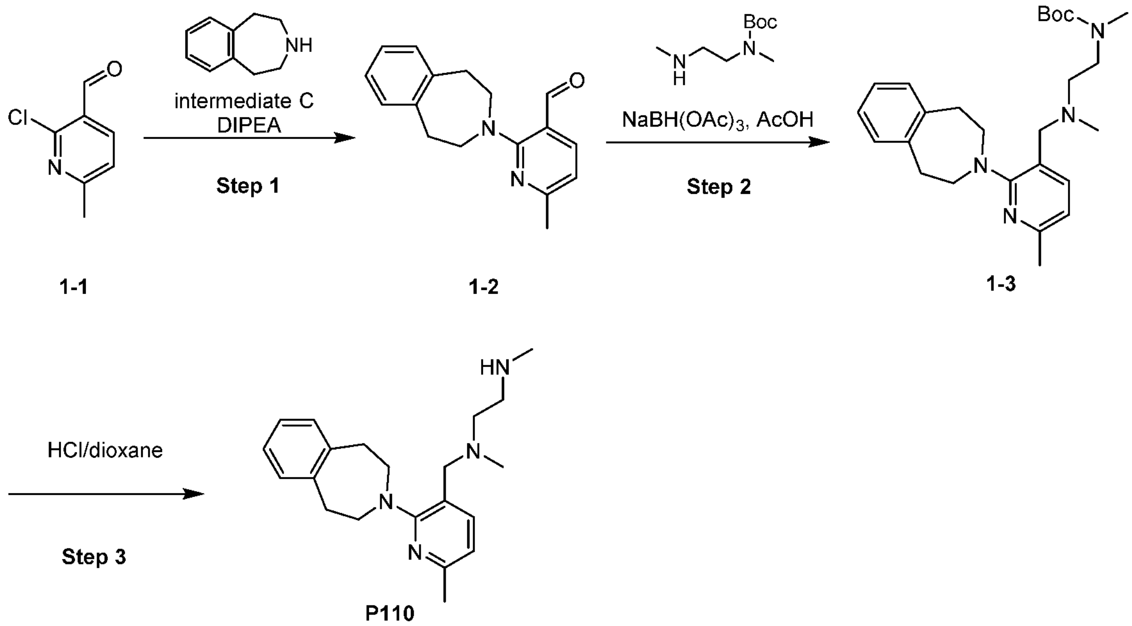


1-1 1-2 1-3

P110
Example 1

Step 1:To a solution of 2-chloro-6-methylnicotinaldehyde (1-1) (100 mg, 643 μmol) and 2,3,4,5-tetrahydro-1H-benzo[d]azepine (**Intermediate C**) (104 mg, 707 μmol) in DMF (2 ml) was added DIPEA (166 mg, 1.29 mmol) under nitrogen protection, and the reaction solution was stirred at 100 °C for 16 hours. LCMS showed completion ofreaction. Then the reaction solution was concentrated and purified by column chromatography (silica gel column, 0-7% ethyl acetate/petroleum ether gradient elution) to give compound 2-(4,5-dihydro-1H-benzo[d]azepin-3(2H)-yl)-6-methylnicotinaldehyde (**1-2**) (90 mg, 314.26 μmol, 48.89% yield, 93% purity) as a yellow solid. LCMS: (ESI) m/z= 267.1 $[M+H]^+$;

Step 2: To a solution of 2-(4,5-dihydro-1H-benzo[d]azepin-3(2H)-yl)-6-methylnicotinaldehyde (**1-2**) (110 mg, 413 μ mol) and tert-butyl methyl (2-(methylamino)ethyl)carbamate (78 mg, 413 μmol) in 10 ML of dichloromethane was added dropwise AcOH (25 mg, 413 μmol.), and the yellow reaction solution was stirred at 20 °C for 2 hours.Then $NaBH(OAc)_3$ (263 mg, 1.24 mmol) was added in batchesand the yellow reaction solution was stirred at 20 °C for 14 h. LCMS showed that the reaction was finished. The reaction was then quenched by dropwise addition of saturated $NaHCO_3$ aqueous solution (20 mL) and extracted with dichloromethane (20 mL × 3). The organic phase was washed with saturated salt water, dried with magnesium sulfate, filtered and concentrated to give a crude product of tert-butyl (2-((2-(4,5-dihydro-1H-benzo[d]azepin-3(2H)-yl)-6-methylpyridin-3-yl)methyl)(methyl)amin o)ethyl)(methyl)carbamate (**1-3**) (150 mg, crude), as a yellow mucus, which was used directly in the next step without further purification. LCMS: (ESI) m/z=439.3 $[M+H]^+$;

**Step 3.**To tert-butyl 2-((2-(4,5-dihydro-1H-benzo[d]azepin-3(2H)-yl)-6-methylpyridin-3-yl)methyl)(methyl)amin o)ethyl)(methyl)carbamate (**1-3**) (150 mg, 342 μmol) was added carefully a solution of HCl/1,4-dioxane (5 mL), and stirred at room temperature for 16 h. LCMS showed completion ofreaction, and the reaction solution was concentrated to obtain acrude product, which was purified by preparative HPLC (column: Boston Green ODS 150×30 mm×5um; mobile phase: [water (0.05%HCl)-ACN]; B%: 0%-38%, 9 min) and collected and lyophilized at low temperature to obtain N1-((2-(4,5-dihydro-1H-benzo[d]azepin-3(2H)-yl)-6-methylpyridin-3-yl)methyl)-N1,N2-dim ethylethane-1,2-diamine (**P110, Example 1**), as a yellow solid (110 mg, 318 μmol, 93.1% yield, 98% purity).LCMS: (ESI) m/z=339.3 $[M+H]^+$; $^1H$ NMR (400 MHz, $D_2O$) δ(ppm) 8.29 (d, 1H), 7.32 (d, 1H), 7.27 (s, 4H), 4.35 (s, 2H), 3.53 (t,4H), 3.39 (s, 4H), 3.16 (t, , 4H), 2.72 (s, 3H), 2.60 (s, 3H), 2.46 (s, 3H)

**Example 2. Synthesis of compound $N^1$,$N^2$-dimethyl-N1-((6-methyl-2-(1-(m-tolyl)piperidin-4-yl)pyridin-3-yl)methyl)ethane-1 ,2-diamine (P081)**

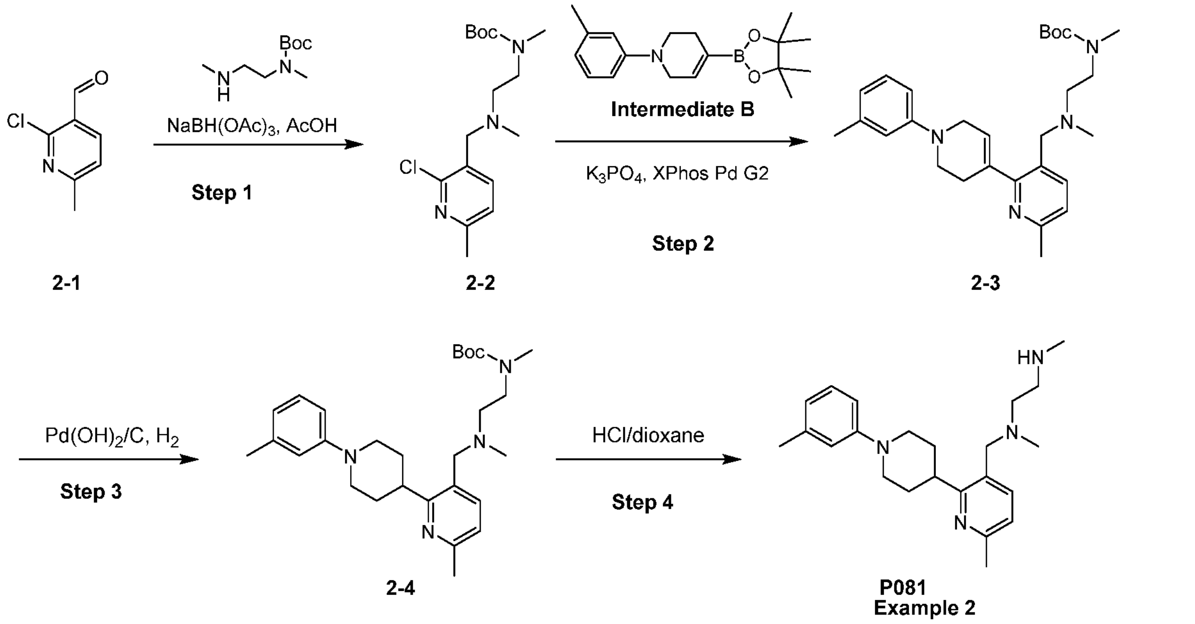


2-1 2-2 2-3 2-4 P081 Example 2

**Step 1:** Compound 2-chloro-6-methylnicotinaldehyde (**2-1**) (10.0 g, 64.3 mmol), tert-butyl methyl (2-(methylamino)ethyl)carbamate (13.3 g, 70.7 mmol) and AcOH (4.25 g, 70.7 mmol) were dissolved in dichloromethane (150 mL) and the reaction was stirred at 25 °C for 2 hours. $NaBH(OAc)_3$ (40.9 g, 1923 mmol) was then added carefully in batches and the reaction continued to stir at room temperature for 16 hours.LCMS showed the disappearance of raw material disappeared. The reaction was then quenched by careful addition of $NaHCO_3$ (300 mL) and extracted with dichloromethane (200 mL × 3). The combined organic phasewasdried with anhydrous magnesium sulfate, filtered, concentrated, then purified by column chromatography (silica gel column, 0-10% ethyl acetate/petroleum ether gradient elution) and concentrated to give a yellow viscous product tert-butyl 2-((2-chloro-6-methylpyridin-3-yl)methyl)(methyl)amino)ethyl)(methyl)carbamate (**2-2**) (13.0 g, 38.4 mmol, 59.8% yield). LCMS: (ESI) m/z = 328.0 $[M+H]^+$; $^1H$ NMR (400 MHz, $CDCl_3$) δ (ppm) 7.65-7.78 (m, 1H), 7.09 (d, 1H), 3.60 (s, 2H), 3.25-3.50 (m, 2H), 2.86 (s, 3H), 2.52-2.65 (m, 2H), 2.52 (s, 3H), 2.30 (s, 3H), 1.35-1.49 (m, 9H)

**Step 2.** To 70 mL of tetrahydrofuran were sequentially added tert-butyl (2-((2-chloro-6-methylpyridin-3-yl)methyl)(methyl)amino)ethyl)(methyl)carbamate (**2-2**) (7.02 g, 21.4 mmol), 4-(4,4,5,5-tetramethyl-1,3,2-dioxaborolan-2-yl)-1-(m-tolyl)-1 ,2,3,6-tetrahydropyridine (**Intermediate B**) (7.05 g, 23.6 mmol), and tripotassium phosphate (9.10 g, 42.9 mmol) aqueous solution (14 mL). The reaction system was depressurized and replaced with nitrogen for several times and added with XPhos-Pd-G2 (510 mg, 648 μmol), then heated in an oil bath under nitrogen protection at 85 °C for 30 h. LCMS showed that the raw material disappeared. After cooling to room temperature, the reaction solution was diluted with saturated saline (50 mL) and ethyl acetate (100 mL). The organic phase was separated, and the aqueous phase was extracted with ethyl acetate.The combined organic phasewasdried with anhydrous

magnesium sulfate, filtered, concentrated, then separated by column chromatography (silica gel column, gradient elution with 0-10-30% of ethyl acetate/dichloromethane), and then concentrated to give tert-butyl methyl(2-(methyl((6-methyl-1'-(m-tolyl)-1 ',2',3',6'-tetrahydro-[2,4'-bipyridin]-3-yl)methyl)amino) ethyl)carbamate (**2-3**) as a yellow viscous liquid (10.9 g, 19.9 mmol, 93.1% yield, 85% purity). LCMS: (ESI) m/z=465.2 $[M+H]^+$; $^1H$ NMR (400 MHz, $CDCl_3$) δ (ppm) 7.70 (d, 1H), 7.18 (t, 1H), 6.99-7.08 (m, 1H), 6.81 (s, 1H), 6.78-6.81 (m, 1H), 6.66 (d, 1H), 5.84-5.93 (m, 1H), 3.86-3.93 (m, 2H), 3.56 (t, 2H), 3.51 (s, 2H), 3.21-3.41 (m, 2H), 2.82 (s, 3H), 2.62-2.71 (m, 2H), 2.54 (s, 3H), 2.39-2.52 (m, 2H), 2.35 (s, 3H), 2.20 (s, 3H), 1.34-1.54 (m, 9H)

**Step 3:** To a solution of tert-butyl methyl(2-(methyl((6-methyl-1'-(m-tolyl)-1',2',3',6'-tetrahydro-[2,4'-bipyridin]-3-yl)methyl)a mino)ethyl)carbamate (**2-3**) (7.84 g, 16.9 mmol) in 250 mL of ethyl acetate was added 10% wet$Pd(OH)_2$/C (3.16 g) under the nitrogen protection, and the system was replaced several times with a hydrogen balloon and then reacted understirring at room temperature for 36 h under hydrogen protection. The completion of reaction was monitored by LCMS. The reaction solution was then filtered through diatomite, and washed with ethyl acetate.The combined organic phases were concentrated, then purified by column chromatography (silica gel column, 0-25% ethyl acetate/dichloromethane gradient elution) to give tert-butyl methyl (2-(methyl ((6-methyl-2-(1-(m-tolyl)piperidin-4-yl)pyridin-3-yl)methyl)amino)ethyl)carbamate (**2-4**), as a yellow mucus (8.86 g, 18.0 mmol, 76.9% yield). LCMS: (ESI) m/z= 467.4 $[M+H]^+$; $^1H$ NMR (400 MHz, $CDCl_3$) δ (ppm) 7.45 (d, 1H), 7.17 (t, 1H), 6.92 (d, 1H), 6.82 (s, 1H), 6.78-6.81 (m, 1H), 6.67 (d, 1H), 3.76-3.86 (m, 2H), 3.50 (s, 2H), 3.23-3.43 (m, 2H), 3.03-3.17 (m, 1H), 2.84 (s, 3H), 2.73-2.83 (m, 2H), 2.49-2.63 (m, 1H), 2.49 (s, 3H), 2.33 (s, 3H), 2.14-2.29 (m, 5H), 1.75-1.84 (m, 2H), 1.33-1.51 (m, 9H)

**Step 4:** To a solution of tert-butyl methyl (2-(methyl((6-methyl-2-(1-(m-tolyl)piperidin-4-yl)pyridin-3-yl)methyl)amino)ethyl)carbamat e (**2-4**) (11.7 g, 25.1 mmol) in methanol (70 ml) was added dropwise HCl/1,4-dioxane (4 M, 75 ml) understirring, and the reaction was stirred under nitrogen protection at room temperature for 16 h. The completion ofreaction was monitored by LCMS. The reaction solution was then concentrated, and added with about 200 mL of methanol, and then spun-dried under reduced pressure.The operation repeated twice to remove hydrochloric acid. The concentrate was diluted with methanol and decolorized by heating with activated charcoal, cooled and filtered at room temperature and washed with methanol, then concentrated to about 100 ml. Isopropanol was added to replace methonal and concentrated to 250 ml. The reaction emulsion was heated at 85 °C for 30 minutes, stirred at 30 °C for 10 hours and cooled to room temperature. Afterfilteration, the solids were washed sequentially with isopropanol (100 mL) and n-pentane (200 mL × 2), and then collected and dried under vacuum. The solid product was dissolved in 150 mL of water, filtered, and the filtrate was lyophilized to give $N^1$,$N^2$-dimethyl-$N^1$-((6-methyl-2-(1-(m-tolyl)piperidin-4-yl)pyridin-3-yl)methyl)ethane-1,2-d iamine (**P081, Example 2**), as a white solid (hydrochloride, 9.8 g, 19.1 mmol, 76.2% yield, 99.9% purity) . LCMS: (ESI) m/z=367.3 $[M+H]^+$; $^1H$ NMR (400 MHz, $D_2O$) δ (ppm) 8.52 (d, 1H), 7.83 (d, 1H), 7.52 (s, 1H), 7.43-7.51 (m, 2H), 7.36-7.42 (m, 1H), 4.72 (s, 2H), 3.93-4.07 (m, 3H), 3.82-3.91 (m, 2H), 3.67-3.76 (m, 2H), 3.55-3.63 (m, 2H), 2.85 (s, 3H), 2.81 (s, 3H), 2.80 (s, 3H), 2.46-2.60 (m, 2H), 2.39 (s, 3H), 2.31-2.39 (m, 2H)

**Example 3. Synthesis of $N^1$-((6-amino-2-(1-(m-tolyl)piperidin-4-yl)pyridin-3-yl)methyl)-$N^1$,$N^2$-dimethylethane-1, 2-diamine (P226)**

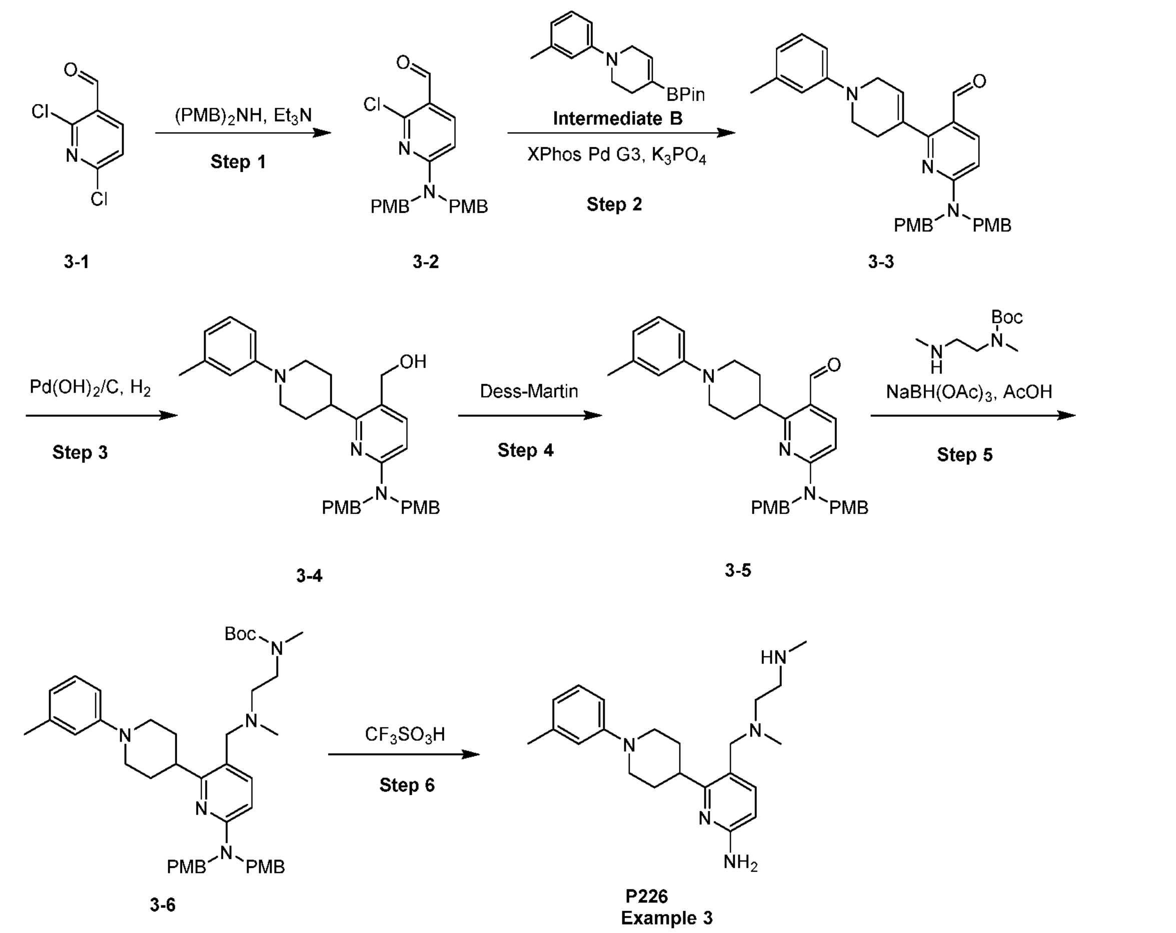


3-1 3-2 3-3 3-4 3-5 3-6

P226
Example 3

**Step 1:** To a solution of 2,6-dichloronicotinaldehyde (**3-1**) (25 g, 142 mmol) and bis(4-methoxybenzyl)amine (40.2 g, 156 mmol) in DMF (200 mL) was added triethylamine (17.2 g, 170 mmol, 23.7 mL), and the reaction solution was stirred at 50 °C for 16 h.The completion of reaction was monitored by LCMS. The mixture was poured into 200 mL of ice water and extracted with ethyl acetate (400 mL × 1, 200 mL × 2). The combined organic phases were washed with saturated salt water, dried with anhydrous magnesium sulfate, filtered, concentrated, and then purified by column chromatography (silica gel column, 0-20% ethyl acetate/(petroleum ether:dichloromethane=5:1) gradient elution) to give compound 6-(bis(4-methoxybenzyl)amino)-2-chloronicotinaldehyde (**3-2**), as a white solid(50 g, 124.83 mmol, 87.88% yield, 99.08% purity). LCMS: (ESI) m/z=397.1 $[M+H]^+$; $^1H$ NMR (400MHz, DMSO-$d_6$), δ (ppm) 10.02 (s, 1H), 7.86 (d, 1H), 7.27 - 7.12 (m, 4H), 6.90 (d, 4H), 6.73 (d, 1H), 4.78 (br, 4H), 3.73 (s, 6H)

**Step 2:** To a reaction flask was added 6-(bis(4-methoxybenzyl)amino)-2-chloronicotinaldehyde (**3-2**) (800 mg, 2.02 mmol), 1-(m-tolyl)-4-(4,4,5,5-tetramethyl-1,3,2-dioxaborolan-2-yl)-3,6-dihydro-2H-pyridine (**Intermediate B**) (1.02 g, 2.40 mmol, 70% purity), tetrahydrofuran (6 mL) and potassium phosphate aqueous solution (856 mg of potassium phosphate dissolved in 1 mL of water) in turn, and the reaction system was depressurized and replaced with nitrogen for several times and then added with XPhos-Pd-G3 (37 mg, 43.71 μmol) under nitrogen protection. The reaction was heated to 70 °C in an oil bath understirring for 2.5 h under nitrogen protection, and the generation of product was monitored by LCMS. After cooling to room temperature, the reaction solution was added with saturated salt waterand extracted three times with ethyl acetate. The organic phases were combined and dried with anhydrous magnesium sulfate, filtered, concentrated, and then separated and purified by column chromatography (silica gel column, 0-10-15% gradient elution of ethyl acetate/petroleum ether) to give a product of 6-(bis(4-methoxybenzyl)amino)-1'-(m-tolyl)-1', 2', 3', 6'-tetrahydro-[2,4'-bipyridyl]-3-carboxaldehyde(**3-3**) (1.01 g, 1.50 mmol, 74.2% yield, 79% purity), as a yellow mucus. LCMS: (ESI) m/z=534.3 $[M+H]^+$;

**Step 3:**To a reaction flask was added6-(bis(4-methoxybenzyl)amino)-1'-(m-tolyl)-1',2',3',6'-tetrahydro-[2,4'-bipyridyl]-3-car boxaldehyde (**3-3**) (801 mg, 1.50 mmol) and ethyl acetate (50 mL), and $Pd(OH)_2/C$ (450 mg, 10% $Pd(OH)_2/C$) was added under nitrogen protection. The reaction system was replaced with hydrogen and then stirred under hydrogen atmosphere (hydrogen balloon)at room temperature for 18 hours. The reaction solution was then filtrated, concentrated and purified by column chromatography (silica gel column, 0-25% ethyl acetate/petroleum ether gradient elution) to give (6-(bis(4-methoxybenzyl)amino)-2-(1-(m-tolyl)piperidin-4-yl)pyridin-3-yl)methanol

(**3-4**), as a yellow mucus (695 mg, 1.23 mmol, 64.9% yield, 95% purity). LCMS: (ESI) m/z=538.3 $[M+H]^+$; $^1H$ NMR (400 MHz, $CDCl_3$) δ (ppm) 7.34 (d, 1H), 7.09-7.24 (m, 5H), 6.77-6.92 (m, 6H), 6.68 (br d, 1H), 6.29 (d, 1H), 4.73 (s, 4H), 4.65 (d, 2H), 3.81 (s, 6H), 3.72-3.81 (m, 1H), 2.99-3.13 (m,1H), 2.75-2.88 (m, 2H), 2.33 (s, 3H), 2.15-2.30 (m, 2H), 1.80-1.92 (m, 2H), 1.40 (t, 1H).

**Step 4:** Compound (6-(bis(4-methoxybenzyl)amino)-2-(1-(m-tolyl)piperidin-4-yl)pyridin-3-yl)methanol (**3-4**) (440 mg, 818 μmol) was added to 10 mL of dichloromethane and Dess-Martin periodinane (347 mg, 818 μmol) was added underan ice bath. The reaction solution was stirred at room temperature for 16 hours.The reaction was quenched by the addition of 20 mL of 10% sodium thiosulfateaqueous solution. After stirring for half an hour, the reaction solution was added with 50 mL of saturated sodium bicarbonate aqueous solution and extracted three times with dichloromethane. The combined organic phases were washed with saturated salt waterand saturated sodium bicarbonate aqueous solution in turn, dried with anhydrous magnesium sulfate, filtered, concentrated, and then separated and purified by column chromatography (silica gel column 0-16.8% ethyl acetate/petroleum ether gradient elution) to give 6-(bis(4-methoxybenzyl)amino)-2-(1-(m-tolyl)piperidin-4-yl)nicotinaldehyde (**3-5**), as a yellow mucus (250 mg , 373 μmol, 45.6% yield). LCMS: (ESI) m/z=536.2 $[M+H]^+$;

**Step 5:** To a reaction flask was added 5 mL of dichloromethane, and then 6-(bis(4-methoxybenzyl)amino)-2-(1-(m-tolyl)piperidin-4-yl)nicotinaldehyde (**3-5**) (250 mg, 466.70μmol), tert-butyl methyl (2-(methylamino)ethyl)carbamate (88 mg, 466μmol), andAcOH (28.03 mg, 466.70 μM) in turn and stirred at room temperature for 2 hours. $NaBH(OAc)_3$ (297 mg, 1.40 mmol) was added in batches, andstirring was continued for 14 hours.The reaction was then quenched by careful dropwise addition of 20 mL of saturated sodium bicarbonate aqueous solution, and extracted with dichloromethane three times after stirring.The combined organic phases were washed with saturated salt water, dried with anhydrous magnesium sulfate,then filtrated, concentrated and purified by column chromatography (silica gel column, 0-17% tetrahydrofuran/petroleum ether gradient elution) to give compound tert-butyl 2-((6-(bis(4-methoxybenzyl)amino)-2-(1-(m-tolyl)piperidin-4-yl)pyridin-3-yl)m-ethyl)(meth yl)amino)ethyl)(methyl)carbamate (**3-6**), as a yellow mucus (60 mg. 83.1 μmol, 17.80% yield, 98% purity). LCMS: (ESI) m/z=708.4 $[M+H]^+$; $^1H$ NMR (400MHz, $CDCl_3$) δ (ppm) 7.24 (d, 1H), 7.10-7.19 (m, 5H), 6.74-6.88 (m, 6H), 6.65 (d, 1H), 6.25 (d, 1H), 4.69 (s, 4H), 3.79 (s, 6 H), 3.70-3.79 (m, 2H), 3.41 (s, 2 H), 3.21-3.41 (m, 2H), 2.98-3.09 (m, 1H), 2.84 (s, 3H), 2.72-2.84 (m, 2H), 2.44-2.59 (m, 2H), 2.31 (s, 3H), 2.09-2.27 (m, 5H), 1.78-1.87 (m, 2H), 1.36-1.49 (m, 9H).

**Step 6:**To a solution of Compound tert-butyl 2-((6-(bis(4-methoxybenzyl)amino)-2-(1-(m-tolyl)piperidin-4-yl)pyridin-3-yl)m-ethyl)(meth yl)amino)ethyl)(methyl)carbamate (**3-6**) (60 mg, 84.7μmol) in 1 mL of trifluoroaceticacidwas addedtrifluoromethanesulfonic acid (29 mg, 194 μmol.), and the reaction was stirred under nitrogen protection at room temperature for 16 hours.The reaction mixture was concentrated, then purified and separated by preparative HPLC (Column: Boston Green ODS 150×30mm×5um; mobile phase: [water (0.05%HCl)-ACN]; B%: 0%-25%, 9 min).The target product was lyophilized to give $N^1$-((6-amino-2-(1-(m-tolyl)piperidin-4-yl)pyridin-3-yl)methyl)-$N^1$,$N^2$-dimethylethane-1,2-di amine (**P226, Example 3**), as a yellow solid (22 mg, 58.7 μmol, 69.2% yield).LCMS: (ESI) m/z=368.3 $[M+H]^+$; $^1H$ NMR (400 MHz, $D_2O$) δ (ppm)7.91 (d, 1H), 7.43-7.51 (m, 2H), 7.36-7.43 (m, 2H), 6.98 (d, 1H), 4.40 (s, 2H), 3.78-3.89 (m, 4H), 3.63-3.72 (m, 1H), 3.49-3.62 (m, 4H), 2.78 (s, 6H), 2.39 (s, 3H), 2.31-2.44 (m, 2H), 2.18-2.30 (m, 2H)

**Example 4. Synthesis of $N^1$,$N^2$-dimethyl-$N^1$-((4-(1-(m-tolyl)piperidin-4-yl)-1H-indazol-5-yl)methyl)ethane-1,2-dia mine (P190):**

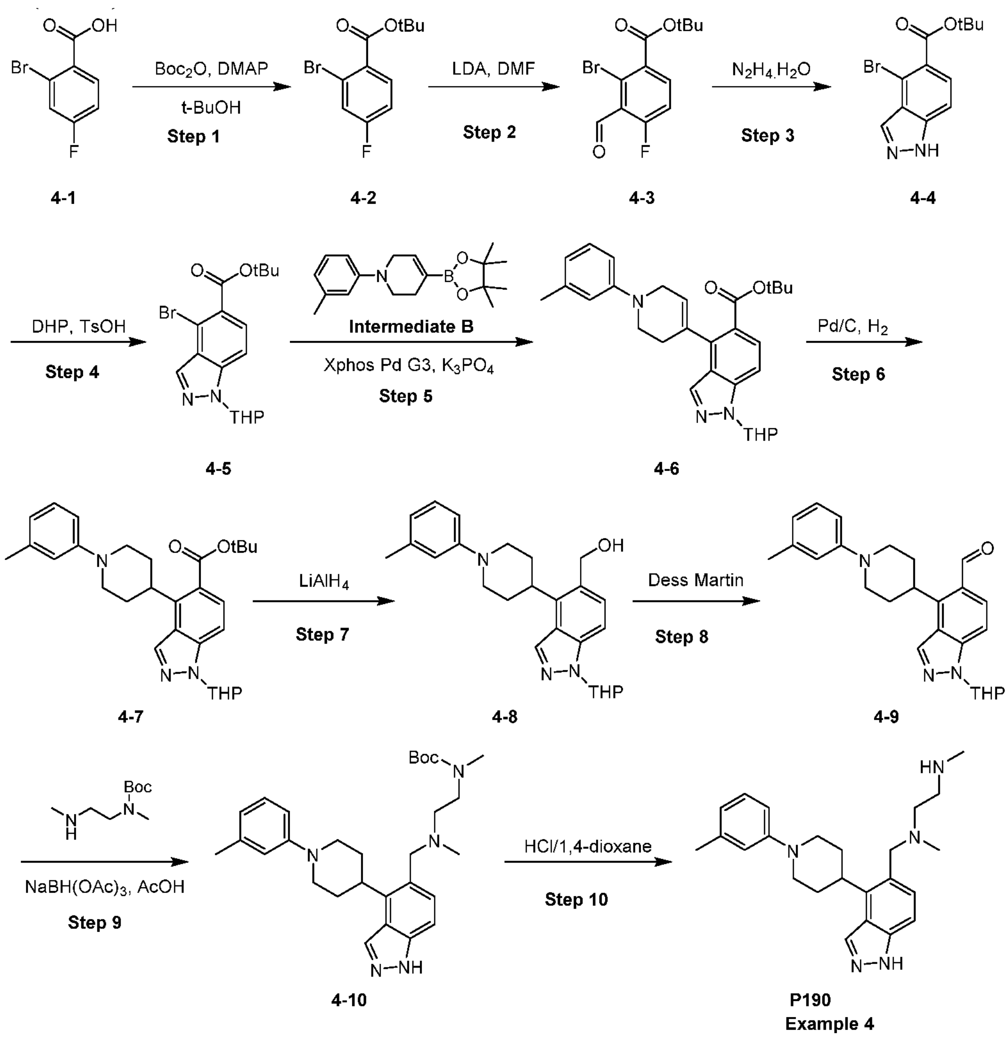


**Step 1:** To a solution of 2-bromo-4-fluorobenzoic acid (**4-1**) (23 g, 105 mmol) in tert-butanol (200 mL) was added 4-dimethylaminopyridine (12.8 g, 105 mmol) and di-tert-butyl dicarbonate (68.8 g, 315 mmol, 3.0 equiv.) at room temperature (vented), and the reaction solutionwas reacted under nitrogen protection at 90 °C for 2 hours. Complete consumption of the raw material and the generation of new spotweredetected by TLC. The reaction solution was then diluted with water (100 mL) and extracted twice with ethyl acetate (150 mL).The organic layer was washed once with saturated salt water(80 mL), dried with magnesium sulfate, andfiltered.The filtrate was concentrated under reduced pressure, and then purified by column chromatography (silica, petroleum ether: ethyl acetate = 10:1) to give tert-butyl 2-bromo-4-fluorobenzoate (**4-2**), as a colorless liquid (26.8 g, 95.0 mmol, 90.4% yield, 97.5% purity).$^1$H NMR (400MHz, $CDCl_3$) δ(ppm) 7.77 (dd, 1H), 7.37 (dd, 1H), 7.02-7.10 (m, 1H), 1.61 (s, 9H).

**Step 2:** Synthesis of lithium diisopropylamide: to a solution of diisopropylamine (5.15 g, 50.9 mmol, 7.19 mL) in tetrahydrofuran (100 mL) was added dropwise n-butyllithium (2.5 M, 17.45 mL) at -70 °C, after addition,the reaction solution was warmed to room temperature and stirred for half an hour to give a yellow solution of diisopropylamide. A solution of tert-butyl 2-bromo-4-fluorobenzoate (**4-2**) (10 g, 36.35 mmol) dissolved in tetrahydrofuran (20 mL) wasadded dropwise to freshly prepared lithium diisopropylamideat -75 °C, and the reaction solution was reacted at -75 °C for 1.5 hours.Dimethylformamide (10.6 g, 145 mmol) was added to the reaction solution at -75 °C to continue to react for 0.5 hours.Complete consumption of the raw material and the generation of new spot were detected by TLC. Then the reaction was quenched with acetic acid (20 mL) at -70 °C, diluted with water (150 mL) and extracted twice with ethyl acetate (200 mL). The organic layer was washed with saturated salt water(100 mL), dried with magnesium sulfate, and filtered. The filtrate was concentrated under reduced pressure, and then purified by column chromatography (silica, 3% ethyl acetate in petroleum ether) to give tert-butyl 2-bromo-4-fluoro-3-formylbenzoate (4-3), as a white solid(8.7 g, 27.7 mmol, 76.2% yield).$^1$H NMR (400MHz, $CDCl_3$) δ(ppm) 10.39 (s, 1H), 7.80 (dd, 1H), 7.19 (t, 1H), 1.63 (s, 9H)

**Step 3:** To a solution of tert-butyl 2-bromo-4-fluoro-3-formylbenzoate (**4-3**) (8.7 g, 28.7 mmol) in ethylene glycol dimethyl ether (100 mL) was added hydrazine hydrate (31.6 g, 537 mmol, 85%, 18.7 equiv.), and the reaction

solution was stirred at 90 °C for 1 hour.Complete consumption of the raw material and the generation of target product were detected by LCMS. The reaction solution was then cooled, diluted with water (100 mL) and extracted twice with ethyl acetate (150 mL). The organic layer was washed with saturated salt water(100 mL), dried with magnesium sulfate, andfiltered. The filtrate was concentrated under reduced pressure, and then purified by column chromatography (silica, 3% ethyl acetate in petroleum ether) to give tert-butyl 4-bromo-1H-indazole-5-carboxylate (**4-4**), as a yellow solid (4.5 g, 15.1 mmol, 52.7% yield). LCMS: (ESI) m/z= 296.9/298.9 $[M+H]^+$;

**Step 4:** To a solution of tert-butyl 4-bromo-1H-indazole-5-carboxylate (**4-4**) (4.5 g, 15.14 mmol) and 3.4 dihydro-2H-pyran (3.82 g, 45.43 mmol, 4.15 ml) in dichloromethane (60 ml) was added p-toluenesulfonic acid monohydrate (288 mg, 1.51 mmol), and the reaction solution was stirred at 15 °C for 1 hour. Complete consumption of the raw material and generation of the target product weredetected by LC-MS. The reaction solution was then diluted with water (50 mL) and extracted twice with ethyl acetate (100 mL).The organic layer was washed with saturated salt water(80 mL), dried with magnesium sulfate, and filtered,. The filtrate was concentrated under reduced pressure, and then purified by column chromatography (silica, 3% ethyl acetate in petroleum ether) to give tert-butyl 4-bromo-1-(tetrahydro-2H-pyran-2-yl)-1H-indazole-5-carboxylate (**4-5**) in a yellow syrup form(4.5 g, 11.6 mmol, 76.7% yield). LCMS: (ESI) m/z=240.9/242.9 $[M+H-THP-tBu]^+$; $^1H$ NMR (400 MHz, $CDCl_3$) $\delta$(ppm) 8.16 (s, 1H), 7.81 (d, 1H), 7.55 (d, 1H), 5.72 (dd, 1H), 3.93-4.03 (m, 1H), 3.67-3.80 (m, 1H), 2.43-2.59 (m, 1H), 2.12-2.21 (m, 1H), 2.02-2.12 (m, 1H), 1.66-1.85 (m, 3H), 1.64 (s, 9H)

**Step 5:** To a solution of tert-butyl 4-bromo-1-(tetrahydro-2H-pyran-2-yl)-1H-indazole-5-carboxylate (**4-5**)(1.0 g, 2.62 mmol, 1.0 equiv.) and 4-(4,4,5,5-tetramethyl-1,3,2-dioxaborolan-2-yl)-1-(m-tolyl)-1,2,3,6-tetrahydropyridine (**Intermediate B**)(863 mg, 2.89 mmol) in tetrahydrofuran(8 mL) was added potassium phosphate(1.67 g, 7.87 mmol)aqueous solution(2 mL). The reaction was degassed under reduced pressure and replaced with nitrogen for multiple times.(2-dicyclohexylphosphine-2,4,6-triaisopropyl-1,1-biphenyl) [2- (2-amino-1,1-biphenyl)] palladium (II) methanesulfonate (XPhos Pd-G3) (67 mg, 78.7 mmol) was added.The reaction solution was stirred at 80°Cfor 16 hours under nitrogen protection. The completion of reaction and generation of new product were detected by LCMS. The reaction solution was diluted with water(30 mL), and extracted with ethyl acetate(20 mL) three times. The organic layer was washed with saturated salt water(20 mL), dried with magnesium sulfate,and filtered. The filtrate was concentrated under reduced pressure, and then purified by column chromatography (silica, 3% ethyl acetate in petroleum ether) to givetert-butyl 1-(tetrahydro-2H--pyran-2-yl)-4-(1-(m-tolyl)-1,2,3,6-tetrahydropyridin-4-yl)-1H-indazole-5-c arboxylate (**4-6**)(800 mg, 1.55mmol, 59.0% yield) in yellow syrup form. LCMS:(ESI) m/z= 474.4 $[M+H]^+$;

**Step 6:**To a solution of tert-butyl 1-(tetrahydro-2H-pyran-2-yl)-4-(1-(m-tolyl)-1,2,3,6-tetrahydropyridin-4-yl)-1H-indazole-5-c arboxylate (**4-6**) (800 mg, 1.69 mmol) in methanol (160 ml) was added 10% Pd/C (150 mg), and the reaction solution was degassed under reduced pressure and replaced with hydrogen several times, then stirred at 50 °C for 32 h under hydrogen balloon atmosphere. The generation of target product was detected by LCMS. The reaction solution was then filtrated, and the filtrate was concentrated to dryness under reduced pressure, then purified by column chromatography (silica, 30% ethyl acetate in petroleum ether) to give tert-butyl 1-(tetrahydro-2H-pyran-2-yl)-4-(l-(m-tolyl)piperidin-4-yl)-1H-indazole-5-carboxylate (**4-7**) in a yellow syrup form (630 mg, 1.24 mmol, 73.2% yield). LCMS: (ESI) m/z= 476.3 $[M+H]^+$; RT=0.857 min

$^1H$ NMR (400 MHz, $CDCl_3$) $\delta$(ppm) 8.31 (s, 1H), 7.64 (d, 1H), 7.45 (d, 1H), 7.19 (t, 1H), 6.80-6.91 (m, 2H), 6.71 (d1H), 5.71 (dd, 1H), 3.99-4.07 (m, 1H), 3.86-3.94 (m, 2H), 3.65-3.79 (m, 2H), 2.82-3.93 (m, 2H), 2.51-2.65 (m, 1H), 2.37-2.49 (m, 2H), 2.35 (s, 3H), 2.11-2.22 (m, 1H), 2.04-2.10 (m, 1H), 1.93-2.02 (m, 2H), 1.71-1.85 (m, 2H), 1.64-1.71 (m, 1H), 1.63 (s, 9H)

**Step 7:** To a solution of tert-butyl 1-(tetrahydro-2H-pyran-2-yl)-4-(1-(m-tolyl)piperidin-4-yl)-1H-indazole-5-carboxylate (**4-7**) (630 mg, 1.32 mmol) in tetrahydrofuran (6 ml) was added dropwise lithium aluminiumhydride (1 M in THF, 5.5 ml) at -5 °C, and the reaction solution was stirred at 20 °C for 3 hours. The complete consumption of the material and the generation of new spots were detected by TLC (petroleum ether / ethyl acetate = 1:1). The reaction solution was then quenched dropwise with water (10 mL) at -5 °C, diluted with water (5 mL) and extracted twice with ethyl acetate (30 mL). The organic layer was washed with saturated salt water(20 mL), dried with magnesium sulfate, and filtered.The filtrate was concentrated to dryness under reduced pressure, then purified by column chromatography (silica, 50% ethyl acetate in petroleum ether) to give (1-(tetrahydro-2H-pyran-2-yl)-4-(1-(m-tolyl)piperidin-4-yl)-1H-indazol-5-yl)methanol (**4-8**), as a white solid(500 mg. 849 $\mu$mol, 64.1% yield). LCMS: (ESI) m/z= 406.3 $[M+H]^+$;

**Step 8:** To a solution of (1-(tetrahydro-2H-pyran-2-yl)-4-(1-(m-tolyl)piperidin-4-yl)-1H-indazol-5-yl)methanol (**4-8**) (200 mg, 493.18 $\mu$mol) in dichloromethane (10 ml) was added Dess-Martin periodinane (220 mg, 518 $\mu$mol), and the reaction solution was reacted at 25 °C for 6 hours. The complete consumption of the raw materialand generation of target product were detected by LC-MS. The reaction solution was then quenchedwith 10% sodium thiosulfate solution (20 mL) and stirred at room temperature for 0.5 h, then added with saturated sodium bicarbonate (50 mL) and extracted three times with dichloromethane (50 mL).The organic layer was washed with saturated salt water(50 mL), dried with magnesium sulfate, and filtered.The filtrate was concentrated to dryness under reduced pressure, and then purified by column chromatography (silica, 30% ethyl acetate in petroleum ether) to give 1-(tetrahydro-2H-pyran-2-yl)-4-(1-(m-tolyl)piperidin-4-yl)-1H-indazole-5-carboxaldehyde (**4-9**) in a yellow syrup form (140 mg. 293 μmol, 59.4% yield). LCMS: (ESI) m/z= 404.3 $[M+H]^+$;

**Step 9:** To a solution of 1-(tetrahydro-2H-pyran-2-yl)-4-(1-(m-tolyl)piperidin-4-yl)-1H-indazole-5-carboxaldehyde (**4-9**) (200 mg, 496 μmol) and tert-butyl methyl (2-(methylamino)ethyl)carbamate (112 mg, 595 μmol) in dichloromethane (5 ml) was added acetic acid (36 mg, 595 μmol), and the reaction solution was stirred at 20 °C for 2 hours. Then sodium triacetoxyborohydride (315 mg, 1.49 mmol, 3 equiv.) was added, and the reaction solution was stirred at 20 °C for 14 hours. The complete consumption of raw materials and generation of target product weredetected by LC-MS. The reaction solution was then quenched with saturated sodium bicarbonate solution (10 mL), diluted with water (5 mL), extracted twice with dichloromethane (20 mL), dried with magnesium sulfate, and filtered.The filtrate was concentrated to dryness under reduced pressure, and then purified by column chromatography (silica, 50% ethyl acetate in dichloromethane) to give tert-butyl methyl (2-(methyl((1-(tetrahydro-2H-pyran-2-yl)-4-(1-(m-tolyl)piperidin-4-yl)-1H-indazol-5-yl)met hyl)amino)ethyl)carbamate (**4-10**) (210 mg, 365 μmol, 73.6% yield) in a yellow syrup form. LCMS: (ESI) m/z= 576.5 $[M+H]^+$;

**Step 10:**To a solution of tert-butyl methyl (2-(methyl((1-(tetrahydro-2H-pyran-2-yl)-4-(1-(m-tolyl)piperidin-4-yl)-1H-indazol-5-yl)met hyl)amino)ethyl)carbamate (**4-10**) (210 mg, 365 μmol) in methanol (2 ml) was added HCl/1,4-dioxane (4 M, 2.10 mL), and the reaction solution was stirred at 25 °C for 16 hours. The complete consumption of raw material and the generation of target product were detected by LC-MS. The reaction solution was concentrated to dryness under reduced pressure, and then purified by preparative liquid chromatography (Boston Green ODS column, Sum silica, 30 mm diameter, 150 mm length; a mixture of water (containing 0.05% hydrochloric acid) and acetonitrile withdecreasing polarity was used as eluent) to give N1,N2-dimethyl-N1-(((4-(1-(m-tolyl)piperidin-4-yl))) -1H-indazol-5-yl)methyl)ethane-1,2-diamine (**P190, Example 4**) as a white solid (hydrochloride, 105 mg, 245 μmol, 67.3% yield). LCMS: (ESI) m/z= 392.2 $[M+H]^+$; $^1$H NMR (400 MHz, $D_2O$) δ(ppm) 8.63 (s, 1H), 7.61 (d, 1H), 7.54 (s, 1H), 7.42-7.51 (m, 3H), 7.36 (d, 1H), 4.74 (s, 2H), 3.86-4.02 (m, 2H), 3.65-3.84 (m, 5H), 3.52-3.64 (m, 2H), 2.87 (s, 3H), 2.70-2.94 (m, 2H), 2.78 (s, 3H), 2.37 (s, 3H), 2.09-2.19 (m, 2H)

**Example 5. Synthesis of N1-((4-(2,3-difluoro-6,7,8,9-tetrahydro-5H-benzo[7]cyclopenten-7-yl)-3-methyl-1H-indazol-5-yl)methyl)-N1,N2-dimethylethane-1,2-diamine (P146):**

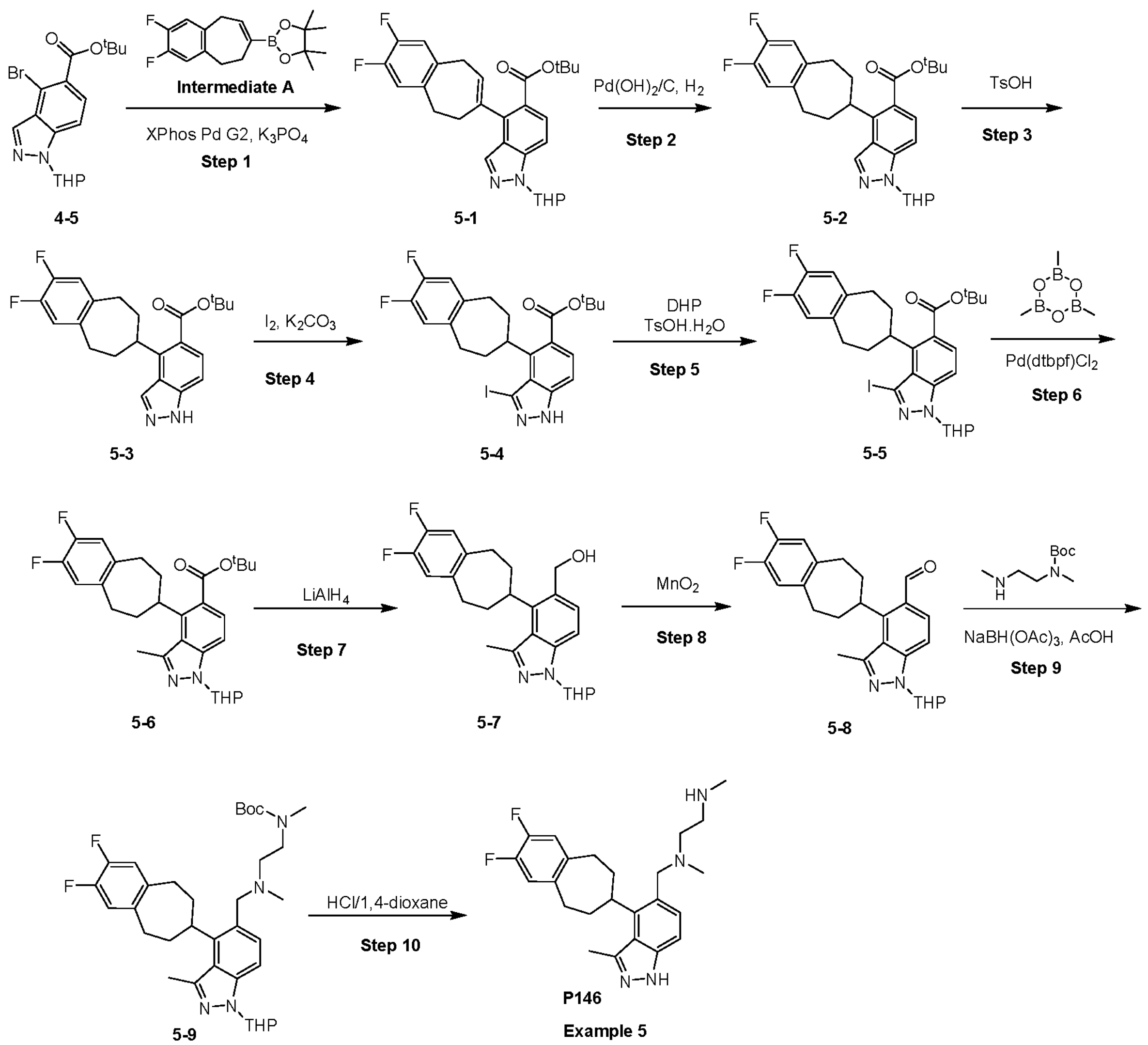


**Step 1:** To a reaction flask was added a solution of tert-Butyl 4-bromo-1-(tetrahydro-2H-pyran-2-yl)-1H-indazole-5-carboxylate (**4-5**) (1.95 g, 5.11 mmol), (2,3-difluoro-6,9-dihydro-5H-benzo[7]cyclopentan-7-yl)-4,4,5,5-tetramethyl-1,3,2-dioxaboro lane (**Intermediate A**) (1.80 g, 5.88 mmol, 1.15 equiv), toluene (45 ml) and potassium phosphate (2.18 g, 10.3 mmol) in water (6.5 mL), and the reactionwas replaced with nitrogen several times, and then added withchloro(2-dicyclohexylphosphino-2,4,6-triisopropyl-1,1 -biphenyllene)[2-(2-amino-1,1 -bi phenyl)]palladium(II) ( 0.03 equiv).The reaction solution was stirred at 60 °C for 24 hours under nitrogen protection. The generation of target product was detected by LCMS. The reaction solution was then cooled to 25 °C, dissolved with water (50 mL) and ethyl acetate (50 mL).The organic phase was separated and then extracted three times withethyl acetate (50 mL).The organic phases were combined, dried with magnesium sulfate, and filtered.The filtrate was concentrated to dryness under reduced pressure, then purified by column chromatography (silica, 15% ethyl acetate in petroleum ether) to give a crude product of tert-butyl 4-(2,3-difluoro-6,9-dihydro-5H-benzo[7]cyclopenten-7-yl)-1-(tetrahydro-2H-pyran-2-yl)-1H-indazole-5 -carboxylate (**5-1**), as a yellow oil. LCMS: (ESI) m/z= 481.2 $[M+H]^+$;

**Step 2:** The crude product of the previous step, tert-butyl 4-(2,3-difluoro-6,9-dihydro-5H-benzo[7]cyclopenten-7-yl)-1-(tetrahydro-2H-pyran-2-yl)-1H-indazole-5 -carboxylate (**5-1**), was dissolved in ethyl acetate (50 mL) and palladium hydroxide/carbon (520 mg, 10%) was added under nitrogen atmosphere.The reaction solution was replaced several times with hydrogen and stirred for 12 h at 30 °C. The consumption of the raw material and the generation of target product were detected by LCMS. The reaction solution was then filtered to give tert-butyl 4-(2,3-difluoro-6,7,8,9-tetrahydro-5H-benzo[7]cyclopenten-7-yl)-1-(tetrahydro-2H-pyran-2-y l)-1H -indazole-5-carboxylate (**5-2**) as a white solid, which was used directly inthe next step without further purification. LCMS: (ESI) m/z=399.3 $[M+H-THP]^+$; $^1H$ NMR (400 MHz, $CDCl_3$) δ(ppm) 7.98 (s, 1H), 7.64 (d, 1H), 7.43 (d, 1H), 6.94-7.05 (m, 2H), 5.70 (dd, 1H), 3.98-4.07 (m, 1H), 3.89-3.98 (m, 1H), 3.69-3.80 (m, 1H), 2.91-3.06 (m, 2H), 2.76-2.91 (m, 2H), 2.46-2.59 (m, 1H), 2.11-2.23 (m, 3H), 1.95-2.10 (m, 3H), 1.66-1.84 (m, 4H), 1.65 (s, 9H)

**Step 3:** To a solution of tert-butyl 4-(2,3-difluoro-6,7,8,9-tetrahydro-5H-benzo[7]cyclopenten-7-yl)-1-(tetrahydro-2H-pyran-2-y l)-1H -indazole-5-carboxylate (**5-2**) (1.9 g, 3.94 mmol) in methanol (30 ml) and tetrahydrofuran (30 ml) was added p-toluenesulfonic acid monohydrate (297 mg , 1.56 mmol), and the reaction solution was stirred at 30 °C for 3.5 days. The generation of target product and the raw material remained were detected by LCMS. The reaction solution was quenched with saturated sodium bicarbonate solution (20 mL) and concentrated under reduced pressure to remove methanol. Then ethyl acetate (100 mL) and water (50 mL) were added, and the organic phase was separated and extracted twice with ethyl acetate (50 mL). The organic phases were combined and dried with anhydrous magnesium sulfate, and filtered. The filtrate was concentrated to dryness under reduced pressure, then purified by thin layer chromatography (silica, 35% ethyl acetate in petroleum ether), then concentrated to dryness under reduced pressure to give tert-butyl 4-(2,3-difluoro-6,7,8,9-tetrahydro-5H-benzo[7]cyclopenten-7-yl)-1H-indazole-5-carboxylate (**5-3**) as a white solid (827 mg, 2.08 mmol, 52.7% yield). LCMS: (ESI) m/z=399.2 $[M+H]^+$; $^1H$ NMR (400 MHz, $CDCl_3$) δ(ppm) 8.06 (s, 1H), 7.65 (d, 1H), 7.34 (d, 1H), 7.00 (t, 2H), 3.90-4.02 (m, 1H), 2.92-3.06 (m, 2H), 2.76-2.90 (m, 2H), 2.14-2.27 (m, 2H), 1.98-2.11 (m, 2H), 1.64 (s, 9H)

**Step 4:**To a solution of tert-butyl 4-(2,3-difluoro-6,7,8,9-tetrahydro-5H-benzo[7]cyclopenten-7-yl)-1H-indazole-5-carboxylate (**5-3**) (424 mg, 1.06 mmol) in dimethylformamide (10 ml,prerviouslyadded with potassium carbonate (458 mg, 3.31 mmol) was added iodine (459 mg, 1.81 mmol), and the reaction solution was stirred at 15 ~20 °C for 16 hours. LCMS indicated69% of the target product and30% of the remaining raw material. Additional iodine (135 mg, 532 μmol) and potassium carbonate (180 mg, 1.30 mmol) were added to the reaction solution and stirring was continued at 15 ~30 °C for 3 hours. LCMS indicated 10% of the remaining raw material and the generation of target product. It was combined with the previous 50 mg of the crude product for process. Ethyl acetate (50 mL) and 10 % sodium thiosulfate solution (50 mL) were added to the reaction solution, the organic phase was separated, and the aqueous phase was extracted with ethyl acetate (50 mL) three times. The organic phases were combined and washed once with 10% sodium thiosulfate solution, three times with 10 % lithium chloride (25 mL), and once with saturated salt water(50 mL), dried, filtered, and concentrated under reduced pressure to give the crude product tert-butyl 4-(2,3-difluoro-6,7,8,9-tetrahydro-5H-benzo[7]cyclopenten-7-yl)-3-iodo-1H-indazole-5-carb oxylate (**5-4**) as a lightyellow foam, which was used directly in the next step without further purification. LCMS: (ESI) m/z= 525.0 $[M+H]^+$;

**Step 5:** To a solution of tert-butyl 4-(2,3-difluoro-6,7,8,9-tetrahydro-5H-benzo[7]cyclopenten-7-yl)-3-iodo-1H-indazole-5-carb oxylate (**5-4**) (589 mg, 1.12 mmol.) and 3,4-dihydropyran (420 mg, 4.99 mmol.) in dichloromethane (15 ml.)was added p-toluenesulfonic acid (50 mg, 263μmol, 0.23 equiv.) underan ice bath at a temperature of 0 to 5 °C, and the reaction solution was stirred for 3 hours at 15-20 °C. The complete consumption of raw materialand the generation of target product were detected by LCMS. The reaction solution was then partitionedin 5 % sodium bicarbonate solution (50 mL) and dichloromethane (50 mL).The organic phase was separated and the aqueous phase was extracted twice with dichloromethane (50 mL). The organic phases were combined and dried with magnesium sulfate, filtered and concentrated to dryness under reduced pressure, and then purified by column chromatography (silica, 12% ethyl acetate in petroleum ether) to give tert-butyl 4-(2,3-difluoro-6,7,8,9-tetrahydro-5H-benzo[7]cyclopenten-7-yl)-3-iodo-1-(tetrahydro-2H-py ran-2- -1)-indazole-5-carboxylate (**5-5**) as a white solid (564 mg, 742μmol, 66.0%yield, 80% purity). LCMS: (ESI) m/z=525.1 $[M+H-THP]^+$; $^1H$ NMR (400 MHz, $CDCl_3$) δ(ppm) 7.46 (d, 1H), 7.40 (d, 1H), 6.97 (t, 2H), 5.67 (dd, 1H), 4.52-4.67 (m, 1H), 3.95-4.03 (m, 1H), 3.67-3.80 (m, 1H), 3.05-3.16 (m, 2H), 2.80 (dd, 2H), 2.42-2.62 (m, 1H), 2.04-2.27 (m, 6H), 1.68-1.79 (m, 3H), 1.27-1.44 (m, 9H)

**Step 6:** Two reactions were set up in parallel: tert-butyl 4-(2,3-difluoro-6,7,8,9-tetrahydro-5H-benzo[7]cyclopenten-7-yl)-3-iodo-1-(tetrahydro-2H-py ran-2- -1 )-indazole-5-carboxylate (**5-5**) (225 mg, 370 μmol, 1.0 eq.), 1,4-dioxane (5 mL) and potassium phosphate (236 mg, 1.11 mmol) were dissolved in water (1 mL), and the reaction solution was replaced with nitrogen, added with 2,4,6-trimethyl-1,3,5,2,4,6-trioxatriborinane (292 mg, 1.16 mmol, 50% in THF) and [1,1-bis(di-tert-butylphosphino)ferrocene]palladium dichloride (17 mg, 26.1 μmol, 0.07 eq.).Then the reaction was stirred at 80 °C for 2 hours under nitrogen protection.The complete consumption of raw materialand the generation of target product were detected by LCMS. The reaction solution was cooled to 20 °C and partitioned in saturated salt water(50 mL) and ethyl acetate (50 mL), the organic layer was separated and the aqueous phase was extracted twice with ethyl acetate (50 mL). The organic phases were combined, dried with magnesium sulfate, filtered and concentrated to dryness under reduced pressure, then purified by column chromatography (silica, 0-5%-7% ethyl acetate in petroleum ether) to give a deiodinated by-product tert-butyl 4-(2,3-difluoro-6,9-dihydro-5H-benzo[7]cyclopenten-7-yl)-1-(tetrahydro-2H-pyran-2-yl)-1H-indazole-5 -carboxylate (**5-1** ) as a bright yellow oil (124 mg, 257 μmol, 34.7% yield) and the target product tert-butyl 4-(2,3-difluoro-6,7,8,9-tetrahydro-5H-benzo[7]cyclopenten-7-yl)-3-methyl-1-(tetrahydro-2H-pyran-2- -1)-indazole-5-carboxylate (**5-6**) as a bright yellow oil (244 mg, 442 μmol, 59.8% yield). LCMS: (ESI) m/z=497.2 $[M+H]^+$; $^1H$ NMR (400 MHz, $CDCl_3$) δ(ppm) 7.38 (d, 1H), 7.33 (d, 1H), 6.97 (t, 2H), 5.60 (dd, 1H), 4.02-4.09 (m, 1H), 3.81-3.92 (m, 1H), 3.68-3.78 (m, 1H), 2.87-2.99 (m, 2H), 2.80-2.87 (m, 2 H), 2.80 (s, 3H), 2.48-2.61 (m, 1H), 2.19-2.33 (m, 2H), 2.06-2.18 (m, 3H), 1.95-2.04 (m, 1H), 1.70-1.84 (m, 2H), 1.62-1.70 (m, 2H), 1.58 (s, 9H).

**Step 7:** To a reaction flask was added tert-butyl 4-(2,3-difluoro-6,7,8,9-tetrahydro-5H-benzo[7]cyclopenten-7-yl)-3-methyl-1-(tetrahydro-2H-pyran-2- -1)-indazole-5-carboxylate (**5-6**) (239 mg, 481 μmol) and tetrahydrofuran (8 ml), and Lithium tetrahydro aluminum (1 M tetrahydrofuran solution, 0.9 mL) was added at 15-2 °C, then the reaction solution continued to stir for 2 hours. TLC detected that the raw materialwas still present and new spots were formed. Lithium tetrahydro aluminum (1 M tetrahydrofuran, 300 μl) was continued to be added and the reaction solution was stirred for 1 hour at 15-20 °C. TLC (petroleum ether: Ethyl acetate = 3:1) detected that most of the raw materialwas consumed and new spots were formed. The reaction solution was quenched with water (50 mL) and 15% sodium hydroxide solution, dissolved with ethyl acetate (50 mL), dried with magnesium sulfate, and stirred for 10 minutes,then filtered, concentrated to dryness under reduced pressure to give a crude product of (4-(2,3-difluoro-6,7,8,9-tetrahydro-5H-benzo[7]cyclopenten-7-yl)-3-methyl-1-(tetrahydro-2H -pyran-2-yl)-1H-indazol-5-yl)methanol (**5-7**) (221 mg, 518 μmol) as a yellow solid. The crude product was used directly in the next step without further purification. LCMS: (ESI) m/z= 427.3 $[M+H]^+$;

**Step 8:** To a solution of (4-(2,3-difluoro-6,7,8,9-tetrahydro-5H-benzo[7]cyclopenten-7-yl)-3-methyl-1-(tetrahydro-2H -pyran-2-yl)-1H-indazol-5-yl)methanol (**5-7**) (210 mg, 492 μmol) in dichloromethane (15 ml) was added manganese dioxide (530 mg, 6.10 mmol). The reaction solution was stirred at 40 °C for 3.5 hours and then filtered and concentrated under reduced pressure to give a crude product of 4-(2,3-difluoro-6,7,8,9-tetrahydro-5H-benzo[7]cyclopenten-7-yl)-3-methyl-1-(tetrahydro-2H-pyran-2-yl)-1H-indazole-5-carboxaldehyde (**5-8**) (210 mg, 495μmol) as anoff -white solid. The crude product was used directly in the next step without further purification. LCMS: (ESI) m/z= 425.1 $[M+H]^+$;

**Step 9:**A solution of 4-(2,3-difluoro-6,7,8,9-tetrahydro-5H-benzo[7]cyclopenten-7-yl)-3-methyl-1-(tetrahydro-2H-pyran-2-yl)-1H-indazole-5-carboxaldehyde (**5-8**) (204 mg, 480μmol), tert-butyl methyl (2-(methylamino)ethyl)carbamate (186 mg, 987μmol) and acetic acid (62 mg, 1.03 mmol, 2.15 equiv.) in dichloromethane (3 mL) wasstirred at 25 °C for 2 h, then sodium triethoxycarbonylborohydride (340 mg, 1.60 mmol) was added.The reaction solution was stirred at 25 °C for 14 hours.The consumption of the raw materialand the generation of target product weredetected by LCMS. The reaction solution was partitionedin 5 % sodium bicarbonate solution (15 mL) and dichloromethane. The organic phase was separated and the aqueous phase was extracted three times with ethyl acetate (10 mL). The organic phases were combined, dried with magnesium sulfate, filtered, concentrated to dryness under reduced pressure, then purified by column chromatography (silica, 30% ethyl acetate in dichloromethane) to give tert-butyl (2-((4-(2,3-difluoro-6,7,8,9-tetrahydro-5H-benzo[7]cyclopenten-7-yl)-3-methyl-1-(tetrahydro -2H-pyran-2-yl)-1H- indazol-5-yl)methyl)(methyl)amino)ethyl)(methyl)carbamate (**5-9**) (204 mg, 325 μmol, 67.6% yield) as a bright yellow oil. LCMS: (ESI) m/z=597.4 $[M+H]^+$;

**Step 10:**To a solution of tert-butyl (2-((4-(2,3-difluoro-6,7,8,9-tetrahydro-5H-benzo[7]cyclopenten-7-yl)-3-methyl-1-(tetrahydro -2H-pyran-2-yl)-1H- indazol-5-yl)methyl)(methyl)amino)ethyl)(methyl)carbamate (**5-9**) (202mg, 338μmol) in methanol was added HCl/1,4-dioxane (4 M, 15 mL), and the reaction solution was stirred at 10-15°C for 12 hours. The complete consumption of raw material and the generation of target product were detected by LC-MS. The reaction solution was then concentrated to dryness under reduced pressure, then purified by reversed phase preparative liquid phase chromatography (YMC-ActusTriart C18 column, Sum silica, 30 mm diameter, 150 mm length; a mixture of water (containing 0.05% ammonia) and acetonitrile with decreasing polarity was used as eluent) to give N1-((4-(2,3-difluoro-6,7,8,9-tetrahydro-5H-benzo[7]cyclopenten-7-yl)-3-methyl-1H-indazol-5-yl)methyl-N1,N2-dimethylethane-1,2-diamine (**P146, Example 5**) as a white solid (Hydrochloride, 105 mg, 214 μmol, 63.3% yield, 99% purity). LCMS: (ESI) m/z=413.4 $[M+H]^+$; $^1H$ NMR (400 MHz, $CD_3OD$) tautomer mixture δ (ppm) 7.84 (d, 0.5H), 7.71 (d, 0.5H), 7.49-7.55 (m, 1H), 7.08-7.17 (m, 2H), 4.74-4.84 (m, 1H), 4.36-4.74 (m, 1H), 4.08-4.20 (m, 0.5H), 3.74-3.87 (m, 1.5H), 3.63-3.72 (m, 1H), 3.43-3.63 (m, 2H), 3.29-3.37 (m, 1H) 2.99-3.05 (m, 2H), 2.96 (s, 1.5H), 2.83-2.87 (m, 1H), 2.83 (s, 1.5H), 2.81 (s, 3H), 2.74 (s, 1.5H), 2.26 (s, 1.5H), 2.09-2.26 (m, 2H), 1.84-2.08 (m, 2H); $^{19}F$ NMR (376 MHz, $CD_3OD$) tautomer mixture δ(ppm) -144.96, -145.32

**Example 6: Synthesis of $N^1$,$N^2$-dimethyl-$N^1$-((3-(7-(trifluoromethyl)-4,5-dihydro-1H-benzo[d]azepin-3(2H)-yl)pyr idin-2-yl)methyl)ethane- 1,2-diamine (P088)**

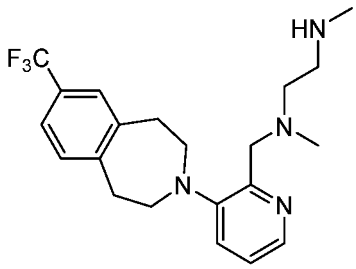

**P088**

**Example 6**

Compound P088 was synthesized as described in Example 1 using3-fluoropyridinecarboxaldehyde and Intermediate D. LCMS: (ESI) m/z=393.4 [M+H]$^+$; $^1$H NMR (400 MHz, $D_2O$) δ(ppm) 8.25 (d, 1H), 7.91 (d, 1H), 7.61 (dd, 1H), 7.50 (s, 1H), 7.47 (d, 1H), 7.32 (d, 1H), 4.24 (s, 2H), 3.28-3.36 (m, 2H), 3.15-3.20 (m, 2H), 3.06-3.13 (m, 8H), 2.71 (s, 3H), 2.43 (s, 3H)

**Example 7: Synthesis of N1-((4-(4,4-bis(ethoxymethyl)cyclohexyl)-1H-indazol-5-yl)methyl)-N1,N2-dimethylethan e-1,2-diamine (P266)**

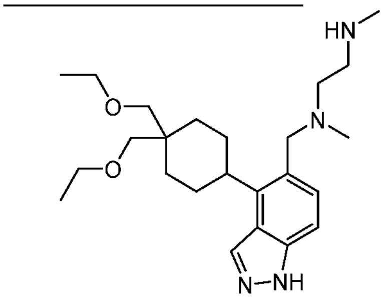

**P266**

**Example 7**

Compound **P266** was synthesized as described in **Example 4**usingintermediate**4-4** and 2-(4,4-bis(ethoxymethyl)cyclohex-1-en-1-yl)-4,4,5,5-tetramethyl-1,3,2-dioxaborolane. LCMS: (ESI) m/z=417.4 [M+H]$^+$; $^1$H NMR (400 MHz, $D_2O$) δ(ppm) 8.30 (s, 1H), 7.52 (d, 1H), 7.39 (d, 1H), 4.60 (s, 2H), 3.65 (s, 2H), 3.62 (q, 2H), 3.58-3.65 (m, 2H), 3.55 (q, 2H), 3.48-3.57 (m, 2H), 3.30 (s, 2H), 2.92-3.05 (m, 1H), 2.82 (s, 3H), 2.75 (s, 3H), 1.97-2.14 (m, 2H), 1.63-1.75 (m, 2H), 1.48-1.58 (m, 2H), 1.33-1.46 (m, 2H), 1.20 (t, 3H), 1.16 (t, 3H)

**Example 8.Svnthesis of N1-((4-(2,3-difluoro-6,7,8,9-tetrahydro-5H-benzo[7]cyclonenten-7-yl)-1H-indazol-5-yl)methyl)-N1,N2 -dimethylethane-1,2-diamine (P167)**

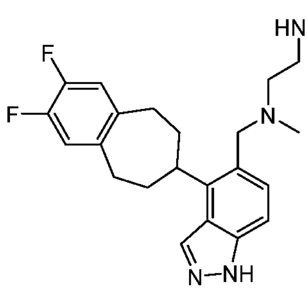

**P167**

**Example 8**

Compound **P167** was synthesized as described in **Example 4**usingIntermediate**4-5** and **Intermediate A**. LCMS: (ESI) m/z=399.2 $[M+H]^+$; $^1H$ NMR (400 MHz, $D_2O$) δ(ppm) 8.05 (s, 1H), 7.56 (d, 1H), 7.45 (d, 1H), 7.05 (t, 2H), 4.71 (s, 2H), 3.65-3.78 (m, 2H), 3.54-3.63 (m, 2H), 3.39-3.52 (m, 1H), 2.95-3.08 (m, 2H), 2.90 (s, 3H), 2.75-2.85 (m, 2H), 2.80 (s, 3H) 1.88-2.12 (m, 4H)

**Example 9. Synthesis of N1-((6-amino-2-(4,4-bis(ethoxymethyl)cyclohexyl)pyridin-3-yl)methyl)-N1,N2-dimethylethane-12-diamine (P293)**

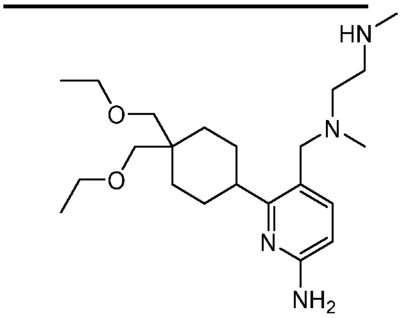

**P293**
**Example 9**

Compound **P293** was synthesized as described in**Example 3**, usingintermediate**3-2** and 2-(4,4-bis(ethoxymethyl)cyclohex-1-en-1-yl)-4,4,5,5-tetramethyl-1,3,2-dioxaborolane. LCMS: (ESI) m/z=393.3 $[M+H]^+$; $^1H$ NMR (400 MHz, $CD_3OD$) δ(ppm)8.41 (s, 2H), 7.61 (d, 1H), 6.57 (d, 1H), 3.56 (s, 2H), 3.54 (q, 2H), 3.49 (s, 2H), 3.48 (q, 2H), 3.24 (s, 2H), 3.10-3.16 (m, 2H), 2.87-2.98 (m, 1H), 2.67-2.71 (m, 2H), 2.67 (s, 3H), 2.24 (s, 3H), 1.81-1.91 (m, 2H), 1.74-1.81 (m, 2H), 1.50-1.62 (m, 2H), 1.33-1.44 (m, 2H), 1.20 (t, 2H), 1.18 (t, 2H)

**Example 10.Synthesis of N1-((2-(2,3-difluoro-6,7,8,9-tetrahydro-5H-benzo[7]cyclonenten-7-yl-6-methylpyridin-3-yl)methyl)-N1,N2 -dimethylethane-1,2-diamine (P038)**

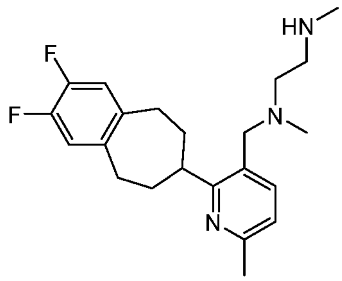

**P038**
**Example 10**

Compound **P038** was synthesized as described in Example 2, using**Intermediate 2-2** and **Intermediate A**. LCMS: (ESI) m/z=374.2 $[M+H]^+$; $^1H$ NMR (400 MHz, $D_2O$) δ(ppm) 8.49 (d, 1H), 7.73 (d, 1H), 7.07 (t, 2H), 4.61 (s, 2H), 3.57-3.70 (m, 3H), 3.48-3.56 (m, 2H), 2.92-3.06 (m, 2H), 2.81-2.89 (m, 2H), 2.78 (s, 3H), 2.77 (s, 3H), 2.70 (s, 3H), 2.01-2.13 (m, 2H), 1.74-1.91 (m, 2H)

**Example 11: Synthesis of the compound N,N'-dimethyl-N'-[[2-morpholin-4-[1-(m-tolyl)-4-piperidinyl]-1H-benzo[d]imidazol-5-yl ]methyl]ethane-1,2-diamine (P406):**

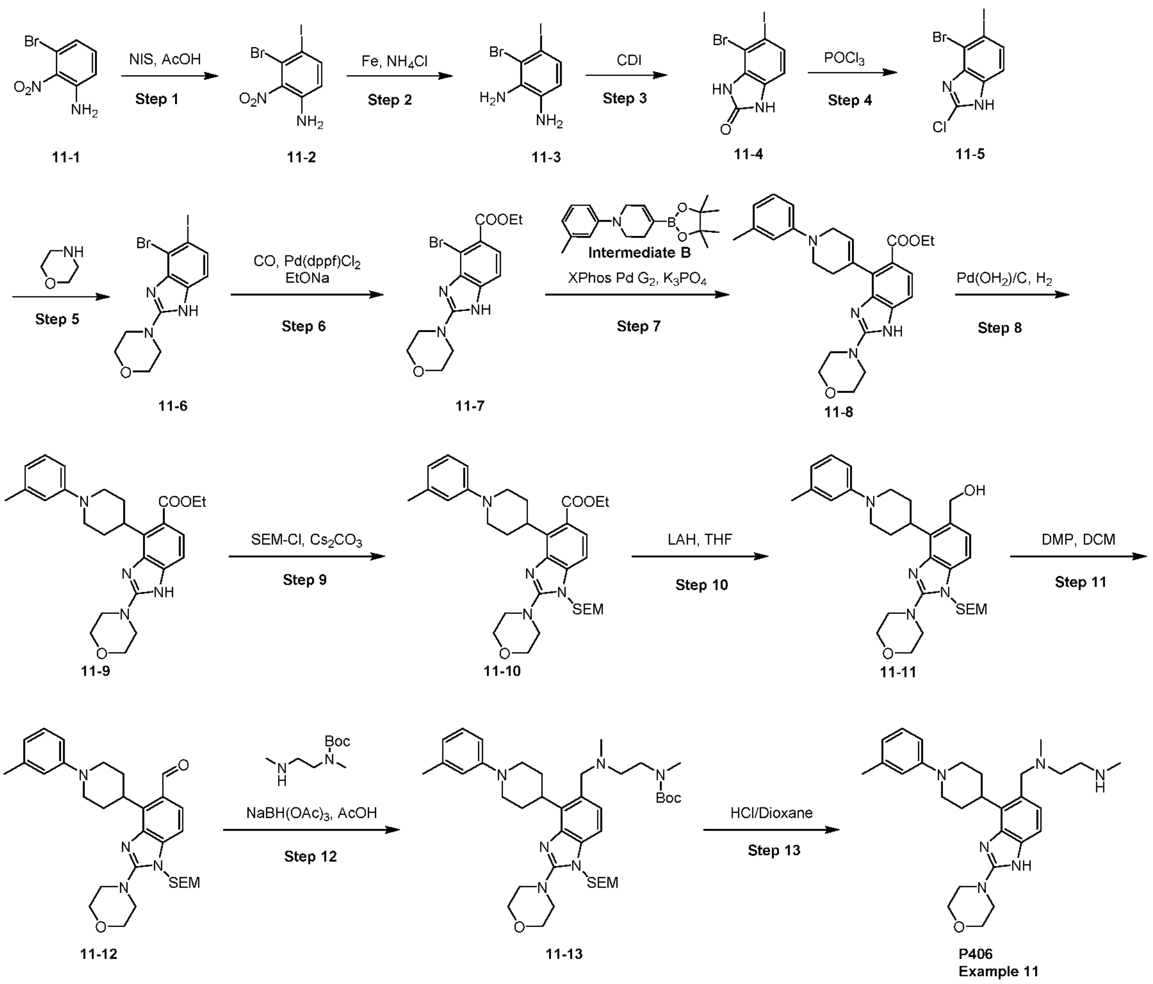


**Step 1:**A mixture of 2-nitro-3-bromoaniline (11-1) (150 g, 691.18 mmol), N-iodosuccinimide (171 g, 760.05 mmol) and acetic acid (1500 ml) was heated andrefluxed for about 2 hours, and cooled after the reactant11-1 was converted. The reaction solution was then poured into 3600 ml of water, and the precipitate was filtered, dried in vacuum,

dissolved in ethyl acetate, washed with water and saturated salt waterin turn, and extracted with ethyl acetate. The combined organic phases were dried with anhydrous magnesium sulfate; filtered and concentrated, then separated by silica gel column chromatography to give 178 g 2-nitro-3-bromo-4-iodoaniline (**11-2**) as a brown solid. $^1$H NMR (400 MHz, DMSO-d6)δ(ppm) 7.66 (d, 1H), 6.70 (d, 1H), 6.23 (s, 2H).

**Step 2:**To a solution of compound 2-nitro-3-bromo-4-iodoaniline (**11-2**) (80.0 g, 233.29 mmol) in a mixed system of tetrahydrofuran (640 ml)/ethanol (640 ml)/water (320 ml) was added $NH_4Cl$ (187 g, 3.50 mol), andthe mixture was heated to 90 °C and iron powder (52.1 g, 933.17 mmol) was added slowly in batches. Heating was continued for 2 hours until the reaction was complete. The reaction solution was thencooled to 60 °C, stirred with diatomite for 10 min, cooled to room temperature and filtered; the filtrate was extracted with ethyl acetate, concentrated to remove most of the water-soluble solvent and then dissolved in a large amount of ethyl acetate, and washed with saturated salt water; the organic phase was dried after extraction, filtered and concentrated to give a crude product of 3-bromo-4-iodo-phenylenediamine (**11-3**) as a brown solid (73.8 g, 224.04 mmol, 96.03% yield, 933.17 mmol), which was used directly in the next step without further purification. LCMS: (ESI) m/z = 314.8 [M+H]$^+$ ; $^1$H NMR (400 MHz, DMSO-d6) δ (ppm) 6.92 (d, 1H), 6.35 (d, 1H), 4.99 (s, 2H), 4.90 (s, 2H).

**Step 3:**A solution of compound 3-bromo-4-iodo-phenylenediamine (**11-3**) (35.0 g, 111.85 mmol) in tetrahydrofuran (350 mL) was cooled in an ice bath, and carbonyl diimidazole (27.2 g, 167.77 mmol) was added;then the reaction solution was gradually warmed to room temperature and stirred for about 2 hours until the reaction was complete. The reaction solution was concentrated and added with water to beat (500 mL) for half an hour, filtered and lyophilized to give a crude product of 4-bromo-5-iodo-1,3-dihydro-2H-benzo[d]imidazol-2-one (**11-4**), asa brown solid (37.8 g, 111.53 mmol, 99.72% yield), which was used directly in the next step without further purification.$^1$H NMR (400 MHz, DMSO-d6) δ (ppm) 11.12 (s, 1H), 11.02 (s, 1H), 7.47 (d, 1H), 6.75 (d, 1H).

**Step 4:** The compound 4-bromo-5-iodo-1,3-dihydro-2H-benzo[d]imidazol-2-one (**11-4**) (28.0 g, 82.61 mmol) was carefully added to phosphoryl trichloride (130 mL).The mixture was heated to 100 °C for 3 hours, and then heated to 120 °C for 16 hoursuntilthe reaction was essentially complete. The mixturewas cooled to room temperature and distilled under pressure to half volume. After carefullypouring in batches into 2 liters of ice water,the pH was carefully adjusted to 8 with potassium carbonate under stirring. A brown solid was precipitated, filtered and dried to give a crude product of 2-chloro-4-bromo-5-iodo-1H-benzo[d]imidazole (**11-5**) (30.0 g, crude), which was used directly in the next step without further purification. LCMS: (ESI) m/z = 358.9 [M+H]$^+$ ; $^1$H NMR (400 MHz, DMSO-d6) δ (ppm) 13.77 (br s, 1H), 7.72 (d, 1H), 7.32 (br d, 1H).

**Step 5:**To a suspension of compound 2-chloro-4-bromo-5-iodo-1H-benzo[d]imidazole (**11-5**) (30.0 g, 83.95 mmol, 2.05 mL) inisopropanol (150 mL) andtetrahydrofuran (150 mL)was added morpholine (37.0 g, 424.70 mmol, 37.37 mL), andthe mixture was heated to 90 °C for 4 days and then cooled to room temperatureafter complete conversion of theraw material.The organic solvent was spun off, thenbeatin water (300 mL); the solids were filtered and purified by silica gel column chromatography to give a brown mucus, which wasbeatingin ethanol to give 30.1 g of a yellow solid crude product, and further recrystallized inethanol to give 2-(4-morpholinyl)-4-bromo-5-iodo-1H-benzo[d]imidazole (**11-6**) as a white solid(15.5 g, 37.99 mmol, 45.25% yield).LCMS: (ESI) m/z = 407.9, 409.9 [M+H]$^+$; $^1$H NMR (400 MHz, DMSO-d6) δ (ppm) 11.82 (s, 0.85H), 11.40 (s, 0.15H), 7.48 (d, 0.15H), 7.40 (d, 0.85H), 6.97-7.05 (m, 1H), 3.65-3.81 (m, 4H), 3.44-3.59 (m, 4H).

**Step 6:** 2-(4-morpholinyl)-4-bromo-5-iodo-1H-benzo[d]imidazole (**11-6**) (15.5 g, 37.99 mmol) was added to asolution of sodium ethoxide (20% purity, 180 mL) in ethanol. The reaction solution was deoxygenated and replaced 3 times withCO (balloon), then added withPd(dppf)$Cl_2$ (1.11 g, 1.52 mmol), deoxygenated again and replaced 3 times with CO; the system was heated to 45 °C to react for 16 hours. The reaction was then cooled in an ice bath and quenched by careful addition of acetic acid (8 mL, pH = 6-7), concentrated, diluted with ethyl acetate, washed and extracted with saturated sodium bicarbonate and saturated salt water; the organic phase was dried with anhydrous magnesium sulfate, filtered and concentrated, then purified by silica gel column chromatography to give ethyl 4-bromo-2-(4-morpholinyl)-1H-benzo[d]imidazole-5-carboxylate (**11-7**) (16.5 g, 46.58 mmol, 77.12% yield), as a light yellow amorphous solid.LCMS: (ESI) m/z = 356.0 [M+H]$^+$

**Step 7:** The compound ethyl 4-bromo-2-(4-morpholinyl)-1H-benzo[d]imidazole-5-carboxylate (**11-7**) (16.5 g, 46.58 mmol), 4-(4,4,5,5-tetramethyl-1,3,2-dioxaborolan-2-yl)-1-(m-tolyl)-1,2,3,6-tetrahydropyridine (**Intermediate B**) (15.3 g, 51.24 mmol), and potassium phosphate (19.8 g, 93.17 mmol) were added to tetrahydrofuran (165 mL) and water (33mL), the reaction solution was replaced with nitrogen, XPhos-PD-G2 (1.10 g, 1.40 mmol) was added, and the reaction was heated at 65 °C for 5 h under nitrogen protection. The reaction solution was cooled to 40 °C,then added with sodium diethylcarbamothionylthiolate (997.25 mg, 5.82 mmol) and continued to stir for 1 hour,then diluted with water (200 mL), and extracted with ethyl acetate.The organic phase was dried with anhydrous magnesium sulfate, filtered and concentrated, and the crude product was purified by silica gel column chromatography to give ethyl 2-(4-morpholinyl)-4-(1-m-tolyl-1,2,3,6-tetrahydropyridin-4-yl)-1H-benzo[d]imidazole-5-carb oxylate (**11-8**) (18.5 g, 41.43 mmol, 88.94%), as a yellow solid.LCMS: (ESI) m/z = 447.3 [M+H]$^+$ ; $^1$H NMR (400 MHz, $CDCl_3$) δ (ppm) 9.59 (br s, 1H), 7.89 (d, 1H), 7.35 (d, 1H), 7.23 (t, 1H), 6.86-6.98 (m, 2H), 6.81 (br d, 1H), 5.75 (br s, 1H), 4.33 (q, 2H), 3.76-3.87 (m, 6H), 3.50-3.64 (m, 6H),

2.88 (br d, 2H), 2.65 (br s, 2H), 2.37 (s, 3H), 1.39 (t, 3H).

**Step 8:** To a suspensionof compound ethyl 2-(4-morpholinyl)-4-(1-m-tolyl-1,2,3,6-tetrahydropyridin-4-yl)-1H-benzo[d]imidazole-5-carb oxylate (**11-8**) (18.5 g, 41.43mmol) in tetrahydrofuran (600 mL) was added $Pd(OH)_2/C$ (7.0 g, 41.43mmol, 10% purity), and the reaction was replaced with hydrogen and then reacted for 16hours at 30 °Cunder hydrogen atmosphere(balloon) until the reaction was fundamentalfinished.The reaction solution was filtered and the filter cake was washed with tetrahydrofuran (200 mL*2). The combined filtrate was concentrated and dried to give a crude productethyl 2-(4-morpholinyl)-4-(1-m-tolyl-4-piperidinyl)-3H-benzo[d]imidazole-5-carboxylate (**11-9**)(19 g), which was used directly in the next step without further purification. LCMS: (ESI) m/z=449.3$[M+H]^+$.

**Step 9:**To a solution of compound ethyl 2-(4-morpholinyl)-4-(1-m-tolyl-4-piperidinyl)-3H-benzo[d]imidazole-5-carboxylate (**11-9**) (19 g, 42.36 mmol) in $CH_3CN$ (550 mL) was added cesium carbonate (69.0 g, 211.79 mmol), and SEM-Cl (19 g, 113.96 mmol, 20.17 mL) was added dropwise understirring. The reaction mixture was reacted at 30°C for 24 hours, and then the reaction was basically complete. The mixture was then filtered, washed with ethyl acetate, and the combined organic phases were concentrated, then purified by silica gel column chromatography to obtain compound ethyl 2-(4-morpholinyl)-4-(1-m-tolyl-4-piperidinyl)-1-(2-trimethylsilylethoxymethylene)benzo[d]i midazole-5-carboxylate (**11-10**) as a colorless oil (22.3 g, 38.53 mmol, 90.96% yield). LCMS: (ESI) m/z = 580.2 $[M+H]^+$ ; $^1H$ NMR (400 MHz, $CDCl_3$) $\delta$ (ppm) 7.59 (d, 1H), 7.08-7.21 (m, 2H), 6.82-6.89 (m, 2H), 6.65 (d, 1H), 5.29 (s, 2H), 4.40 (q, 2H), 3.91 (br d, 2H), 3.81-3.87 (m, 4H), 3.64-3.76 (m, 4H), 3.38-3.47 (m, 4H), 2.98-3.12 (m, 2H), 2.85-2.96 (m, 2H), 2.35 (s, 3H), 1.79 (br d, 2H), 1.44 (t, 3H), 0.05 (s, 2H), 0.02 (s, 8H)

**Step 10:** To a solution of aluminum lithium hydride (3.0 g, 79.05 mmol, 79.05 ml) in tetrahydrofuran (300 ml) was added dropwise a solution of compound ethyl 2-(4-morpholinyl)-4-(1-m-tolyl-4-piperidinyl)-1-(2-trimethylsilylethoxymethylene)benzo[d]i midazole-5-carboxylate (**11-10**) (22.0 g, 38.01 mmol) in tetrahydrofuran (100 ml) underan ice bath, then gradually returned to room temperature after the dropwise addition, and heated to 30 °C for 1 hour.The reaction was basically complete. The reaction was then quenched by carefully dripping 3 ml of water, 3 ml of 15% sodium hydroxide aqueous solution, and 6 ml of water under an ice bath, then stirred and dried with anhydrous magnesium sulfate at room temperature for 15 minutes, and then filtered. The organic phase was concentrated to obtain a crude product of 2-(4-morpholinyl)-4-(1-m-tolyl-4-piperidinyl)-1-(2-trimethylsilylethoxymethylene)benzo[d]i midazole-5-methanol (**11-11**) (19.3 g, 35.96 mmol, 94.60% yield) as a colorless viscous liquid, which was directly used in the next step without further purification. LCMS: (ESI) m/z = 537.3 $[M+H]^+$ ; $^1H$ NMR (400 MHz, $CDCl_3$) $\delta$ (ppm) 7.28 (s, 1H), 7.10-7.22 (m, 3H), 6.83-6.92 (m, 2H), 6.68 (d, 1H), 5.23-5.34 (m, 2H), 4.85 (s, 2H), 3.90 (br d, 2H), 3.80-3.86 (m, 4H), 3.75-3.80 (m, 1H), 3.67-3.75 (m, 2H), 3.39-3.47 (m, 4H), 3.27-3.38 (m, 1H), 3.06 (m, 2H), 2.88-2.99 (m, 2H), 2.35 (s, 3H), 0.95-1.01 (m, 2H), 0.05 (s, 2H), 0.02 (s, 8H).

**Step 11**: To a solution of compound 2-(4-morpholinyl)-4-(1-m-tolyl-4-piperidinyl)-1-(2-trimethylsilylethoxymethylene)benzo[d]i midazole-5-methanol (**11-11**) (9.5 g, 17.70 mmol) in dichloromethane (200 ml) was added DMP (9.01 g, 21.24 mmol, 6.58 ml), then reacted at 30°C with stirring for half an hour; the reaction was basically complete. 100 mL of saturated sodium bicarbonate aqueous solution and 100 mL of sodium thiosulfate were added and the reaction was stirred for 30 min.The organic phase was dried with anhydrous magnesium sulfate after dichloromethane extraction, filtered, concentrated and then purified by silica gel column to give compound 2-(4-morpholinyl)-4-(1-m-tolyl-4-piperidinyl)-1-(2-trimethylsilyl ethoxymethylene)benzo[d]imidazole-5-carbaldehyde (**11-12**) as a beige white amorphous solid (11.7 g, 21.88 mmol, 61.81% yield). LCMS: (ESI) m/z = 535.3 $[M+H]^+$ ; $^1H$ NMR (400 MHz, $CDCl_3$) $\delta$ (ppm) 10.50 (s, 1H), 7.75 (d, 1H), 7.25 (d, 1H), 7.19 (t, 1H), 6.81-6.90 (m, 2H), 6.67 (d, 1H), 5.31 (s, 2H), 4.10-4.16 (m, 1H), 3.92 (br d, 2H), 3.80-3.88 (m, 4H), 3.69-3.77 (m, 2H), 3.39-3.49 (m, 4H), 2.90-3.08 (m, 4H), 2.36 (s, 3H), 1.82 (br d, 2H), 0.94-1.04 (m, 2H), 0.00-0.07 (m, 9H).

**Step 12**: To a solution of compound 2-(4-morpholinyl)-4-(1-m-tolyl-4-piperidinyl)-1-(2-trimethylsilyl ethoxymethylene)benzo[d]imidazole-5-carbaldehyde (**11-12**) (10.7 g, 20.01 mmol) and tert-butyl N-methyl-N-[2-(methylamino) ethyl] carbamate (4.52 g, 24.01 mmol) in 150 ml of dichloromethane was added acetic acid (1.44 g, 24.01 mmol, 1.37 ml), and the reaction solution was stirred at 30 °C for 1 hour, then sodium acetate borohydride (12.7 g, 60.03 mmol) was added in batches, and the reaction was stirred at 30°C for 15 hours until the reaction was basically complete.The reaction was quenched by careful addition of 100 mL of saturated sodium bicarbonate aqueous solution and stirred for 1 hour, then extracted with dichloromethane. The combined organic phase was dried with anhydrous magnesium sulfate, filtered, and concentrated, then purified by silica gel column chromatography to obtain the product N-tert-butoxycarbonyl-N,N'-dimethyl-N'-[[2-morpholinyl-4-[1-(m-tolyl)-4-piperidinyl]-1H-b enzo[d]imidazol-5-yl]methyl]ethane-1,2-diamine (**11-13**) (10.6 g, 14.99 mmol, 68.12% yield) and by-product 2-(4-morpholinyl)-4-(1-m-tolyl-4-piperidinyl)-1-(2-trimethylsilylethoxymethylene)benzo[d]i midazole-5-methanol (**11-11**) (1.9 g, 3.54 mmol, 17.69% yield). LCMS: (ESI) m/z = 535.3 $[M+H]^+$ ; $^1H$ NMR (400 MHz, $CDCl_3$) $\delta$ (ppm) 7.17 (t, 1H), 7.04 (s, 2H), 6.83-6.89 (m, 2H), 6.65 (d, 1H), 5.25 (s, 2H), 3.89 (br d, 2H), 3.78-3.85 (m, 4H), 3.68-3.76 (m, 2H), 3.61 (s, 2H), 3.37-3.44 (m, 5H), 3.30 (br s, 2H), 3.02 (m, 2H), 2.80-2.91 (m, 5H), 2.46-2.64 (m, 2H), 2.34 (s, 3H), 2.22 (br s, 3H), 1.69 (br d, 2H), 1.36-1.45 (m, 9H), 0.92-1.00 (m, 2H), -0.02-0.03 (m, 9H).

**Step 13**: To a solution of compound N-tert-butoxycarbonyl-N,N'-dimethyl-N'-[[2-morpholinyl-4-[1-(m-tolyl)-4-piperidinyl]-1H-b enzo[d]imidazol-5-yl]methyl]ethane-1,2-diamine (**11-13**) (10.6 g, 14.99 mmol) in 50 mL of 1,4-dioxane was added a solution of HCl/dioxane (50 mL) and stirred at 70°C for 2 hours to precipitatewhite solid. After cooling to room temperature, 10 ml of methanol was added. After concentration, 50 ml of methanol was added, and 500 ml of isopropyl ether was added dropwise understirring toget white precipitates, which continued to beatunder stirring at room temperature for 16 hours; then filtered and lyophilized to give N,N'-dimethyl-N'-[[2-morpholinyl-4-[1-(m-tolyl)-4-piperidinyl]-1H-benzo[d]imidazol-5-yl] methyl]ethane-1,2-diamine hydrochloride as a white solid (quantitatively determined as hydrochloride coefficient of 4). LCMS: (ESI) m/z = 477.3 $[M+H]^+$ ; $^1H$ NMR (400 MHz, $D_2O$) $\delta$ (ppm) 7.33-7.55 (m, 6H), 3.89-4.02 (m, 6H), 3.69-3.87 (m, 9H), 3.54-3.66 (m, 2H), 2.87 (s, 3H), 2.80 (s, 3H), 2.60-2.77 (m, 2H), 2.39 (s, 3H), 2.21 (br d, 2H)

To a solution of N,N'-dimethyl-N'-[[2-morpholinyl-4-[1-(m-tolyl)-4-piperidinyl]-1H-benzo[d]imidazol-5-yl] methyl]ethane-1,2-diamine hydrochloride (400 mg) in 5 ml of water wascarefully added dropwise saturated sodium carbonate aqueous solution to adjust the pH to 8-10, then extracted with ethyl acetate.The organic phase was washed with saturated salt waterand dried with anhydrous magnesium sulfate, filtered, and concentrated;then the crude product was lyophilized to give N,N'-dimethyl-N'-[[2-morpholinyl-4-[1-(m-tolyl)-4-piperidinyl]-1H-benzo[d]imidazol-5-yl] methyl]ethane-1,2-diamine (**P406, Example 11**) as a light yellow freecompound (270 mg. 0.566 mmol, 88.15% yield). LCMS: (ESI) m/z = 477.3 $[M+H]^+$ ; $^1H$ NMR (400 MHz, DMSO-d6) $\delta$ (ppm) 11.28 (br s, 1H), 7.08 (t, 1H), 6.94 (br d, 1H), 6.80 (s, 1H), 6.75 (br t, 2H), 6.55 (br d, 1H), 3.84 (br d, 2H), 3.68 (br s, 4H), 3.48 (s, 2H), 3.05-3.29 (m, 6H), 2.92 (m, 2H), 2.69-2.80 (m, 2H), 2.55-2.63 (m, 2H), 2.41-2.48 (m, 2H), 2.26 (d, 6H), 2.03 (s, 3H), 1.50 (br d, 2H).

## EXAMPLE 12: Synthesis of compound 5-((methyl(2-(methylamino)ethyl)amino)methyl)-4-(1-methylphenyl)piperidin-4-yl)indol in-2-one (P180).

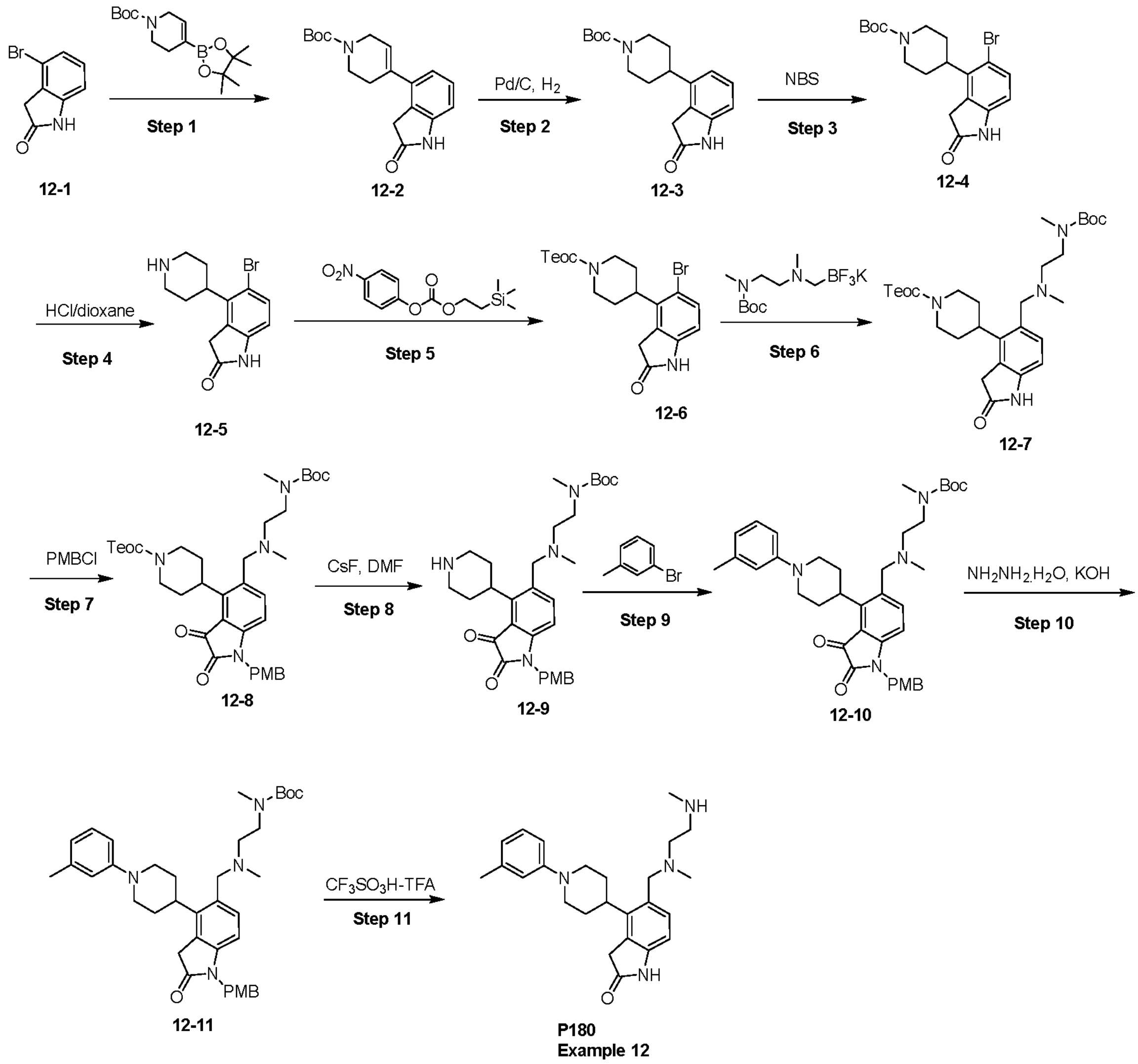


**Step 1:** Compound 4-bromoindolin-2-one (**12-1**) (2.00 g, 9.43 mmol), tert-butyl 4-(4,4,5,5-tetramethyl-1,3,2-dioxaborolan-2-yl)-3,6-dihydropyridine-1(2H)-carboxylate (2.92 g, 9.43 mmol) and potassium phosphate (6.01 g, 28.30 mmol) were added to a mixture of 100 mL of tetrahydrofuran and 20 mL of water, and XphosPd G3 (239 mg, 0.28 mmol) was added after nitrogen replacement.The reaction was heated to 65 °C and stirred under nitrogen protection for 2 hours after another nitrogen replacement. The reaction solution was then diluted with saturated salt waterand extracted with ethyl acetate; the combined organic phases were dried with anhydrous magnesium sulfate, filtered, and concentrated; then the crude product was purified by silica gel column chromatography to give compound 4-(1-tert-butoxycarbonyl-1,2,3,6-tetrahydropyridin-4-yl)-indolin-2-one (**12-2**) as a brown solid (2.8 g, 8.91 mmol, 94.43% yield). LCMS: (ESI) m/z = 214.9 $[M+H]^+$ ; $^1H$ NMR (400 MHz, DMSO-d6) δ (ppm) 10.40 (s, 1H), 7.16 (t, 1H), 6.88 (d, 1H), 6.73 (d, 1H), 5.96 (br s, 1H), 3.98 (br s, 2H), 3.54 (s, 2H), 3.46-3.53 (m, 2H), 2.41 (br d, 2H), 1.44 (s, 9H).

**Step 2**: Compound 4-(1-tert-butoxycarbonyl-1,2,3,6-tetrahydropyridin-4-yl)-indolin-2-one (**12-2**) (2.30 g, 7.32 mmol) was added to 50 mL of methanol, and 10% palladium-carbon (460 mg, 0.43 mmol) was added after nitrogen replacement.The reaction solution was stirred at room temperature for 16 hours under hydrogen atmosphere (balloon) after hydrogen replacement. When the reaction was basically complete, it wasfiltered and washed with methanol; the filtrate was concentrated to give a yellow crude product of 4-(1-tert-butoxycarbonyl-piperidin-4-yl)-indolin-2-one (**12-3**) (2.2 g, 6.95 mmol, 95.04% yield), which was used directly in the next step without further purification. LCMS: (ESI)m/z = 216.9 $[M+H]^+$; $^1H$ NMR (400 MHz, $CDCl_3$) δ(ppm) 8.55(br s, 1H), 7.22(t, 1H), 6.89(d, 1H), 6.78(d, 1H), 4.28 (br s, 2H), 3.52(s, 2H), 2.83(br t, 2H), 2.63(tt, 1H), 1.75-1.82(m, 2H), 1.61-1.73(m, 2H), 1.51(s, 9H).

**Step 3**: To a solution of the crude product of 4-(1-tert-butoxycarbonyl-piperidin-4-yl)-indolin-2-one (**12-3**) (3.70 g, 11.69 mmol) in 100 mL of dichloromethane was added N-bromosuccinimide (2.29 g, 12.86 mmol, 1.1 eq) in batches, and the reaction was stirred at room temperature for 16 hours, and the reaction was basically complete. The reaction solution was then carefully added with 50 mL of 15% potassium carbonate aqueous solution under stirring; the organic

phase was washed with saturated salt waterafter dichloromethane extraction, dried with anhydrous magnesium sulfate, filtered, concentrated and then pulped with ethyl acetate at room temperature to give a crude product of 4-(1-tert-butoxycarbonyl-piperidin-4-yl)-5-bromo-indolin-2-one (**12-4**) (3.60 g, 9.11 mmol, 77.88% yield) as a white solid, which was used directly in the next step without further purification. $^1$H NMR (400 MHz, $CDCl_3$) δ (ppm) δ 8.90 (br s, 1H), 7.47 (d, 1H), 6.67 (d, 1H), 4.28 (br d, 2H), 3.65 (s, 2H), 3.37 (br s, 1H), 2.83 (br t, 2H), 1.78 (br d, 4H), 1.51 (s, 9H).

**Step 4**: To a solution of the crude product of 4-(1-tert-butoxycarbonyl-piperidin-4-yl)-5-bromo-indolin-2-one (**12-4**) (3.60 g, 9.11 mmol) in 20 mL of 1,4-dioxane was added a solution of 20 mL of 4M hydrochloric acid in1,4-dioxane, and stirred at room temperature for 16 hours and the reaction was basically complete. After filtration, the filter cake was washed with methyl tert-butyl ether, concentrated and dried to give a white crude product of 5-bromo-4-(piperidin-4-yl)indolin-2-one (**12-5**) hydrochloride (3.00 g, 9.05 mmol, 99.33% yield), which was used directly in the next step without further purification. LCMS: (ESI) m/z=295.0[M+H]$^+$;$^1$H NMR(400 MHz,$D_2O$) δ(ppm) 7.42(d,1H), 6.66(d,1H), 3.57-3.65(m, 2H), 3.46(br d,2H), 3.36(br s,1H), 3.07(m,2H), 1.64-2.30(m,4H).

**Step 5**: To a stirred suspension of compound 5-bromo-4-(piperidin-4-yl)indolin-2-one (**12-5**) hydrochloride (1.90 g, 5.73 mmol) in acetonitrile (20 mL) was added N,N-diisopropylethylamine (1.55 g, 12.03 mmol, 2.10 mL), and ethyl (4-nitrophenyl) 2-trimethylsilyl ethyl carbonate (1.79 g, 6.30 mmol) was added dropwise. The reaction solution was stirred at room temperature for 15 h and then filtered; the filter cake was washed with acetonitrile. The combined mother liquors were concentrated, and then purified by silica gel column chromatography to give 5-bromo-4-(1-trimethylsilylethoxycarbonyl-piperidin-4-yl)-indolin-2-one (**12-6**) as a yellow solid (2.50 g, 5.40 mmol, 94.34% yield, 95% purity). LCMS: (ESI) m/z = 413.1 [M-2Me+H]$^+$

**Step 6**: To a mixture of the compound 5-bromo-4-(1-trimethylsilylethoxycarbonyl-piperidin-4-yl)-indolin-2-one (**12-6**) (2.00 g, 4.55 mmol), anhydrous [2-(tert-butoxycarbonylmethylethylamino)(methyl)amino]potassium methylborontrifluoride (4.21 g, 13.65 mmol), potassium phosphate (2.90 g, 13.65 mmol), and RuphosPd G2 (353 mg, 0.455 mmol) were added to a mixture of 40 mL of tetrahydrofuran and 4 mL of water. The reaction was replaced with nitrogen, and heated to 80 °C under nitrogen protection for 16 handthe reaction was basically complete. After cooling to room temperature, 30 ml of saturated saline was added;the organic phase was extracted with ethyl acetate and dried with anhydrous magnesium sulfate, filtered and concentrated, then purified by silica gel column chromatography to give compound 2-trimethylsilyl ethyl-4-(5-(((2-((tert-butoxycarbonyl)(methyl)amino)ethyl)(methyl)amino)methyl-2-oxoindol in-4- yl)piperidine-1-carboxylate (**12 -7**) as a yellow viscous liquid (2.8 g, 4.49 mmol, 98.73% yield, 90% purity). LCMS:(ESI)m/z= 561.5 [M+H]$^+$.

**Step 7**: A mixture of compound 2-trimethylsilyl ethyl-4-(5-(((2-((tert-butoxycarbonyl)(methyl)amino)ethyl)(methyl)amino)methyl-2-oxoindol in-4- yl)piperidine-1-carboxylate (**12-7**) (1.50 g, 2.41 mmol, 90% purity) and cesium carbonate (5.00 g, 15.35 mmol) was added to 20 mL of acetonitrile and stirred for 3 h; then 4-methoxybenzyl chloride (565 mg, 3.61 mmol, 0.49 mL) was added dropwise, and the reaction was basically complete after stirring for 1 h at room temperature. Afterfilteration, the filtrate was concentrated, and then purified by silica gel column chromatography to give compound 2-trimethylsilyl ethyl-4-(5-(((2-((tert-butoxycarbonyl)(methyl)amino)ethyl)(methyl)amino)methyl)-1 -(4-methoxybenzyl)-2,3-dioxoindolin-4-yl)piperidine-1-carboxylate (**12-8**) as a yellow solid (1.32 g, 1.46 mmol, 60.76% yield, 77% purity). LCMS: (ESI) m/z = 695.6 [M+H]$^+$.

**Step 8**: To a solution of compound 2-trimethylsilyl ethyl-4-(5-(((2-((tert-butoxycarbonyl)(methyl)amino)ethyl)(methyl)amino)methyl-1-(4-meth oxybenzyl)-2,3-dioxoindolin-4-yl)piperidine-1-carboxylate (**12-8**) (2.10 g, 2.33 mmol, 77% purity) in 30 mL of N,N- dimethylformamide was added cesium fluoride (1.77 g, 11.63 mmol, 0.43 mL), and the reaction was heated to 65 °C and stirred for 5 hoursand the reaction was basically complete. Aftercoolingto room temperature, the reaction was diluted with water, adjusted to pH 3-4 with 10% phosphoric acidaqueous solution, extracted with methyl tert-butyl etherand washed.The aqueous phase was adjusted to pH 12 with 10M sodium hydroxideaqueous solution, and extracted with ethyl acetate.The organic phase was dried with anhydrous magnesium sulfate, filtered, concentrated and then vacuum-dried to obtain a crude product of tert-butyl (2-(((1-(4-methoxybenzyl)-2,3-dioxo-4-(piperidin-4-yl)indolin-5-yl)methyl)(methyl)amino)et hyl)(methyl)carbamate (**12-9**) as a yellow solid (1.40 g, 2.16 mmol, 92.87% yield, 85% purity), which was used directly in the next step without further purification. LCMS: (ESI) m/z = 551.4 [M+H]$^+$.

**Step 9:** To a mixture of compound tert-butyl (2-(((1-(4-methoxybenzyl)-2,3-dioxo-4-(piperidin-4-yl)indolin-5-yl)methyl)(methyl)amino)et hyl)(methyl)carbamate (**12-9**) (1.30 g, 2.36 mmol), m-bromo-toluene (605 mg, 3.54 mmol, 0.43 ml) and cesium carbonate ( 1.54 g, 4.72 mmol) was added 20 mL of toluene.The reaction solution was replaced with nitrogen, and added with RuPhosPd G3 (200 mg, 0.24 mmol).thenthe reaction solution was replaced with nitrogen and heated to 100 °C with stirring for 16 hours;the reaction was essentially complete. After filtration, it was concentrated and purified by silica gel column chromatography to give compound tert-butyl (2-(((1-(4-methoxybenzyl)-2,3-dioxo-4-(1-m-tolylpiperidin-4-yl)indolin-5-yl)methyl)(methyl)amino)ethyl)(methyl)carbamate (**12-10**) as an orange solid (900 mg, 1.40 mmol, 59.49% yield, 100% purity). LCMS: (ESI) m/z = 641.4 [M+H]$^+$. $^1$H NMR (400 MHz, $CD_3OD$) δ (ppm) 7.45 (br s, 1H), 7.33 (br d, 2H), 7.13 (t, 1H), 6.72-6.94 (m, 5H), 6.67 (d, 1H), 3.68-3.91 (m, 5H), 3.52 (s, 2H), 3.25-3.40 (m, 5H), 2.67-2.91 (m, 4H), 2.37-2.67 (m, 4H), 2.09-2.36 (m, 7H), 1.60 (br d, 2H), 1.17-1.41 (m, 9H).

**Step10:**To a solution of compound tert-butyl (2-(((1-(4-methoxybenzyl)-2,3-dioxo-4-(1-m-tolylpiperidin-4-yl)indolin-5-yl)methyl)(methyl)amino)ethyl)(methyl)carbamate (**12-10**) (190 mg, 0.30 mmol) in 5 mL of ethanol was added hydrazine hydrate (5.15 g, 87.44 mmol, 5.00 mL, 85% purity) and potassium hydroxide (332 mg, 5.93 mmol), and the mixture was heated to 80 °Cand stirred for 3 hours, the reaction was essentially complete. Most of the ethanol was removed by concentration, the aqueous phase was extracted with ethyl acetate, and the combined organic phases were dried with anhydrous magnesium sulfate, filtered and concentrated to obtain a crude product of tert-butyl (2-(((1-(4-methoxybenzyl)-2-oxo-4-(1-m-tolylpiperidin-4-yl)indolin-5-yl)(methyl)(methyl)amino)ethyl)(methyl)carbamate (**12-11**) (220 mg) as a yellow viscous liquid, which was used directly in the next step without further purification. LCMS: (ESI) m/z = 627.5 $[M+H]^+$.

**Step 11**: To a stirred solution of compound tert-butyl (2-(((1-(4-methoxybenzyl)-2-oxo-4-(1-m-tolylpiperidin-4-yl)indolin-5-yl)methyl)(methyl)am ino)ethyl)(methyl)carbamate (**12-11**) (220 mg, 0.32 mmol, 92% purity) in 5 mL of trifluoroacetic acid was added dropwise trifluoromethanesulfonic acid (200 mg, 1.33 mmol, 0.12 mL), and the reaction solution was stirred at room temperature for 5 hours and then heated to 40 °C for 30 hours; the reaction was essentially complete. The reaction solution was then concentrated and diluted with 5 mL of dichloromethane and extracted with 0.5 M diluted hydrochloric acid aqueous solution. The combined aqueous phases were adjusted to pH 12 with 1 M sodium hydroxide aqueous solution and then extracted with dichloromethane. The combined organic phases were dried with anhydrous magnesium sulfate, filtered and concentrated to give a free base product of 5-((methyl(2-(methylamino)ethyl)amino)methyl)-4-(1-m-tolyl)piperidin-4-yl)indolin-2-one (**P18**0). To a stirred solution of the product in 2 mL of 1,4-dioxane was added dropwise 2 mL of 4 M hydrochloric acid-1,4-dioxane solution. The reaction solution was then concentrated and lyophilized to give 5-((methyl(2-(methylamino)ethyl)amino)methyl)-4-(1-methylphenyl)piperidin-4-yl)indolin-2 -one **(P180, Example 12)** hydrochloride, as a white solid (80 mg, 48.12% yield, quantitative hydrochloride coefficient of 3). LCMS: (ESI) m/z = 407.3 $[M+H]^+$. $^1$H NMR (400 MHz, $D_2O$) δ (ppm) 7.46 (s, 1H), 7.39-7.44 (m, 2H), 7.37 (d, 1H), 7.31-7.36 (m, 1H), 6.97 (d, 1H), 4.55 (s, 2H), 3.78-3.92 (m, 4H), 3.70-3.77 (m, 2H), 3.59-3.70 (m, 2H), 3.48-3.57 (m, 2H), 3.41 (br t, 1H), 2.83 (s, 3H), 2.73 (s, 3H), 2.41-2.59 (m, 2H), 2.34 (s, 3H), 2.07 (br d, 2H).

**EXAMPLE 13 Synthesis of the compound N-((5-amino-3-(1-(m-tolyl)piperidin-4-yl)pyrazin-2-yl)methyl)-N,N'-dimethyl-1,2-ethyle nediamine (P292).**

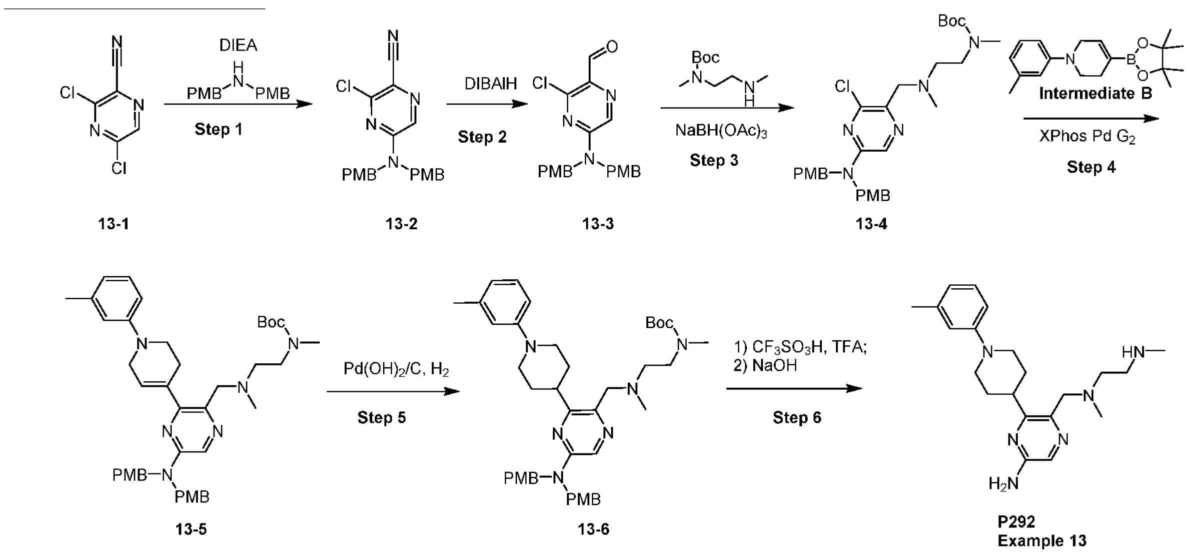


13-1 13-2 13-3 13-4

13-5 13-6 P292 Example 13

**Step 1:** To a solution of compound 2-cyano-3, 5-dichloropyrazine **(13-1)** (145 g, 833.39 mmol) in 1000 mL of N,N-dimethylformamide was added dropwise diisopropylethylamine (109 g, 843.37 mmol, 146.90 mL) at room temperature, and to which a solution of di(4-methoxybenzyl)amine (217.50 g, 845.23 mmol) in 500 mL of N,N-dimethylformamide was added dropwise understirring after cooling to 5-10 °C. The reaction solution was then slowly warmed to room temperature with continous stirring for 3 hours, and the reaction was essentially complete. The reaction solution was then poured into 5000 ml of ice-water mixture and stirred for 5 minutes to precipitatesolid. After filtration, the solid was dissolved in 4000 ml of ethyl acetate and then washed with saturated salt water; the organic phase was dried with anhydrous magnesium sulfate, filtrated and concentrated. The crude product was beatingwith 700 ml of methyl tert-butyl ether at 20 °C for 16 hours, filtered and dried to give compound 2-cyano-3-chloro-5-(bis(4-methoxybenzyl)amino)pyrazine

**(13-2)**as a yellow solid, which was directly used in the next step without further purification. $^1$H NMR (400 MHz, $CDCl_3$) δ (ppm) 7.87 (s, 1H), 7.14 (br d, 4H), 6.85-6.92 (m, 4H), 4.75 (br s, 4H), 3.81 (s, 6H).

**Step 2:** A solution of compound 2-cyano-3-chloro-5-(bis(4-methoxybenzyl)amino)pyrazine (**13-2**) in 600 mL of tetrahydrofuran was cooled to -78 °C under nitrogen protection, and DIBALH (1 mole/liter, 548 mL) was added dropwise udnerstirring.The reaction continued stirring for 2 hours until the reaction was almost complete. The reaction was quenched by carefully adding 500 ml of 10% acetic acid aqueous solution under stirring at -78 °C. The reaction was then slowly warmed to room temperature, filtered through diatomite and washed with ethyl acetate.The filtrate was extracted with ethyl acetate, and the combined organic phases were adjusted to pH 8-9 with saturated sodium bicarbonate aqueous solution.The separated organic phases were washed with saturated salt water, and dried with anhydrous magnesium sulfate, filtered, and concentrated. Then the product was beatingwith methyl tert-butyl ether/petroleum ether (1:2, v/v) for 3 hours to give 5-(bis(4-methoxybenzyl)amino)-3-chloro-pyrazine-2-carboxaldehyde (**13-3**) (100 g, 252.35 mmol, 92.69% yield) as a yellow solid, which was used in the next step directly. $^1$H NMR (400 MHz, $CDCl_3$) δ (ppm)10.15 (s, 1H), 8.03 (s, 1H), 7.12-7.20 (m, 4H), 6.86-6.91 (m, 4H), 4.71-4.87 (m, 4H), 3.81 (s, 6H).

**Step 3:** To a solution of compound 5-(bis(4-methoxybenzyl)amino)-3-chloro-pyrazine-2-carbaldehyde (13-3) (53.0 g, 133.21 mmol) and N-tert-butyloxycarbonyl-N,N'-dimethylethylenediamine (30.0 g, 159.35 mmol) in 1000 ml of dichloromethane was added acetic acid ( 9.60 g, 159.86 mmol, 9.14 mL), then sodium borohydride acetate (71.0 g, 335.00 mmol) was added in batches at room temperature, and the reaction continued stirring for 2 h. The reaction was then quenched by careful addition of 1,000 mL of saturated sodium carbonate aqueous solution, then extracted with ethyl acetate; the organic phase was washed with saturated salt water, dried with anhydrous magnesium sulfate, filtered, concentrated, then purified by silica gel column chromatography to give compound tert-butyl (2-((((5-(bis(4-methoxybenzyl)amino)-3-chloropyrazin-2-yl)(methyl)amino)ethyl)(methyl)ca rbamate (**13-4**) as a yellow mucus (112 g, 196.45 mmol. 73.73% yield); the crude product was used directly in the next step without further purification. $^1$H NMR (400 MHz, $CDCl_3$) δ (ppm) 7.83 (s, 1H), 7.15 (d, 4H), 6.83-6.89 (m, 4H), 4.62-4.73 (m, 4H), 3.80 (s, 6H), 3.69 (br s, 2H), 3.36 (br s, 2H), 2.85 (s, 3H), 2.63 (br s, 2H), 2.34 (s, 3H), 1.44 (s, 9H).

**Step 4:** A mixture of compound tert-butyl (2-((((5-(bis(4-methoxybenzyl)amino)-3-chloropyrazin-2-yl)(methyl)amino)ethyl)(methyl)ca rbamate **(13-4)** (37 g, 64.90 mmol), 4-(4,4,5,5-tetramethyl-1,3,2-dioxaborolan-2-yl)-1-(m-tolyl)-1,2,3,6-tetrahydropyridine **(Intermediate B)** (25 g, 83.55 mmol) and potassium phosphate (35 g, 164.89 mmol) was added to tetrahydrofuran (250 mL) and water (50 mL), and the reaction solution was rerplaced with nitrogen followed by the addition of XPhos-Pd-G2 (1.65 g, 1.95 mmol), then heated to 65 °C with stirring under nitrogen protection for 16 hours; the reaction was essentially completed. To the mixture was added 500 ml of saturated saline and extracted with ethyl acetate; the organic phase was dried with anhydrous magnesium sulfate, filtered, concentrated and then dissolved with 500 ml of methyl tert-butyl ether, washed with 10% phosphoric acid aqueous solution (150 ml x 3), and the aqueous phase was adjusted to pH 13-14 with 10-15%sodium hydroxide aqueous solution. After extraction with ethyl acetate, the organic phase was dried with anhydrous magnesium sulfate, filtered and concentrated to give 101 g of brown viscous crude product tert-butyl (2-(((5-(bis(4-methoxybenzyl)amino)-3-(1-(m-tolyl)-1,2,3,6-tetrahydropyridin-4-yl)pyrazin-2 -yl)methyl)(methyl)amino)ethyl)(methyl)carbamate **(13-5),** which was used directly in the next step. $^1$H NMR (400 MHz, $CDCl_3$) δ (ppm) 7.84 (s, 1H), 7.13-7.19 (m, 4H), 6.85 (br d, 4H), 6.77-6.82 (m, 2H), 6.67 (br d, 1H), 6.57 (br s, 1H), 4.72 (s, 4H), 4.13 (q, 1H), 3.93 (br s, 2H), 3.80 (s, 6H), 3.54-3.64 (m, 2H), 3.49 (br t, 2H), 3.20-3.39 (m, 1H), 2.69-2.85 (m, 5H), 2.48-2.66 (m, 2H), 2.35 (s, 3H), 2.28 (br s, 2H), 2.05 (s, 1H), 1.47 (br s, 1H), 1.43 (br s, 9H).

**Step 4:** To a solution of compound tert-butyl (2-(((5-(bis(4-methoxybenzyl)amino)-3-(1-(m-tolyl)-1,2,3,6-tetrahydropyridin-4-yl)pyrazin-2 -yl)methyl)(methyl)amino)ethyl)(methyl)carbamate **(13-5)** (46.5 g, 65.78 mmol) in 600 ml of tetrahydrofuran was added palladium hydroxide/C (15 g, 21.36 mmol, 20% purity) under nitrogen protection, then stirred under hydrogen atmosphere (balloon) at room temperature for 4 days after hydrogen replacement. After filtration, the concentrated crude product was purified by silica gel column chromatography to give compound tert-butyl (2-(((5-(bis(4-methoxybenzyl)amino)-3-(1-(m-tolyl)piperidin-4-yl)pyrazin-2-yl)methyl)(met hyl)amino)ethyl)(methyl)carbamate **(13-6)** as a yellow viscous liquid (83.5 g, 117.78 mmol, 89.53% yield). $^1$H NMR (400 MHz, $CDCl_3$) δ (ppm) 7.75 (s, 1H), 7.10-7.19 (m, 5H), 6.77-6.90 (m, 6H), 6.67 (d, 1H), 4.69 (s, 4H), 3.79 (s, 7H), 3.60 (br s, 2H), 3.25-3.42 (m, 2H), 3.09 (br s, 1H), 2.74-2.85 (m, 5H), 2.48-2.65 (m, 2H), 2.32 (s, 3H), 2.27 (br s, 3H), 2.14 (dq, 2H), 2.05 (s, 1H), 1.86 (br d, 2H), 1.41 (br s, 9H).

**Step 4:** To a compound tert-butyl (2-(((5-(bis(4-methoxybenzyl)amino)-3-(1-(m-tolyl)piperidin-4-yl)pyrazin-2-yl)methyl)(met hyl)amino)ethyl)(methyl)carbamate **(13-6)** (56 g, 78.99 mmol) was added 300 mL of trifluoroacetic acid and then trifluoromethanesulfonic acid (30.0 g, 199.90 mmol, 17.65 mL), and the reaction solution was essentially complete afterstirringat room temperature for 16 h. The reaction solution was then concentrated under reduced pressure, added with 1 liter of water and then 2 liters of ethyl acetate, and stirred until the solid was dissolved.After separation and extraction, the organic phase was extracted with 1M hydrochloric acid solution, and the aqueous phase was neutralized with 30% sodium hydroxide aqueous solution to pH 9, andthen extracted with ethyl acetate; the organic phase was dried, concentrated, then purified by silica gel column chromatography to give compound N-((5-amino-3-(1-(m-tolyl)piperidin-4-yl)pyrazin-2-yl)methyl)-2-yl)methyl)-N,N'-dimethyl-1,2-ethylenediamine **(P292, Example 13),** as a white solid. LCMS: (ESI) m/z = 369.3 $[M+H]^+$ ; $^1$H NMR (400 MHz, methanol-d4) δ (ppm) 7.90 (s, 1H), 7.62 (s, 1H), 7.56 (br

d, 1H), 7.46 (t, 1H), 7.33 (d, 1H), 6.02 (s, 3H), 5.01 (br s, 25H), 4.50 (s, 2H), 3.88-3.99 (m, 2H), 3.74 (br d, 2H), 3.42-3.63 (m, 5H), 2.90 (s, 3H), 2.81 (s, 3H), 2.41-2.56 (m, 5H), 2.19-2.32 (m, 20H), 2.13 (br d, 3H).

Similar methods as described above were used to synthesize the compounds P001-P448, the characterization data of which were shown in the right column:

| Patent_ ID | Structure | [M+H]+ (ESI) m/z | Patent_ ID | Structure | [M+H]+ (ESI) m/z |
|---|---|---|---|---|---|
| P001 | 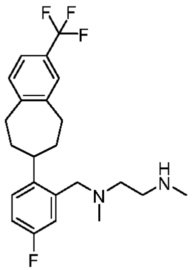 | 409.2 | P196 | 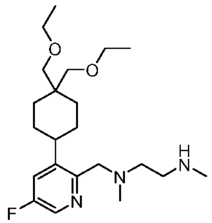 | 396.1 |
| P002 | 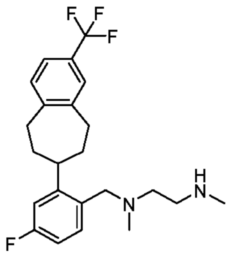 | 409.2 | P197 | 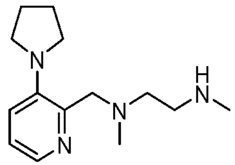 | 249.3 |
| P003 | 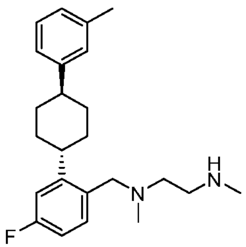 | 369.2 | P198 | 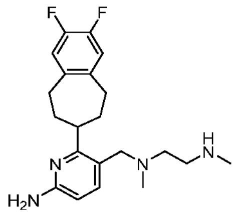 | 375.3 |
| P005 | 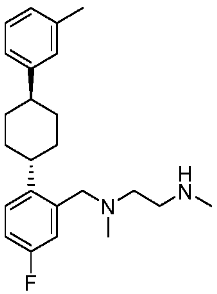 | 369.3 | P199 | 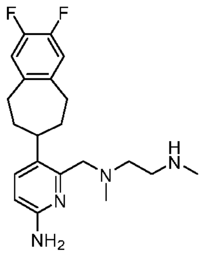 | 375.2 |

(continued)

| Patent_ ID | Structure | [M+H]+ (ESI) m/z | Patent_ ID | Structure | [M+H]+ (ESI) m/z |
|---|---|---|---|---|---|
| P006 | 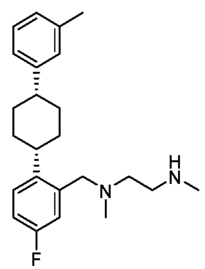 | 369.2 | P200 | 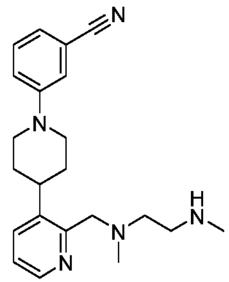 | 364.3 |
| P007 | 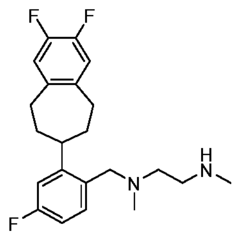 | 377.2 | P201 | 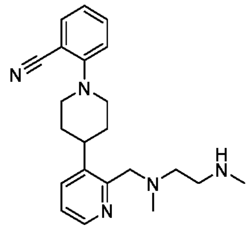 | 364.0 |
| P008 | 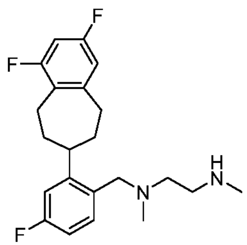 | 377.2 | P202 | 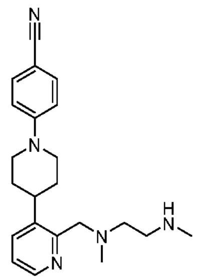 | 364.0 |
| P009 | 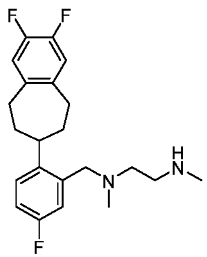 | 377.2 | P203 | 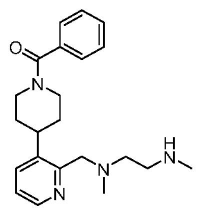 | 367.3 |

(continued)

| Patent_ ID | Structure | [M+H]+ (ESI) m/z | Patent_ ID | Structure | [M+H]+ (ESI) m/z |
|---|---|---|---|---|---|
| P010 | 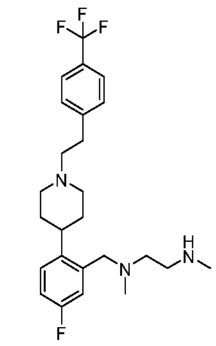 | 452.4 | P204 | 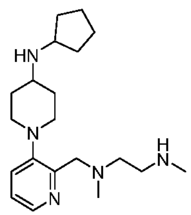 | 346.3 |
| P011 | 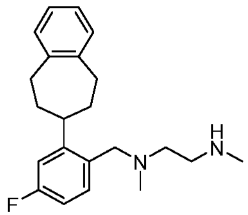 | 341.2 | P205 | 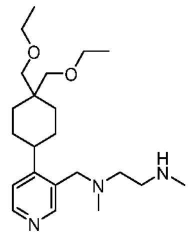 | 378.2 |
| P012 | 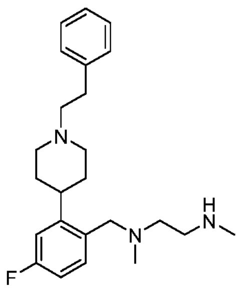 | 384.2 | P206 | 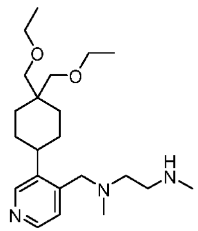 | 378.2 |

(continued)

| Patent_ ID | Structure | $[M+H]^+$ (ESI) m/z | Patent_ ID | Structure | $[M+H]^+$ (ESI) m/z |
|---|---|---|---|---|---|
| P013 | 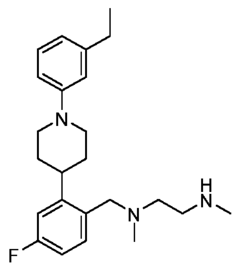 | 384.3 | P207 | 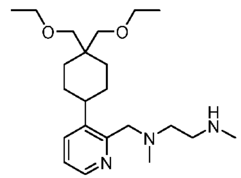 | 378.4 |
| P014 | 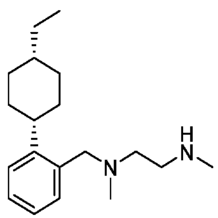 | 289.1 | P208 | 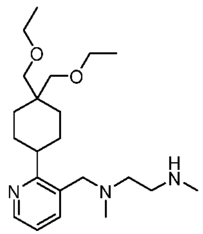 | 378.3 |
| P015 | 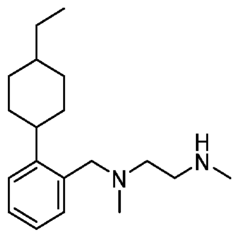 | 289.1 | P209 | 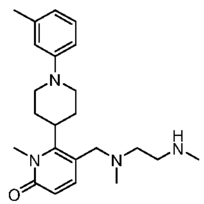 | |
| P016 | 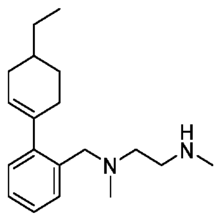 | 287.0 | P210 | 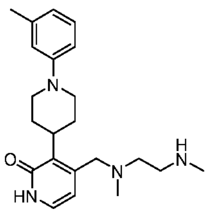 | 369.3 |

(continued)

| Patent_ ID | Structure | [M+H]+ (ESI) m/z | Patent_ ID | Structure | [M+H]+ (ESI) m/z |
|---|---|---|---|---|---|
| P017 | 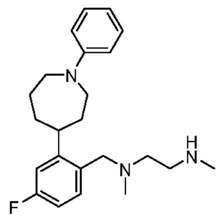 | 370.3 | P211 | 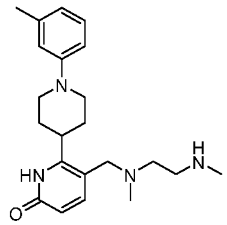 | 369.2 |
| P018 | 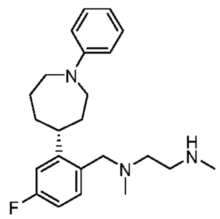 | 370.3 | P212 | 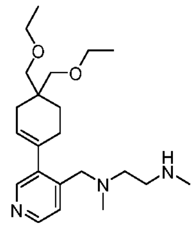 | 376.1 |
| P019 | 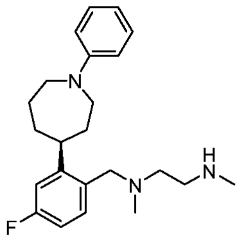 | 370.3 | P213 | 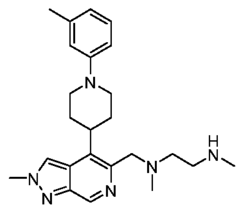 | |
| P020 | 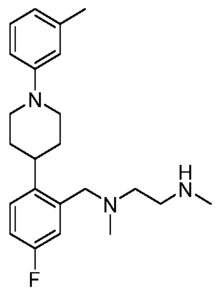 | 370.2 | P214 | 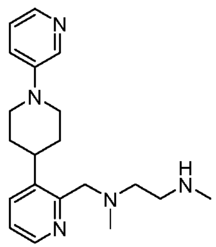 | 340.2 |

(continued)

| Patent_ ID | Structure | [M+H]+ (ESI) m/z | Patent_ ID | Structure | [M+H]+ (ESI) m/z |
|---|---|---|---|---|---|
| P021 | 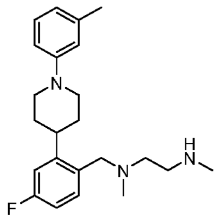 | 370.4 | P215 | 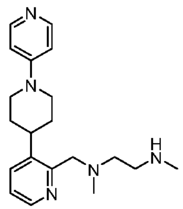 | 340.2 |
| P022 | 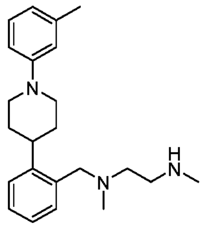 | 351.9 | P216 | 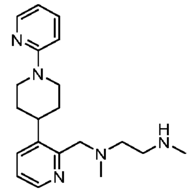 | 340.3 |
| P023 | 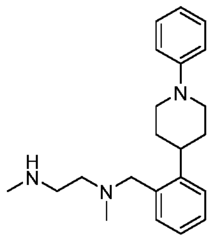 | 338.3 | P217 | 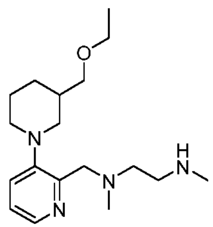 | 321.0 |
| P024 | 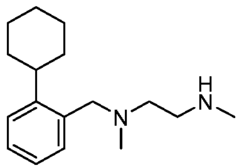 | 261.1 | P218 | 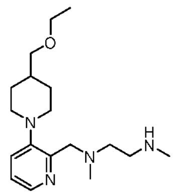 | 321.0 |

(continued)

| Patent_ ID | Structure | $[M+H]^+$ (ESI) m/z | Patent_ ID | Structure | $[M+H]^+$ (ESI) m/z |
|---|---|---|---|---|---|
| P025 | 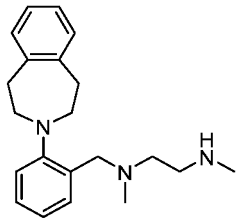 | 324.2 | P219 | 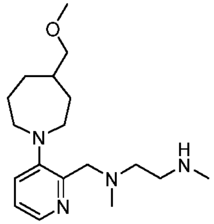 | 321.0 |
| P026 | 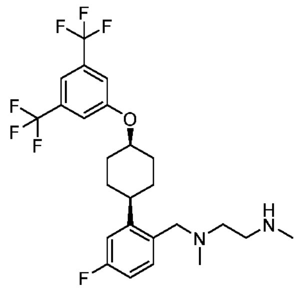 | 507.2 | P220 | 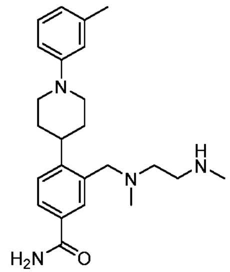 | 395.2 |
| P027 | 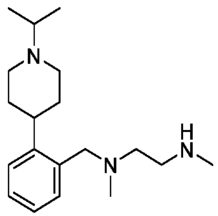 | 304.1 | P221 | 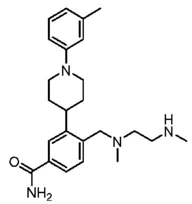 | 395.2 |
| P028 | 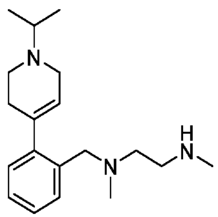 | 302.3 | P222 | 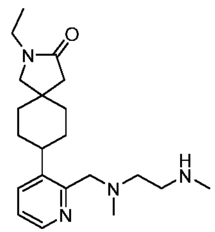 | 359.4 |

(continued)

| Patent_ ID | Structure | $[M+H]^+$ (ESI) m/z | Patent_ ID | Structure | $[M+H]^+$ (ESI) m/z |
|---|---|---|---|---|---|
| P029 | 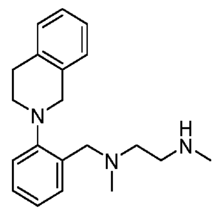 | 310.2 | P224 | 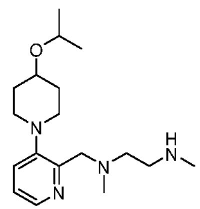 | 320.9 |
| P030 | 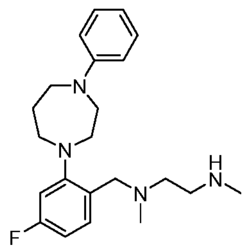 | 371.3 | P225 | 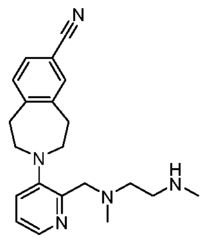 | 350.3 |
| P031 | 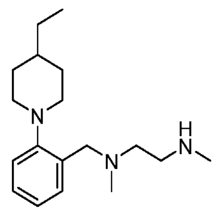 | 290.3 | P226 | 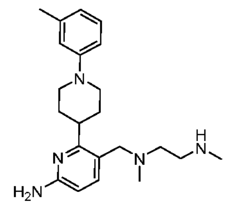 | 368.3 |
| P032 | 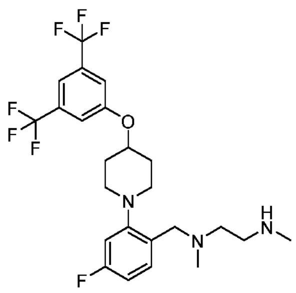 | 508.2 | P227 | 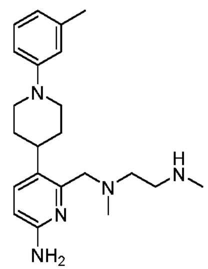 | 368.3 |

(continued)

| Patent_ ID | Structure | $[M+H]^+$ (ESI) m/z | Patent_ ID | Structure | $[M+H]^+$ (ESI) m/z |
|---|---|---|---|---|---|
| P033 | 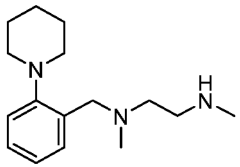 | 262.2 | P228 | 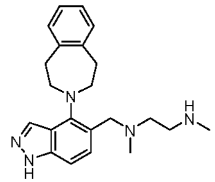 | 364.3 |
| P034 | 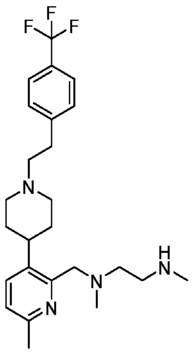 | 449.4 | P229 | 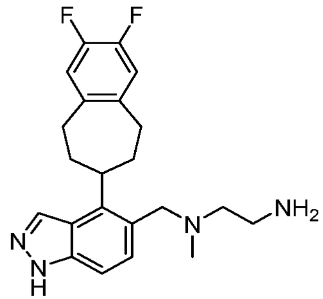 | |
| P035 | 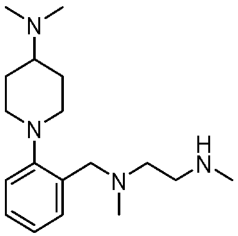 | 305.3 | P230 | 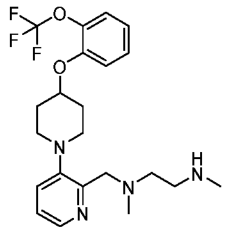 | 439.3 |
| P036 | 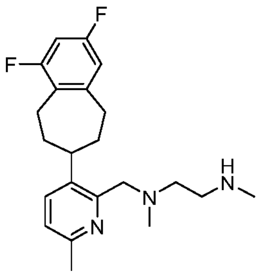 | 374.3 | P231 | 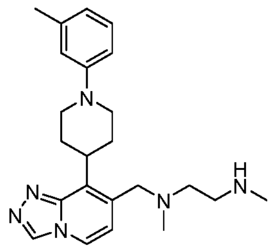 | |

(continued)

| Patent_ ID | Structure | $[M+H]^+$ (ESI) m/z | Patent_ ID | Structure | $[M+H]^+$ (ESI) m/z |
|---|---|---|---|---|---|
| P037 | 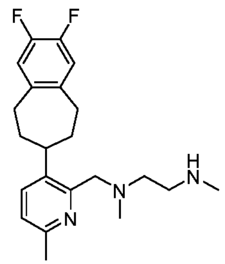 | 374.2 | P232 | 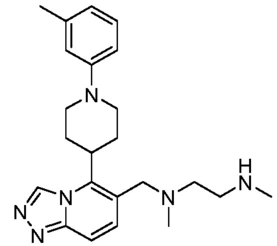 | 393.3 |
| P038 | 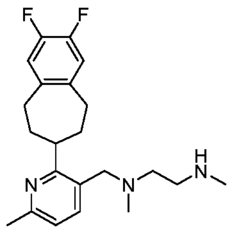 | 374.2 | P233 | 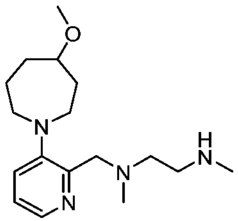 | 306.9 |
| P039 | 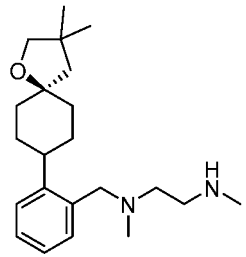 | 345.1 | P234 | 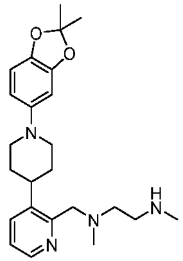 | 411.4 |
| P040 | 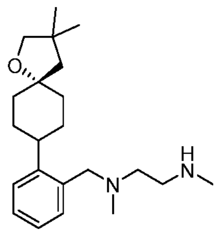 | 345.1 | P235 | 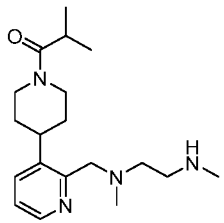 | 333.3 |

(continued)

| Patent_ ID | Structure | [M+H]+ (ESI) m/z | Patent_ ID | Structure | [M+H]+ (ESI) m/z |
|---|---|---|---|---|---|
| P042 | 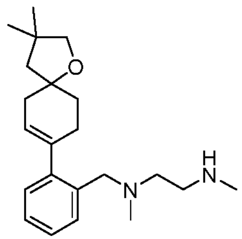 | 343.3 | P236 | 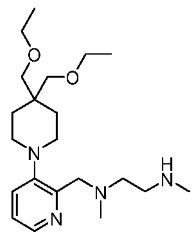 | 379.3 |
| P043 | 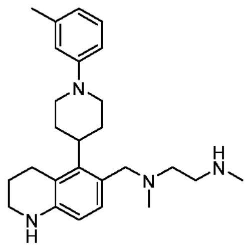 | | P237 | 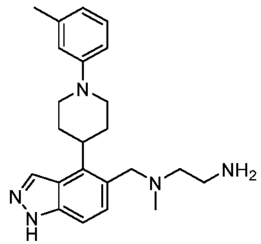 | |
| P044 | 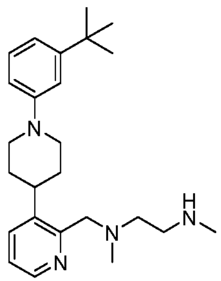 | 395.3 | P238 | 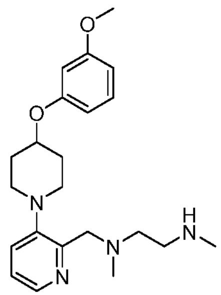 | 385.0 |
| P046 | 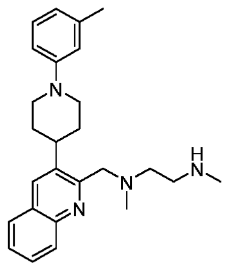 | 403.3 | P239 | 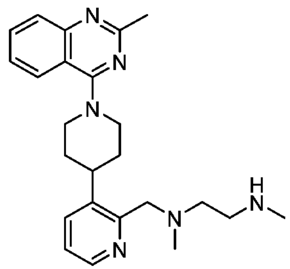 | 405.2 |

(continued)

| Patent_ ID | Structure | [M+H]+ (ESI) m/z | Patent_ ID | Structure | [M+H]+ (ESI) m/z |
|---|---|---|---|---|---|
| P047 | 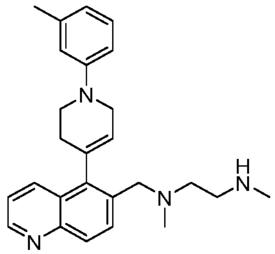 | 401.3 | P240 | 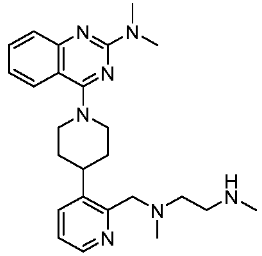 | 434.3 |
| P048 | 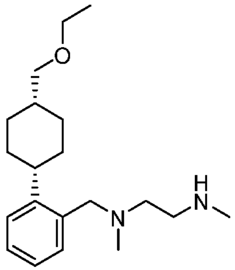 | 319.3 | P241 | 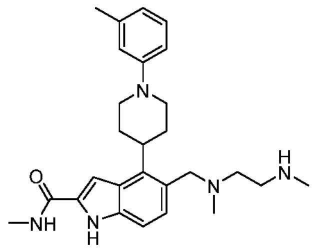 | |
| P049 | 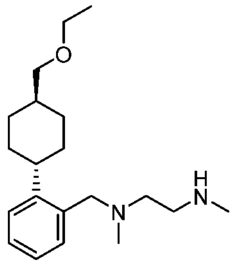 | 319.3 | P242 | 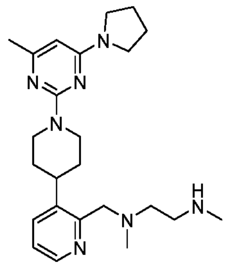 | 424.2 |
| P050 | 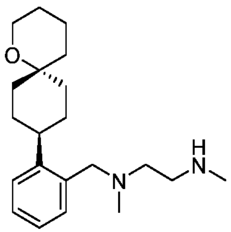 | 331.1 | P243 | 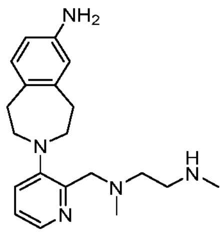 | 340.3 |

(continued)

| Patent_ ID | Structure | [M+H]+ (ESI) m/z | Patent_ ID | Structure | [M+H]+ (ESI) m/z |
|---|---|---|---|---|---|
| P051 | 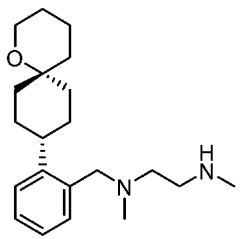 | 331.1 | P244 | 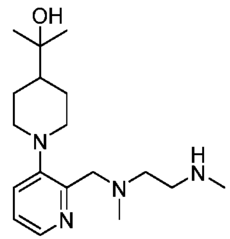 | 320.9 |
| P052 | 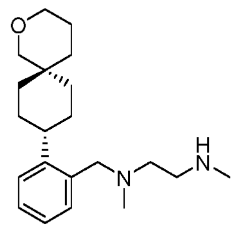 | 331.1 | P245 | 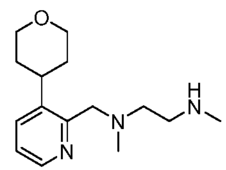 | 264.2 |
| P053 | 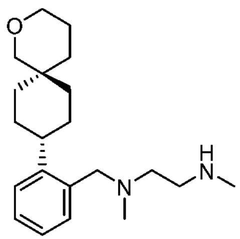 | 331.1 | P246 | 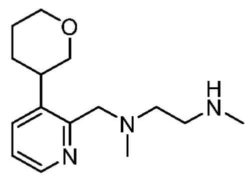 | 264.3 |
| P054 | 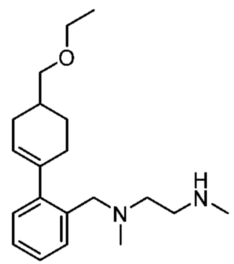 | 317.1 | P247 | 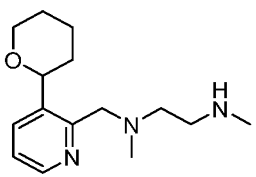 | 264.2 |

(continued)

| Patent_ ID | Structure | $[M+H]^+$ (ESI) m/z | Patent_ ID | Structure | $[M+H]^+$ (ESI) m/z |
|---|---|---|---|---|---|
| P055 | 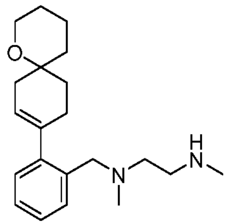 | 329.1 | P248 | 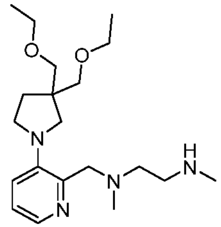 | 365.0 |
| P056 | 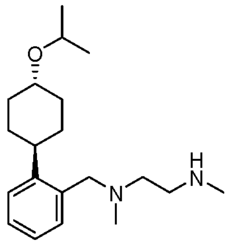 | 319.1 | P249 | 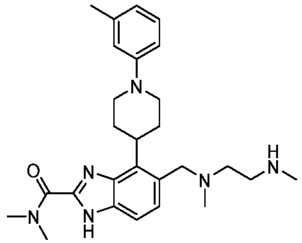 | |
| P057 | 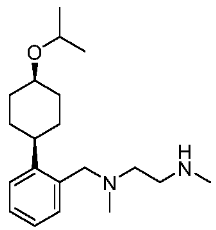 | 319.1 | P250 | 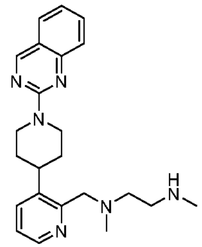 | 391.3 |
| P058 | 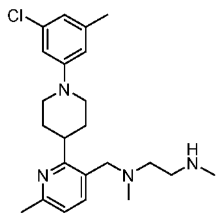 | 401.3 | P251 | 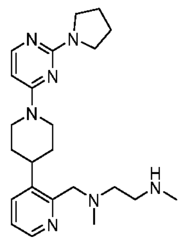 | 410.0 |

(continued)

| Patent_ ID | Structure | [M+H]+ (ESI) m/z | Patent_ ID | Structure | [M+H]+ (ESI) m/z |
|---|---|---|---|---|---|
| P059 | 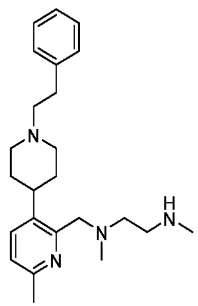 | 381.0 | P252 | 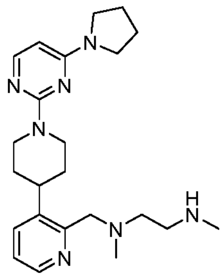 | 410.3 |
| P060 | 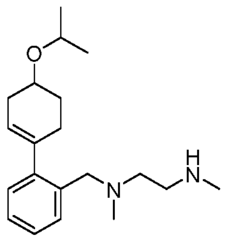 | 317.1 | P253 | 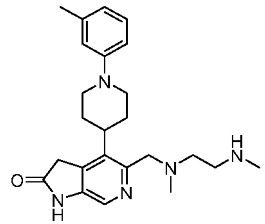 | |
| P061 | 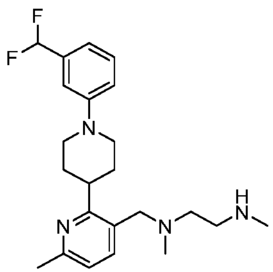 | 403.3 | P254 | 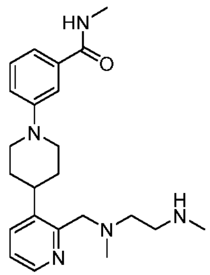 | 396.3 |

(continued)

| Patent_ ID | Structure | [M+H]+ (ESI) m/z | Patent_ ID | Structure | [M+H]+ (ESI) m/z |
|---|---|---|---|---|---|
| P062 | 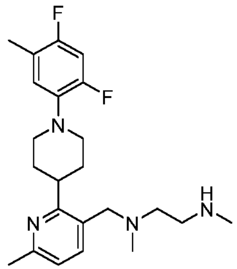 | 403.3 | P255 | 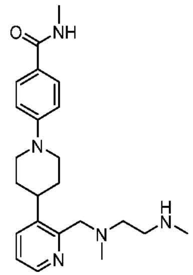 | 396.3 |
| P063 | 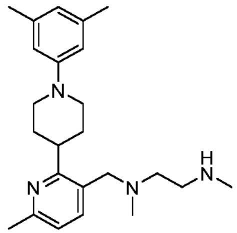 | 381.3 | P256 | 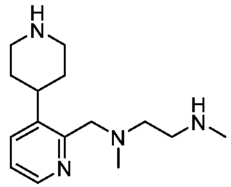 | 363.3 |
| P064 | 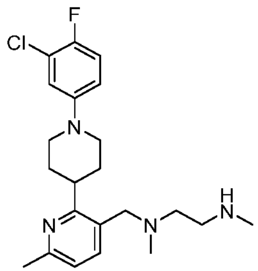 | 405.3 | | | |

(continued)

| Patent_ ID | Structure | [M+H]+ (ESI) m/z | Patent_ ID | Structure | [M+H]+ (ESI) m/z |
|---|---|---|---|---|---|
| P065 | 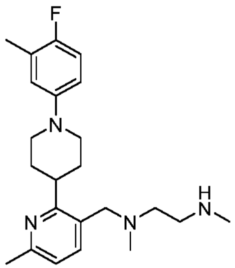 | 385.2 | P258 | 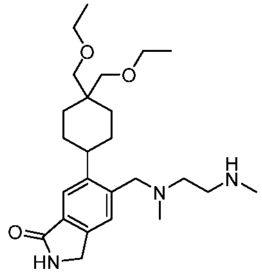 | 432.4 |
| P066 | 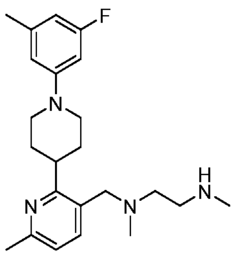 | 385.3 | P259 | 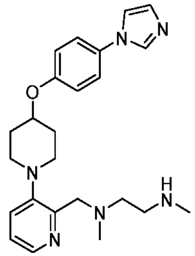 | 421.3 |
| P067 | 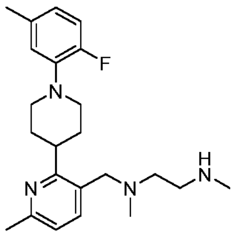 | 385.2 | P260 | 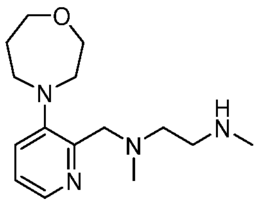 | 278.9 |
| P068 | 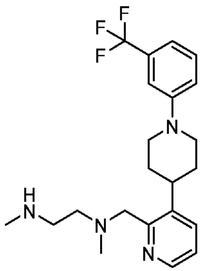 | 407.0 | P261 | 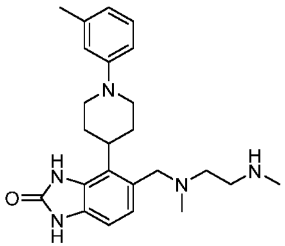 | 408.3 |

(continued)

| Patent_ ID | Structure | $[M+H]^+$ (ESI) m/z | Patent_ ID | Structure | $[M+H]^+$ (ESI) m/z |
|---|---|---|---|---|---|
| P069 | 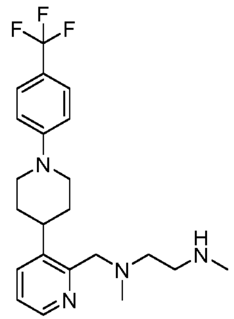 | 406.9 | P262 | 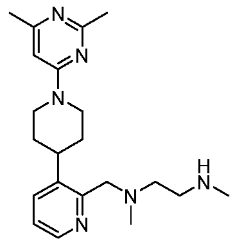 | 369.4 |
| P070 | 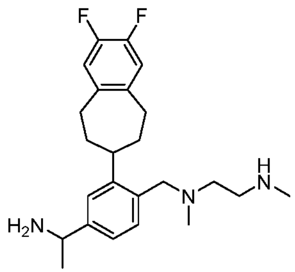 | 402.2 | P263 | 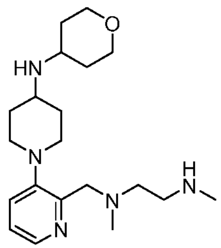 | 362.2 |
| P071 | 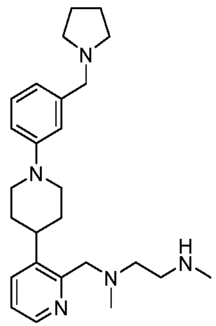 | 422.4 | P264 | 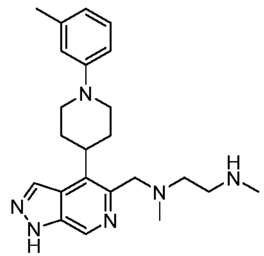 | |

(continued)

| Patent_ ID | Structure | [M+H]+ (ESI) m/z | Patent_ ID | Structure | [M+H]+ (ESI) m/z |
|---|---|---|---|---|---|
| P072 | 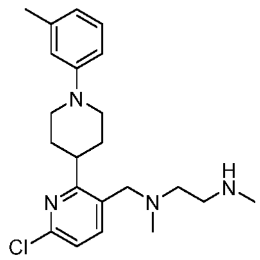 | 387.3 | P265 | 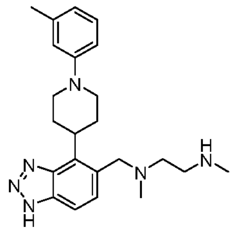 | 393.2 |
| P073 | 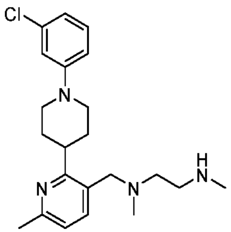 | 387.3 | P266 | 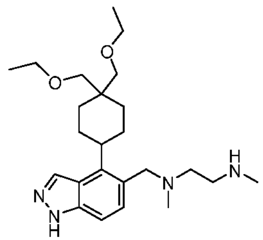 | 417.4 |
| P074 | 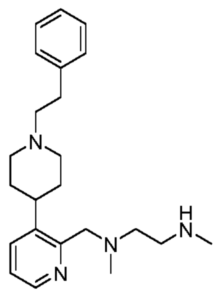 | 367.4 | P267 | 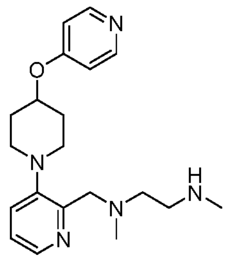 | 356.3 |
| P075 | 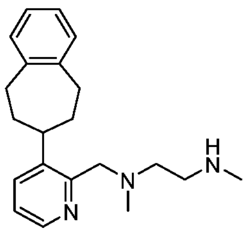 | 324.3 | P268 | 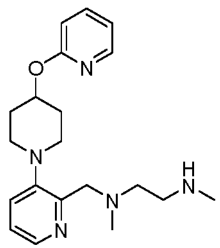 | 356.3 |

(continued)

| Patent_ ID | Structure | [M+H]+ (ESI) m/z | Patent_ ID | Structure | [M+H]+ (ESI) m/z |
|---|---|---|---|---|---|
| P076 | 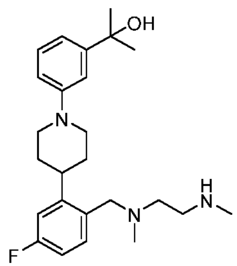 | 414.3 | P269 | 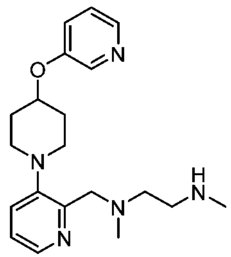 | 356.3 |
| P077 | 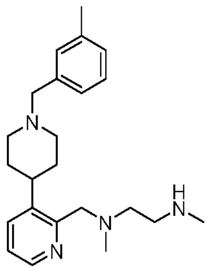 | 367.1 | P270 | 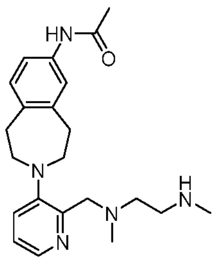 | 382.3 |
| P078 | 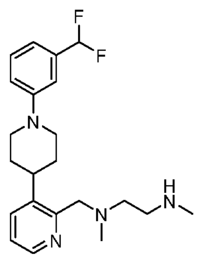 | 389.0 | P271 | 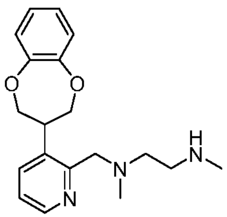 | 327.9 |
| P079 | 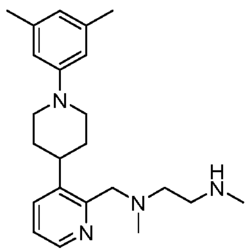 | 367.4 | P272 | 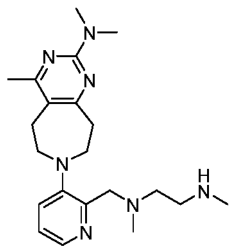 | 384.3 |

(continued)

| Patent_ ID | Structure | [M+H]+ (ESI) m/z | Patent_ ID | Structure | [M+H]+ (ESI) m/z |
|---|---|---|---|---|---|
| P080 | 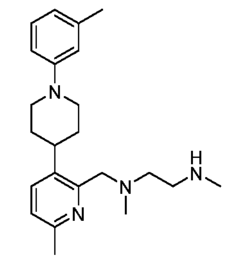 | 367.3 | P273 | 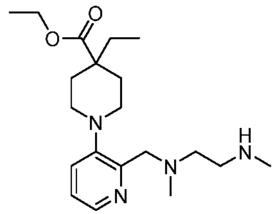 | 363.0 |
| P081 | 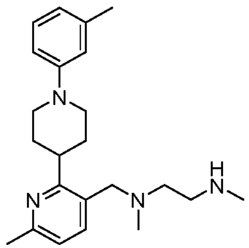 | 367.3 | P274 | 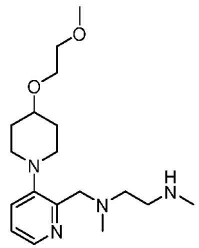 | 337.1 |
| P082 | 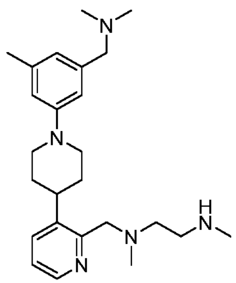 | 410.0 | P275 | 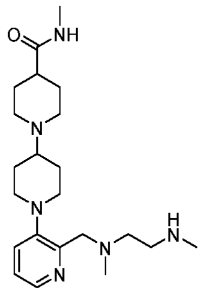 | 403.3 |

(continued)

| Patent_ ID | Structure | [M+H]+ (ESI) m/z | Patent_ ID | Structure | [M+H]+ (ESI) m/z |
|---|---|---|---|---|---|
| P083 | 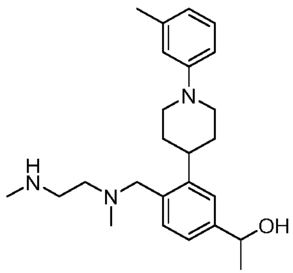 | 396.3 | P276 | 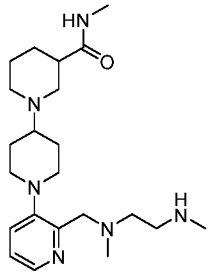 | 403.4 |
| P084 | 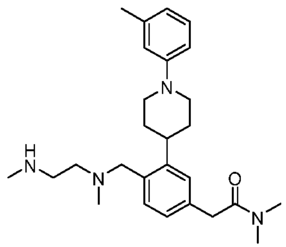 | 437.3 | P277 | 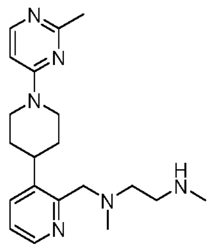 | 355.3 |
| P085 | 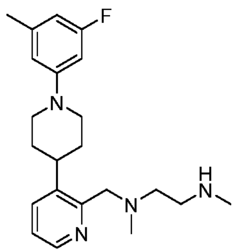 | 371.2 | P278 | 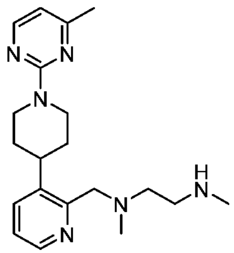 | 354.9 |
| P086 | 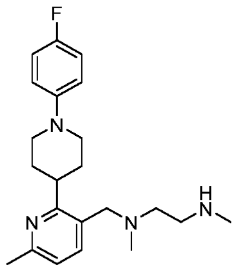 | 371.3 | P279 | 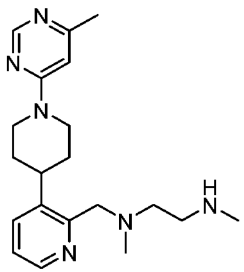 | 355.3 |

(continued)

| Patent_ ID | Structure | $[M+H]^+$ (ESI) m/z | Patent_ ID | Structure | $[M+H]^+$ (ESI) m/z |
|---|---|---|---|---|---|
| P087 | 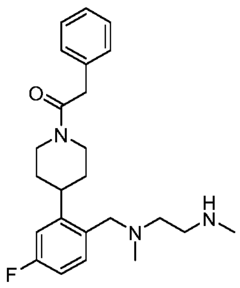 | 398.3 | P280 | 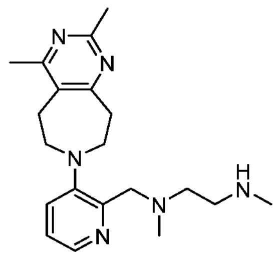 | 355.2 |
| P088 | 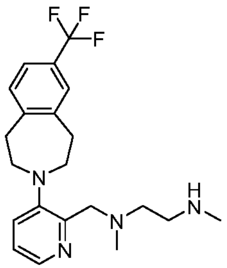 | 393.4 | P281 | 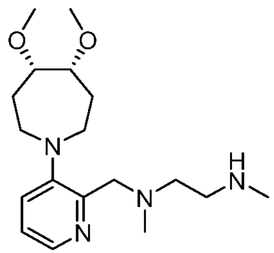 | 336.9 |
| P089 | 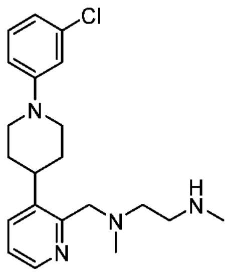 | 372.9 | P282 | 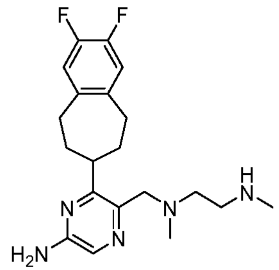 | |

(continued)

| Patent_ ID | Structure | [M+H]⁺ (ESI) m/z | Patent_ ID | Structure | [M+H]⁺ (ESI) m/z |
|---|---|---|---|---|---|
| P090 | 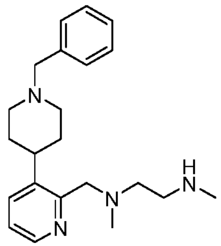 | 353.3 | P283 | 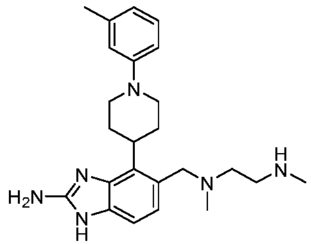 | |
| P091 | 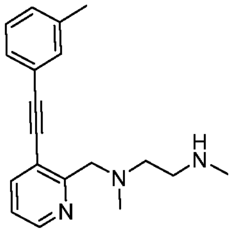 | 293.9 | P284 | 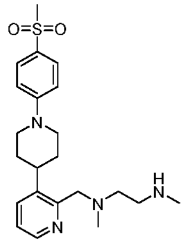 | 417.0 |
| P092 | 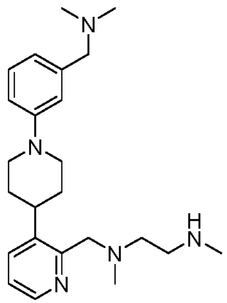 | 396.4 | P285 | 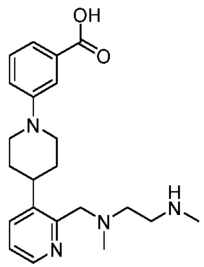 | 383.3 |

(continued)

| Patent_ ID | Structure | $[M+H]^+$ (ESI) m/z | Patent_ ID | Structure | $[M+H]^+$ (ESI) m/z |
|---|---|---|---|---|---|
| P093 | 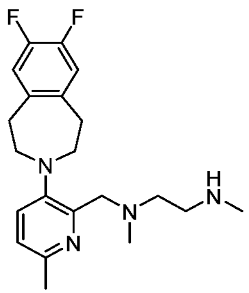 | 374.9 | P286 | 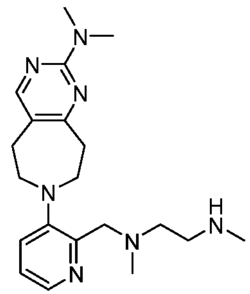 | 370.3 |
| P094 | 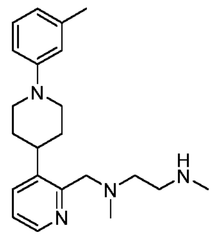 | 353.3 | P287 | 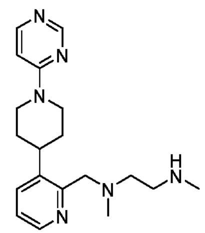 | 341.3 |
| P095 | 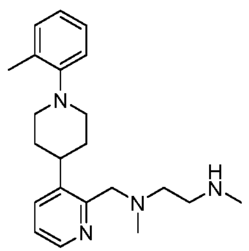 | 353.3 | P288 | 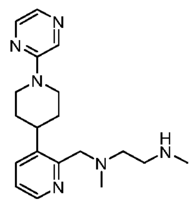 | 341.3 |

(continued)

| Patent_ ID | Structure | $[M+H]^+$ (ESI) m/z | Patent_ ID | Structure | $[M+H]^+$ (ESI) m/z |
|---|---|---|---|---|---|
| P096 | 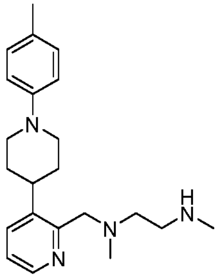 | 353.3 | P289 | 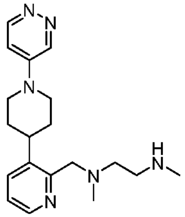 | 341.3 |
| P097 | 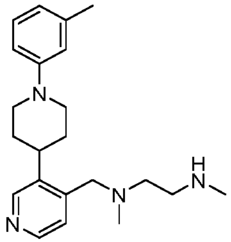 | 353.2 | P290 | 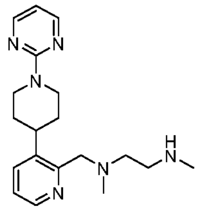 | 340.9 |
| P098 | 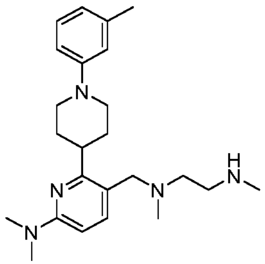 | 396.2 | P291 | 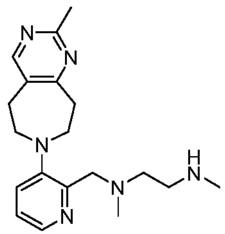 | 341.2 |
| P099 | 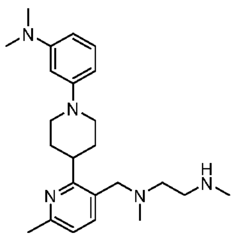 | 396.2 | P292 | 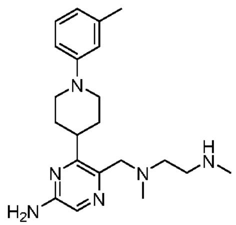 | 369.2 |

(continued)

| Patent_ ID | Structure | $[M+H]^+$ (ESI) m/z | Patent_ ID | Structure | $[M+H]^+$ (ESI) m/z |
|---|---|---|---|---|---|
| P100 | 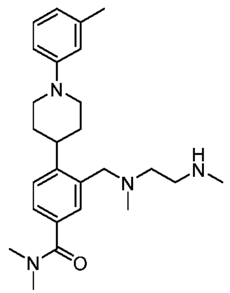 | 423.2 | P293 | 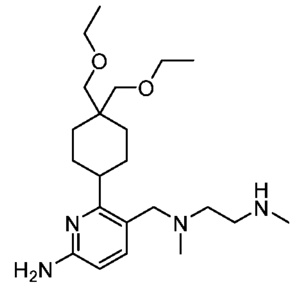 | 393.3 |
| P101 | 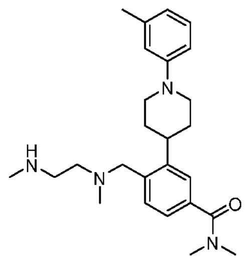 | 423.2 | P294 | 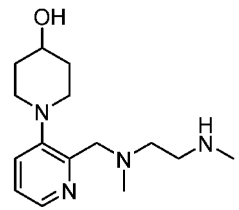 | 279.3 |
| P102 | 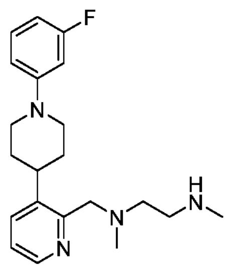 | 357.0 | P295 | 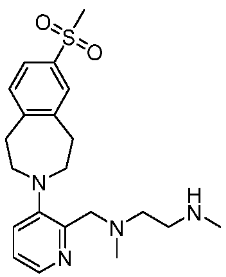 | 403.2 |

(continued)

| Patent_ ID | Structure | [M+H]+ (ESI) m/z | Patent_ ID | Structure | [M+H]+ (ESI) m/z |
|---|---|---|---|---|---|
| P103 | 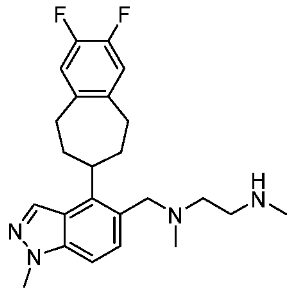 | 413.4 | P296 | 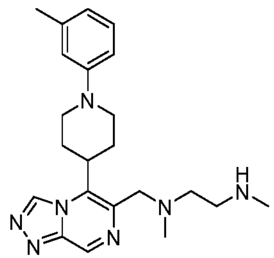 | |
| P104 | 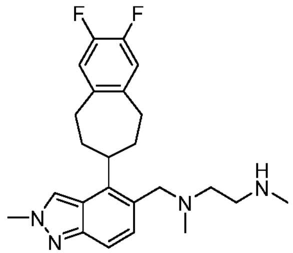 | 413.3 | P297 | 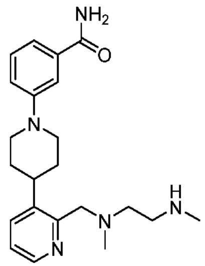 | 382.3 |
| P105 | 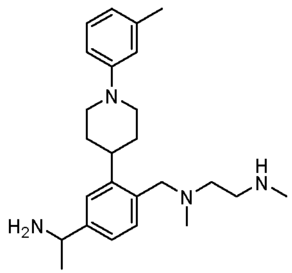 | 395.3 | P298 | 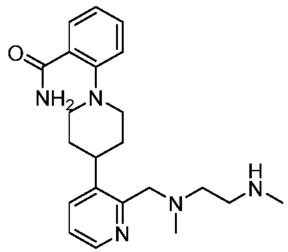 | 382.0 |

(continued)

| Patent_ ID | Structure | [M+H]$^+$ (ESI) m/z | Patent_ ID | Structure | [M+H]$^+$ (ESI) m/z |
|---|---|---|---|---|---|
| P106 | 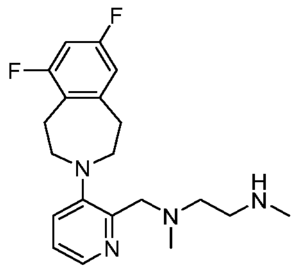 | 361.0 | P299 | 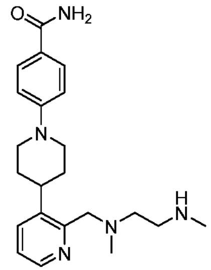 | 382.0 |
| P107 | 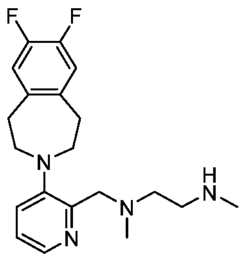 | 361.2 | P300 | 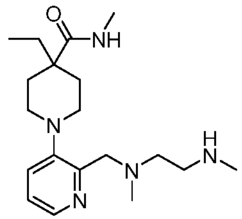 | 348.0 |
| P108 | 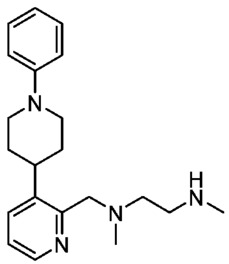 | 339.3 | P301 | 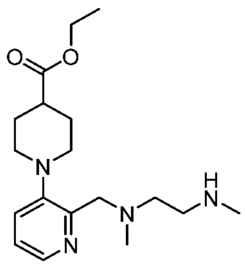 | 335.0 |

(continued)

| Patent_ ID | Structure | [M+H]+ (ESI) m/z | Patent_ ID | Structure | [M+H]+ (ESI) m/z |
|---|---|---|---|---|---|
| P109 | 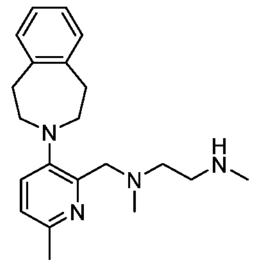 | 339.2 | P302 | 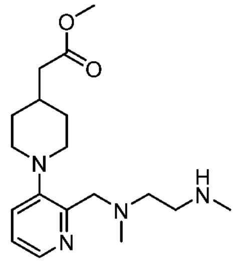 | 335.0 |
| P110 | 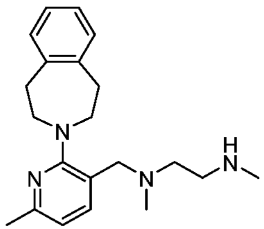 | 339.3 | P303 | 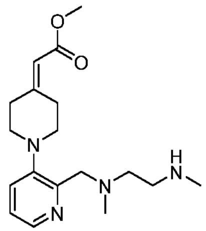 | 333.2 |
| P111 | 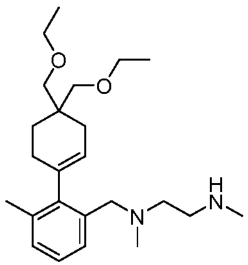 | 389.3 | P304 | 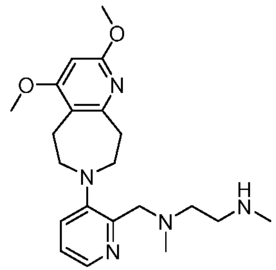 | 386.0 |
| P112 | 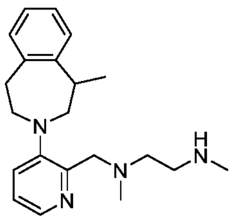 | 339.3 | P305 | 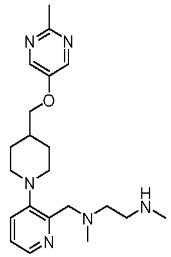 | 385.0 |

(continued)

| Patent_ ID | Structure | [M+H]+ (ESI) m/z | Patent_ ID | Structure | [M+H]+ (ESI) m/z |
|---|---|---|---|---|---|
| P113 | 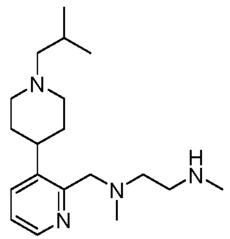 | 319.3 | P306 | 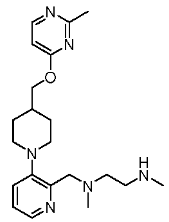 | 385.3 |
| P114 | 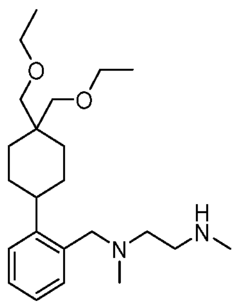 | 377.2 | P307 | 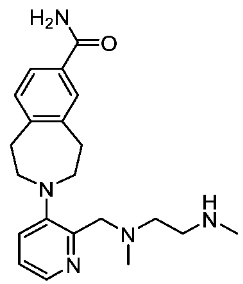 | 368.3 |
| P115 | 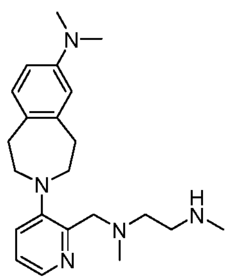 | 368.3 | P308 | 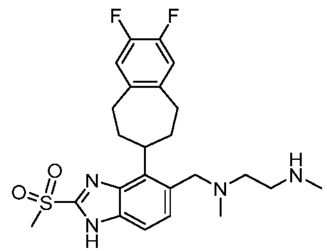 | |

(continued)

| Patent_ ID | Structure | [M+H]+ (ESI) m/z | Patent_ ID | Structure | [M+H]+ (ESI) m/z |
|---|---|---|---|---|---|
| P116 | 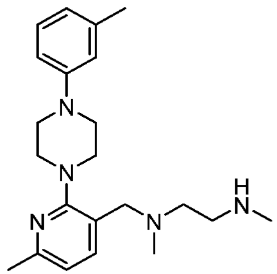 | 368.3 | P309 | 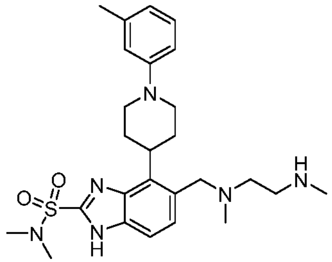 | |
| P117 | 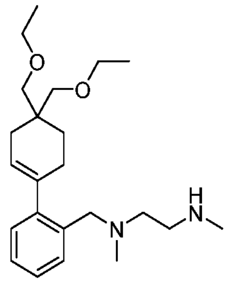 | 375.2 | P310 | 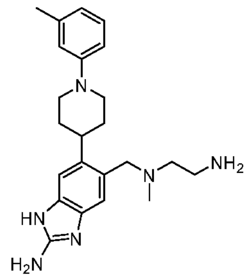 | |
| P118 | 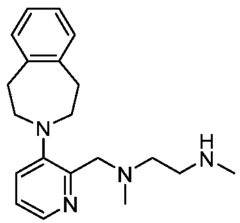 | 325.3 | P311 | 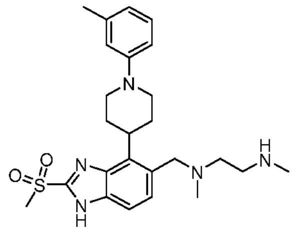 | |
| P119 | 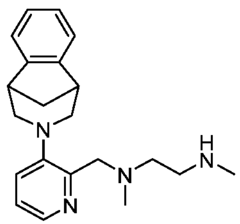 | 337.0 | P312 | 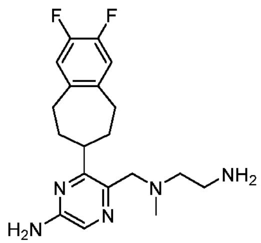 | |

(continued)

| Patent_ ID | Structure | $[M+H]^+$ (ESI) m/z | Patent_ ID | Structure | $[M+H]^+$ (ESI) m/z |
|---|---|---|---|---|---|
| P120 | 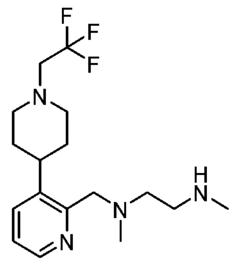 | 345.3 | P313 | 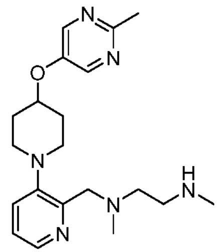 | 371.2 |
| P122 | 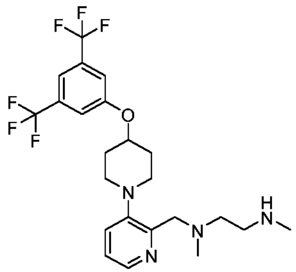 | 491.4 | P314 | 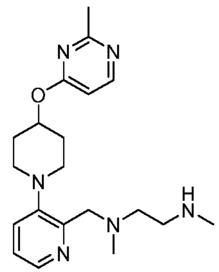 | 371.3 |
| P123 | 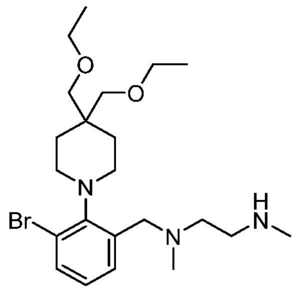 | 456.2 | P315 | 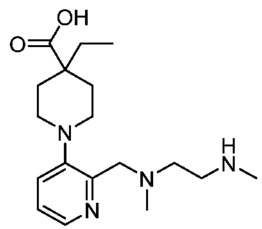 | 335.0 |

(continued)

| Patent_ ID | Structure | [M+H]+ (ESI) m/z | Patent_ ID | Structure | [M+H]+ (ESI) m/z |
|---|---|---|---|---|---|
| P124 | 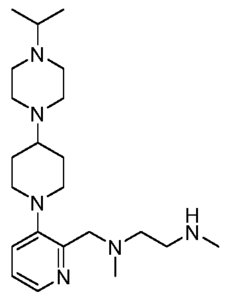 | 389.3 | P316 | 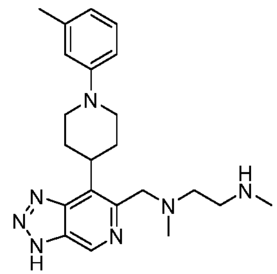 | |
| P125 | 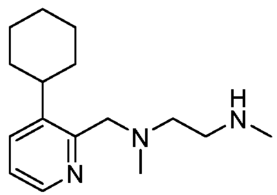 | 262.3 | P317 | 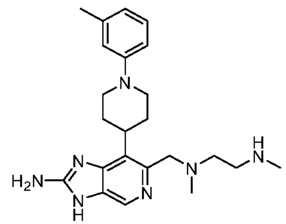 | |
| P126 | 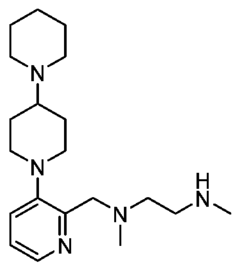 | 346.3 | P318 | 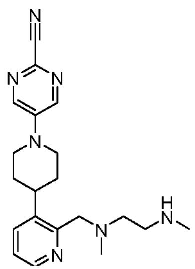 | 366.0 |
| P127 | 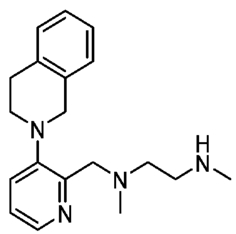 | 311.2 | P319 | 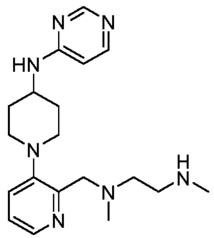 | 356.3 |

(continued)

| Patent_ ID | Structure | $[M+H]^+$ (ESI) m/z | Patent_ ID | Structure | $[M+H]^+$ (ESI) m/z |
|---|---|---|---|---|---|
| P128 | 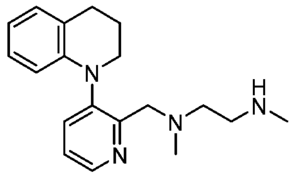 | 311.3 | P320 | 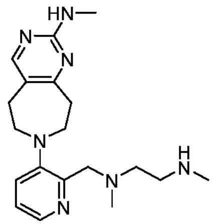 | 356.2 |
| P129 | 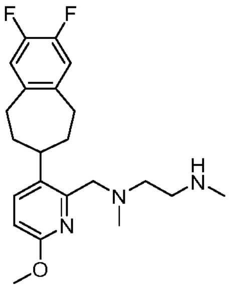 | 390.3 | P321 | 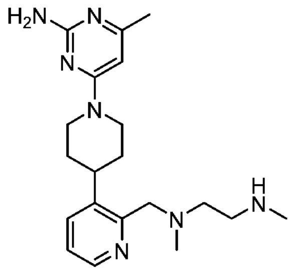 | 370.3 |
| P130 | 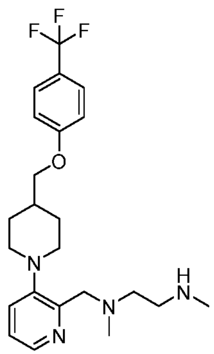 | 437.3 | P322 | 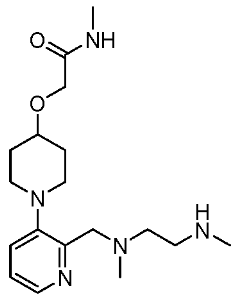 | 350.4 |

(continued)

| Patent_ ID | Structure | $[M+H]^+$ (ESI) m/z | Patent_ ID | Structure | $[M+H]^+$ (ESI) m/z |
|---|---|---|---|---|---|
| P131 | 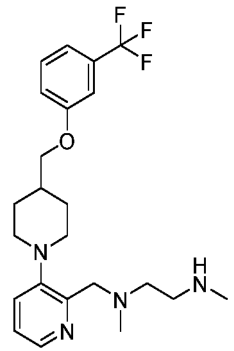 | 437.3 | P323 | 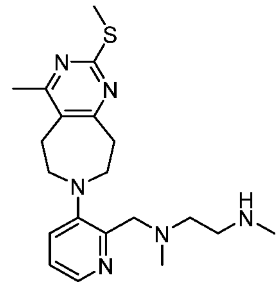 | 387.0 |
| P132 | 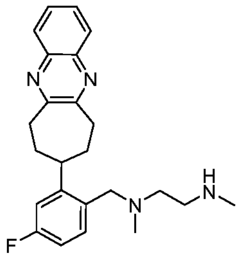 | 393.1 | P324 | 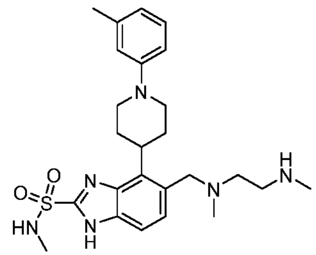 | |
| P133 | 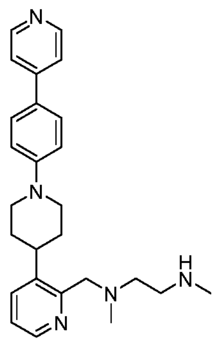 | 416.0 | P325 | 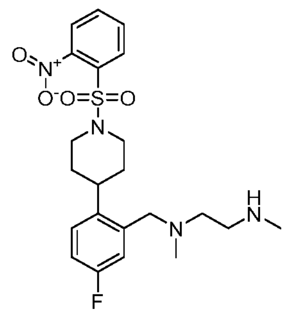 | 465.2 |

(continued)

| Patent_ ID | Structure | $[M+H]^+$ (ESI) m/z | Patent_ ID | Structure | $[M+H]^+$ (ESI) m/z |
|---|---|---|---|---|---|
| P134 | 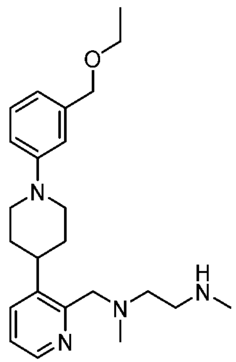 | 397.0 | P326 | 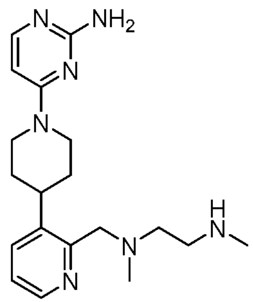 | 356.2 |
| P135 | 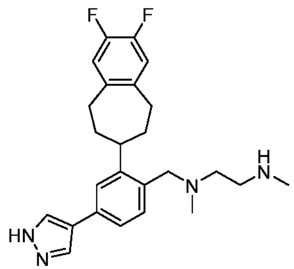 | 425.2 | P327 | 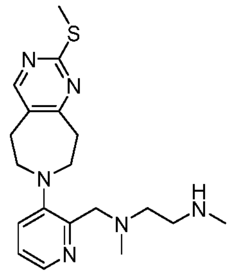 | 373.4 |
| P136 | 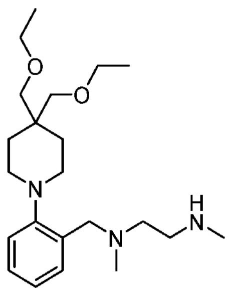 | 378.2 | P328 | 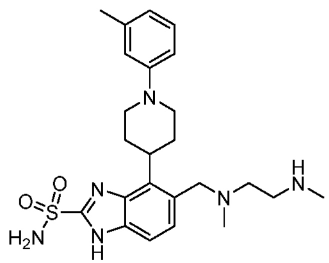 | |

(continued)

| Patent_ ID | Structure | $[M+H]^+$ (ESI) m/z | Patent_ ID | Structure | $[M+H]^+$ (ESI) m/z |
|---|---|---|---|---|---|
| P137 | 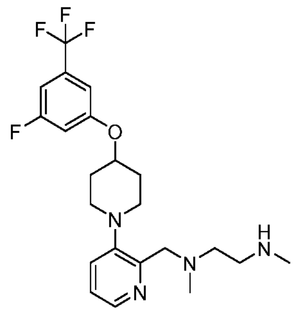 | 441.3 | P329 | 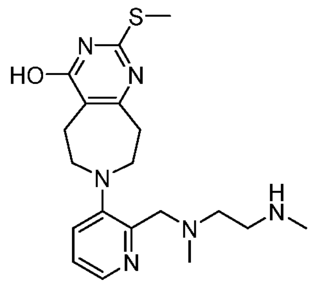 | 389.2 |
| P138 | 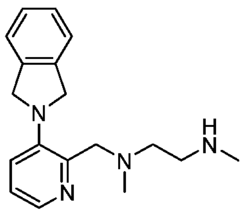 | 297.2 | P330 | 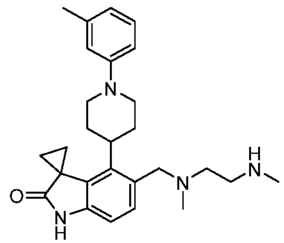 | |
| P139 | 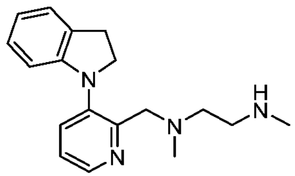 | 295.2 | P350 | 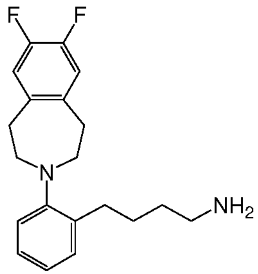 | 331.1 |

(continued)

| Patent_ ID | Structure | $[M+H]^+$ (ESI) m/z | Patent_ ID | Structure | $[M+H]^+$ (ESI) m/z |
|---|---|---|---|---|---|
| P140 | 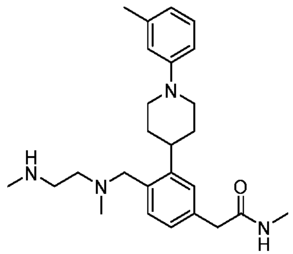 | 423.3 | P351 | 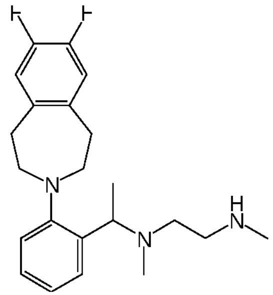 | 374.2 |
| P141 | 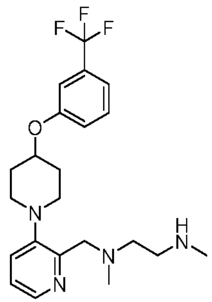 | 423.4 | P352 | 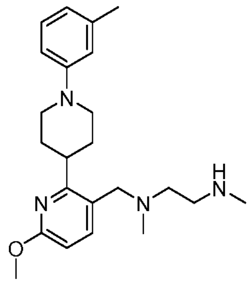 | 383.4 |
| P142 | 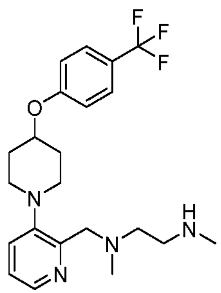 | 423.3 | P353 | 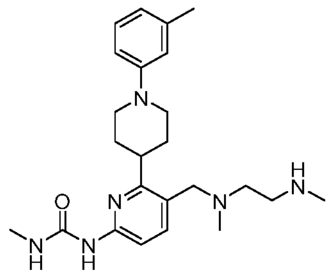 | 425.3 |

(continued)

| Patent_ ID | Structure | [M+H]+ (ESI) m/z | Patent_ ID | Structure | [M+H]+ (ESI) m/z |
|---|---|---|---|---|---|
| P143 | 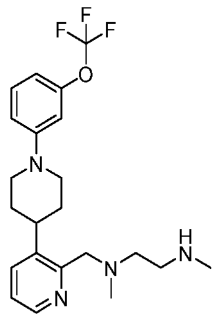 | 423.3 | P354 | 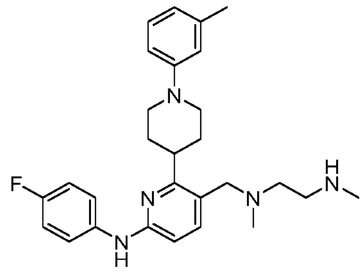 | 462.3 |
| P144 | 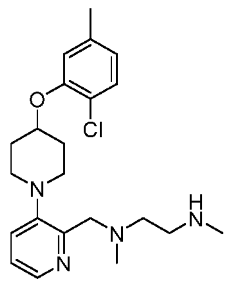 | 403.3 | P355 | 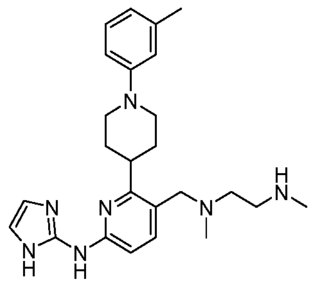 | 434.3 |
| P145 | 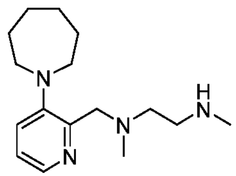 | 276.9 | P356 | 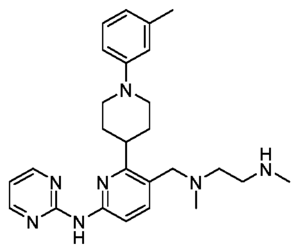 | 446.4 |

(continued)

| Patent_ ID | Structure | $[M+H]^+$ (ESI) m/z | Patent_ ID | Structure | $[M+H]^+$ (ESI) m/z |
|---|---|---|---|---|---|
| P146 | 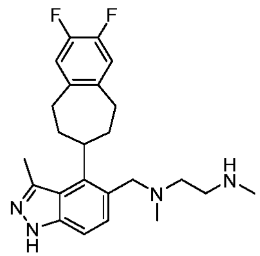 | 413.4 | P357 | 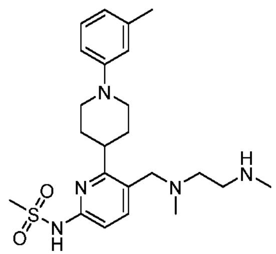 | 446.3 |
| P147 | 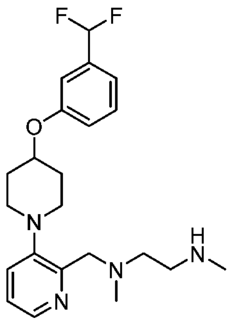 | 405.4 | P358 | 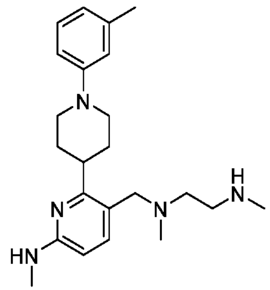 | 382.3 |
| P148 | 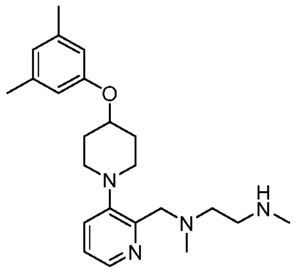 | 383.0 | P359 | 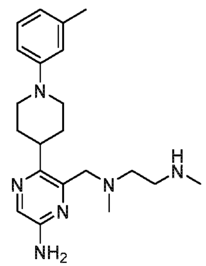 | 369.2 |

(continued)

| Patent_ ID | Structure | $[M+H]^+$ (ESI) m/z | Patent_ ID | Structure | $[M+H]^+$ (ESI) m/z |
|---|---|---|---|---|---|
| P149 | 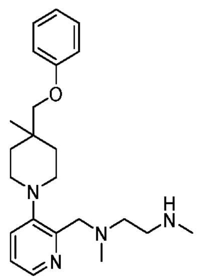 | 383.0 | P360 | 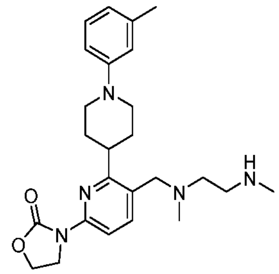 | 438.2 |
| P150 | 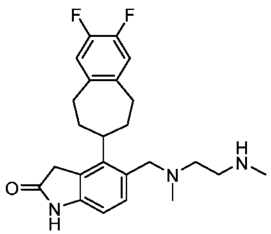 | | P361 | 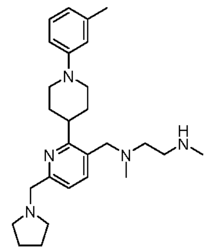 | 436.4 |
| P151 | 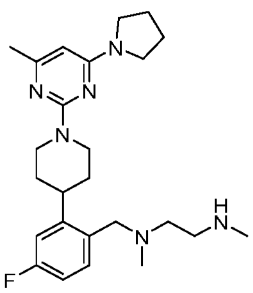 | 441.2 | P362 | 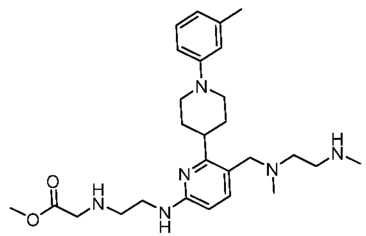 | 483.3 |

(continued)

| Patent_ ID | Structure | [M+H]+ (ESI) m/z | Patent_ ID | Structure | [M+H]+ (ESI) m/z |
|---|---|---|---|---|---|
| P152 | 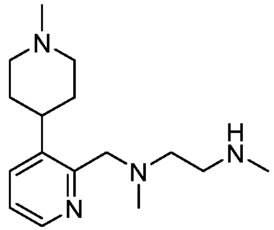 | 277.2 | P363 | 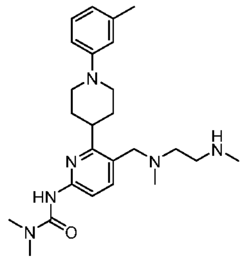 | 439.4 |
| P153 | 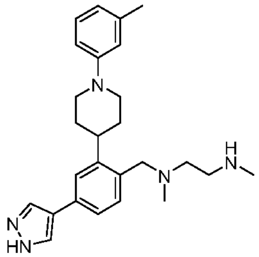 | 418.2 | P364 | 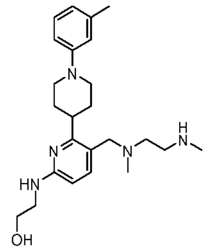 | 412.3 |
| P154 | 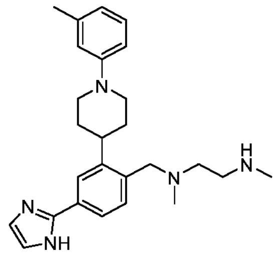 | 418.3 | P365 | 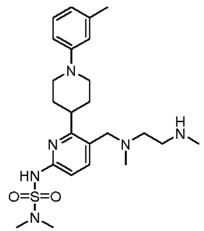 | 475.4 |

(continued)

| Patent_ ID | Structure | [M+H]$^+$ (ESI) m/z | Patent_ ID | Structure | [M+H]$^+$ (ESI) m/z |
|---|---|---|---|---|---|
| P155 | 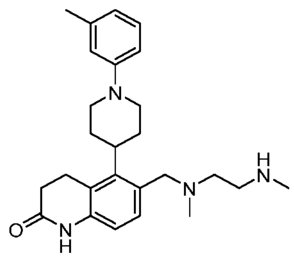 | 421.3 | P366 | 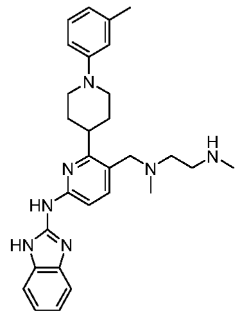 | 484.3 |
| P156 | 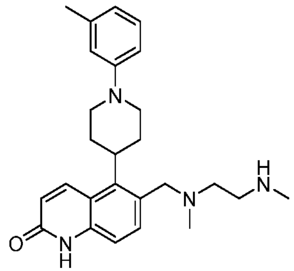 | | P367 | 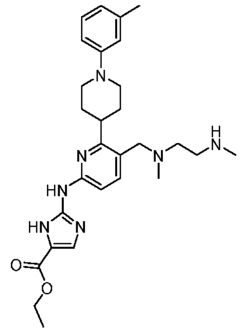 | 506.5 |
| P157 | 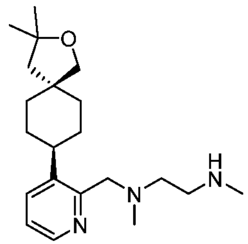 | 346.0 | P368 | 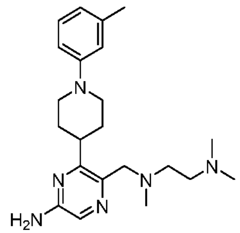 | 383.3 |

(continued)

| Patent_ ID | Structure | $[M+H]^+$ (ESI) m/z | Patent_ ID | Structure | $[M+H]^+$ (ESI) m/z |
|---|---|---|---|---|---|
| P158 | 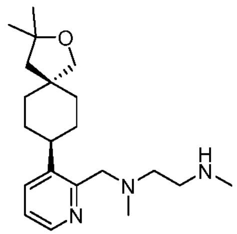 | 346.1 | P369 | 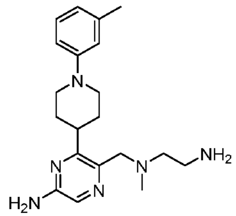 | 355.2 |
| P159 | 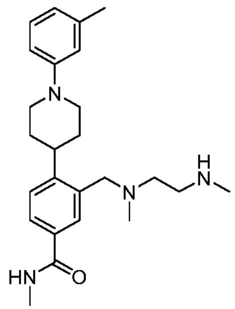 | 409.3 | P400 | 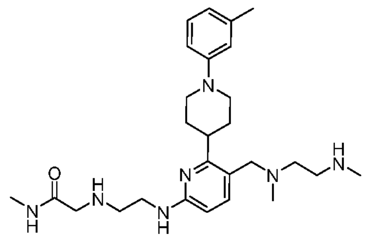 | 482.4 |
| P160 | 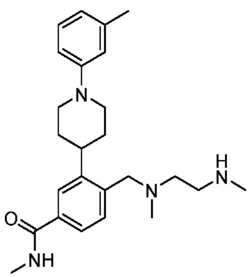 | 409.2 | P401 | 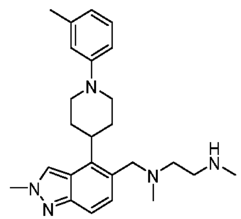 | 406.3 |
| P161 | 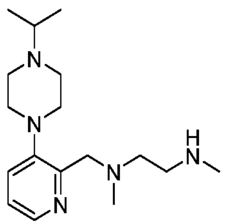 | 306.3 | P265 | 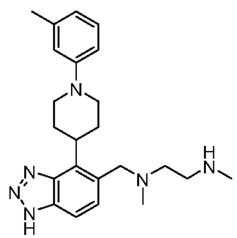 | 393.2 |

(continued)

| Patent_ ID | Structure | $[M+H]^+$ (ESI) m/z | Patent_ ID | Structure | $[M+H]^+$ (ESI) m/z |
|---|---|---|---|---|---|
| P162 | 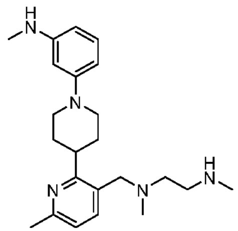 | 382.2 | P155 | 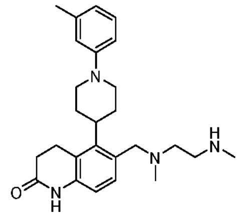 | 421.4 |
| P163 | 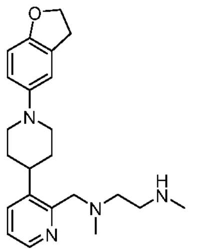 | 381.3 | P402 | 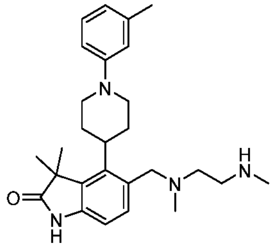 | 435.3 |
| P164 | 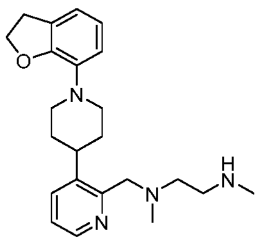 | 381.2 | P403 | 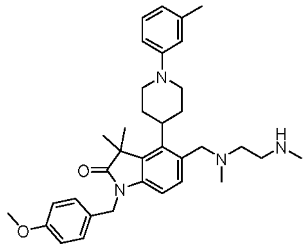 | 555.4 |
| P165 | 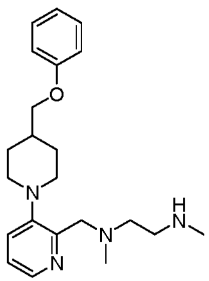 | 369.2 | P404 | 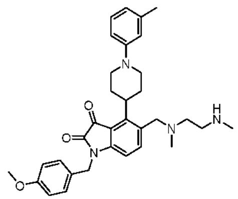 | 541.3 |

(continued)

| Patent_ ID | Structure | [M+H]+ (ESI) m/z | Patent_ ID | Structure | [M+H]+ (ESI) m/z |
|---|---|---|---|---|---|
| P166 | 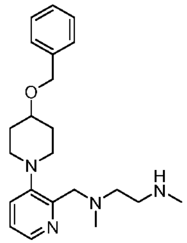 | 369.0 | P189 | 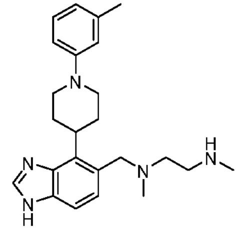 | 392.4 |
| P167 | 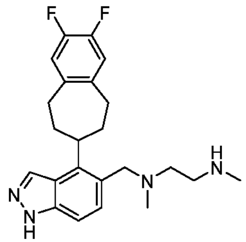 | 399.2 | P405 | 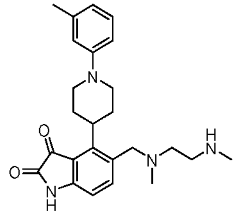 | 421.3 |
| P168 | 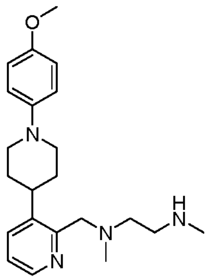 | 369.2 | P406 | 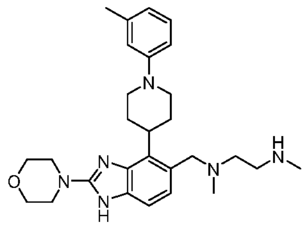 | 477.3 |
| P169 | 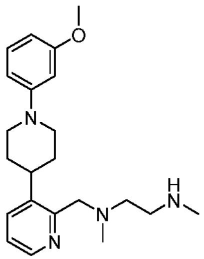 | 369.2 | P407 | 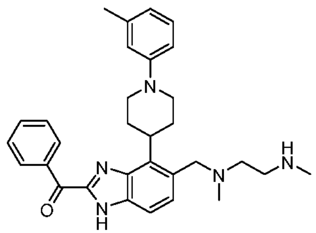 | 496.4 |

(continued)

| Patent_ ID | Structure | [M+H]+ (ESI) m/z | Patent_ ID | Structure | [M+H]+ (ESI) m/z |
|---|---|---|---|---|---|
| P170 | 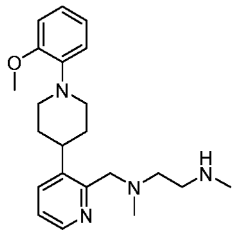 | 369.3 | P408 | 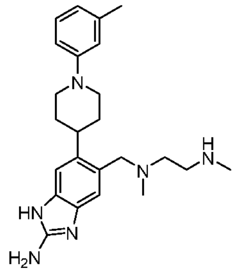 | 407.2 |
| P171 | 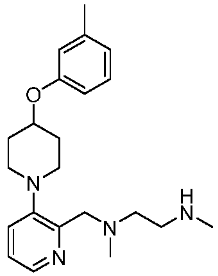 | 368.9 | P409 | 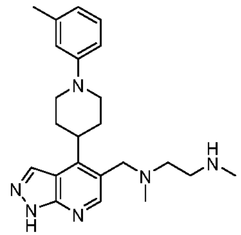 | 393.2 |
| P172 | 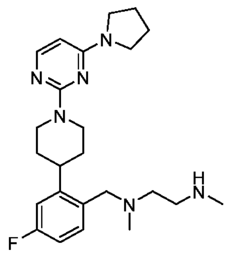 | 427.3 | P410 | 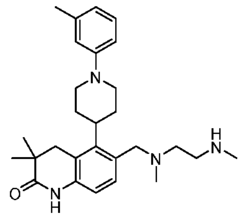 | 449.3 |

(continued)

| Patent_ ID | Structure | [M+H]+ (ESI) m/z | Patent_ ID | Structure | [M+H]+ (ESI) m/z |
|---|---|---|---|---|---|
| P173 | 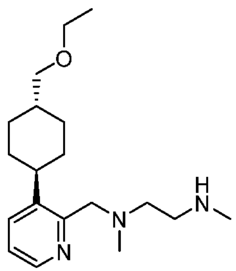 | 320.3 | P411 | 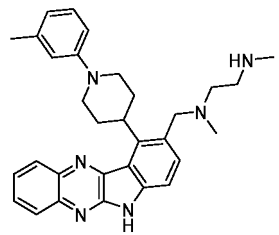 P411 | 494.3 |
| P174 | 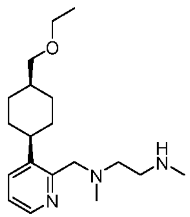 | 320.4 | P412 | 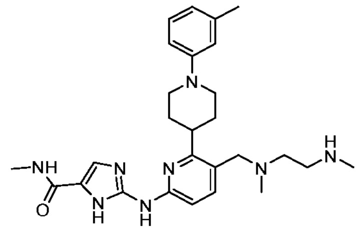 P412 | 491.3 |
| P176 | 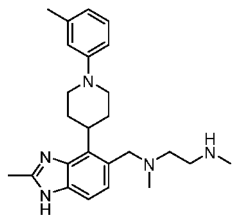 | 406.4 | P413 | 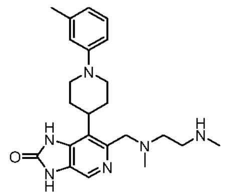 P413 | 409.3 |
| P177 | 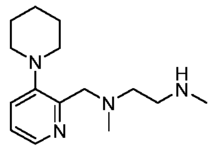 | 263.0 | P414 | 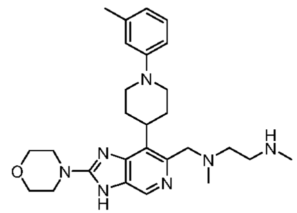 P414 | 478.3 |

(continued)

| Patent_ ID | Structure | [M+H]+ (ESI) m/z | Patent_ ID | Structure | [M+H]+ (ESI) m/z |
|---|---|---|---|---|---|
| P178 | 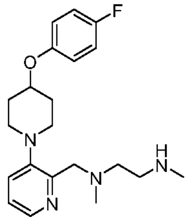 | 373.3 | P415 | 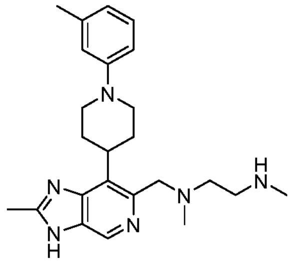 P415 | 407.3 |
| P179 | 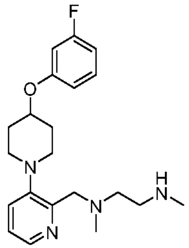 | 372.9 | P416 | 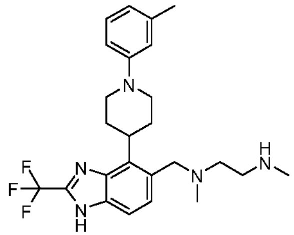 P416 | 460.3 |
| P180 | 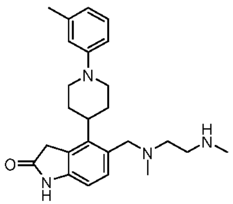 | 407.3 | P418 | 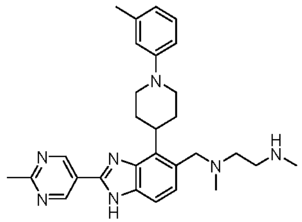 P418 | 484.4 |
| P181 | 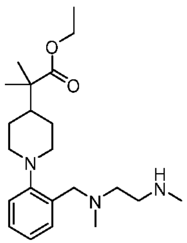 | 377.1 | P419 | 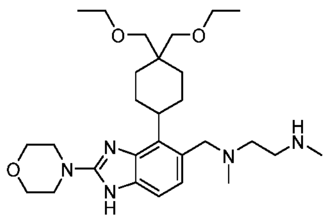 P419 | 502.4 |

(continued)

| Patent_ ID | Structure | [M+H]+ (ESI) m/z | Patent_ ID | Structure | [M+H]+ (ESI) m/z |
|---|---|---|---|---|---|
| P182 | 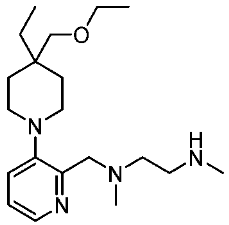 | 349.0 | P420 | 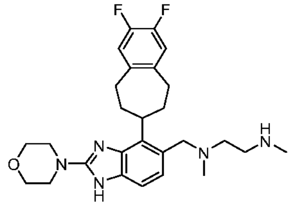 P420 | 484.4 |
| P183 | 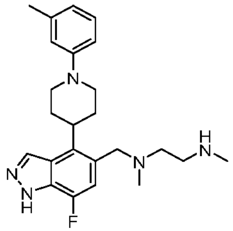 | 410.3 | P421 | 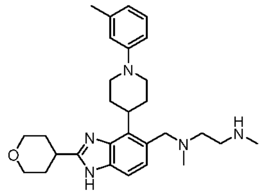 P421 | 476.4 |
| P184 | 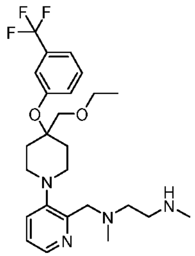 | 481.1 | P422 | 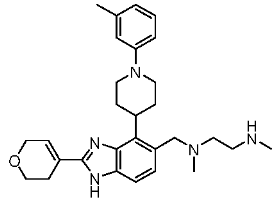 P422 | 474.4 |
| P185 | 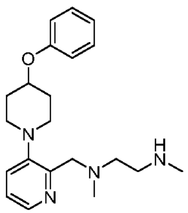 | 355.3 | P423 | 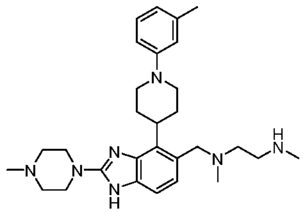 P423 | 490.4 |

(continued)

| Patent_ ID | Structure | [M+H]+ (ESI) m/z | Patent_ ID | Structure | [M+H]+ (ESI) m/z |
|---|---|---|---|---|---|
| P186 | 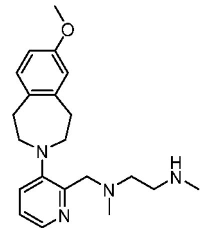 | 355.3 | P424 | 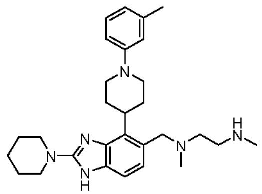 P424 | 475.4 |
| P187 | 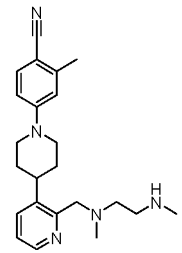 | 378.0 | P425 | 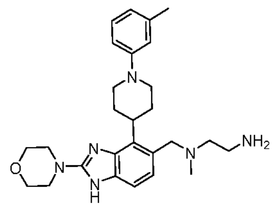 P425 | 463.3 |
| P188 | 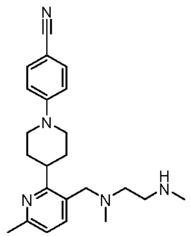 | 378.2 | P426 | 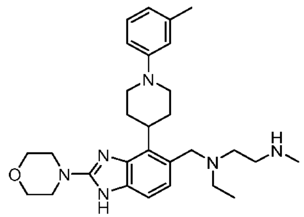 P426 | 491.4 |
| P189 | 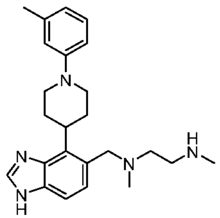 | 392.3 | P428 | 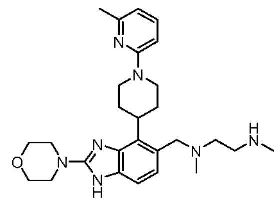 P428 | 478.4 |

(continued)

| Patent_ ID | Structure | [M+H]+ (ESI) m/z | Patent_ ID | Structure | [M+H]+ (ESI) m/z |
|---|---|---|---|---|---|
| P190 | 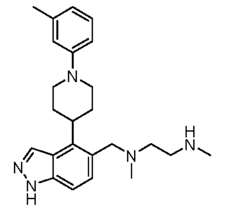 | 392.2 | P429 | 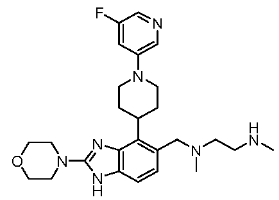 P429 | 482.3 |
| P191 | 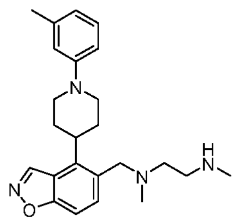 | | P438 | 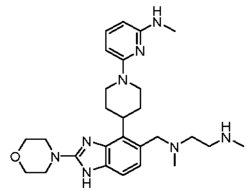 P438 | 493.4 |
| P192 | 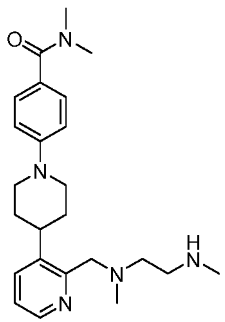 | 410.3 | P440 | 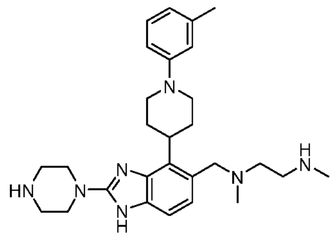 P440 | 476.4 |
| P193 | 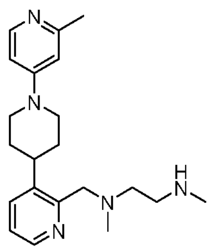 | 354.3 | P446 | 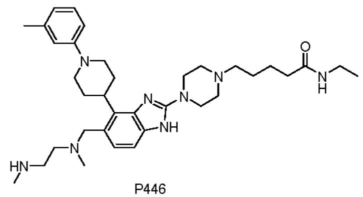 P446 | 603.4 |

(continued)

| Patent_ ID | Structure | [M+H]+ (ESI) m/z | Patent_ ID | Structure | [M+H]+ (ESI) m/z |
|---|---|---|---|---|---|
| P194 | 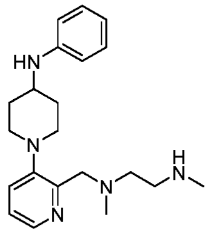 | 354.4 | P447 | 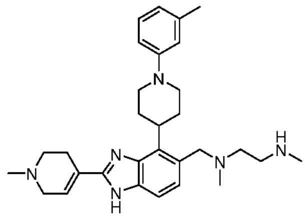 P447 | 487.3 |
| P195 | 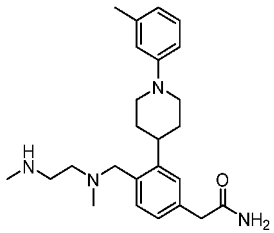 | 409.3 | P448 | 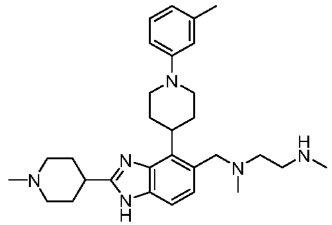 P448 | 489.4 |
| P442 | 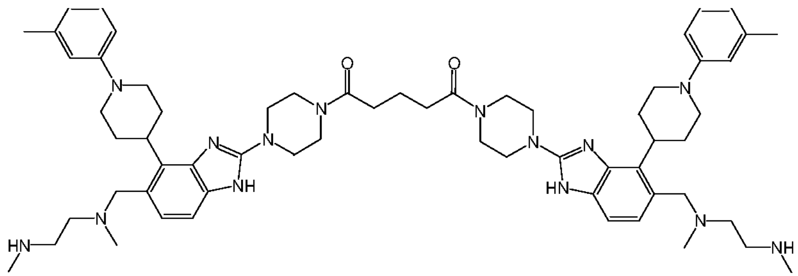 | | | | 1047.7 |
| P257 | 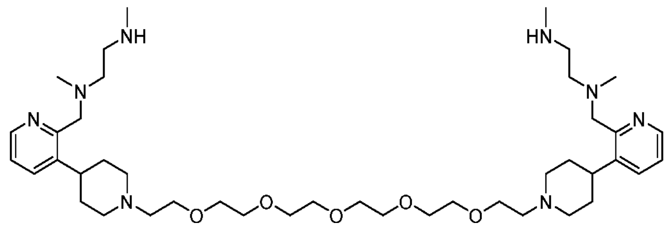 | | | | 771.8 |

**Biological Test Example**

**Test Example** 1: **PRMT1 Enzyme Activity Test Methods**

PRMT1 enzyme activity test were performed in a buffer prepared on the same day consisting of 10 mMTirs-HCl (pH = 8.0), 0.01% Tween-20 and 1 mM DTT. The compounds were prepared to the highest inhibitor concentration ultimately required in the 100X reaction with 100% DMSO and then transferred to one well of a 384-well Echo plate (Echo-eligible 384-well polypropylene microplates 2.0, transparent, flat-bottomed) and sequentially diluted in the next well with a 3-fold dilution of 100% DMSO, and so on, with a total of 10 concentrations based on Precision. 100% DMSO was added to two empty wells to serve as control wellwithout compound and control well without enzyme in the same 384-well Echo plate. 250 nL of compound was transferred from each well of the 384-well Echo plate to a 384-well assay plate (384-well polypropylene storage microplate) via the Echo 550 liquid processor. For wells with compoundsand control well without compound, a mixture (15 uL) containing PRMT1 enzyme was added to the 384-well assay plate. For control wells without enzyme, the mixture containing the assay buffer (15uL) was added instead. The compounds were allowed to incubate with PRMT1 for 15 minutes at room temperature, and then a mixture containing $^{3}$H-SAM and peptide (10uL) was added to start the reaction (final volume = 25uL). The final concentrations of the components were as follows: 0.5 nM for PRMT1, 0.25 uM for $^{3}$H-SAM, 0.1 uM for the peptide, and 1% DMSO. After 120 min of incubation, the assay was terminated by the addition of non-radiolabeled SAM (5 uL) to a final concentration of 125 uM, which diluted 3H-SAM to a level at which its incorporation into the peptide substrate was no longer detected. 25 $\mu$L of thereaction solution from the 384-well assay plate was then transferred to a 384-well detectionplate (Streptavidin FlashPlate HTS PLUS, high volume, 384 wells). The biotinylated peptide was allowed to bind to the surface of the antibiotic protein streptavidin for at least 60 minutes at room temperature. The Flashplate was then washed three times with 0.1% Tween-20 in a BioTek plate washer. Plates were read on a microplate counter (MicroBeta2) to measure the amount of $^{3}$H-labeled peptide bound to the surface of the detectionplate, measured in counts per minute (cpm).

Curve Fitting:

The raw data were copied from the Reader and the inhibition values were calculated in Excel by Equation (1), Equation (1): Inh %=( CPMmax-CPMcmpd)/ (CPMmax-CPMmin)*100, wherein the Max signal was obtained from the enzyme-substrate interaction and the Min signal was obtained from the substrate. IC50 values were obtained by fitting the data through equation (2) using XLFit add-in for Excel version 45.4.0.8. Equation (2): Y=Bottom + (Top-Bottom) / (1+ ((IC50/X)*HillSlope)), wherein, Y represents inhibition percentage and X represents compound concentration.

**Test Example 2:**

**Toledo Cell Proliferation Inhibition Assay Method**

A. Purpose: to determine the effects of compounds on Toledo cells through a six-day cell proliferation assay.

B. Reagents and materials:

1. Cell culture medium: RPMI 1640 + 10% FBS
2. 384-well cell culture plate (Corning, item #3764)
3. 0.4% Taipan Blue Staining Solution (Thermo Fisher, item #T10282)
4. cell counting plate (Thermo Fisher, item #C10228)
5. 50ml reagent reservoir (Corning, item #4870)
6. 50 ml conical sterile polypropylene centrifuge tube (Thermo Fisher, item #339653)
7. Automatic cell counter (Thermo Fisher, item # AMQAX1000)
8. CellTiter-Glo Luminescent Cell Viability Detection Reagent (Promega, item #G7572)
9. SpectraMax i3x Multi-Mode Enzyme Labeling Instrument (Megaman Molecular Instruments, item #5024062)

C. Experimental method

1. Resuspending the cells with a pipette.
2. 0.4% Taipan blue staining solution and Toledo cells were mixed in equal proportion, and the cells were counted using an automatic cell counter.
3. Toledo cells were diluted to a final concentration of 2000 cells per well and transferred to a 50 ml reagent

reservoir.

4. Diluted Toledo cells were added to a 384-well cell culture plate, 40ul per well.

5. The cells were shortly centrifuged and settled.

6. The cells were incubated in a 37°C, 5% $CO_2$ cell culture incubator.

7. The substrate in the CellTiter-Glo Luminescent Cell Viability Detection Reagentwas mixed with the solution.

8. After six days, the 384-well cell culture plate was takenfrom the incubator and 20 ul of configured CellTiter-Glo reagent was added to each well.

9. 384-well cell culture platewasmixed in an orbital shaker for 30 minutes.

10. Signal of luminescence was detected with a SpectraMax i3x multi-mode enzyme labeling instrument.

**MV-4-11 cell proliferation inhibition assay method**

Similar to the Toledo cell proliferation inhibition assay method, the difference was that MV-4-11 cells were used and the cell culture medium was IMDM + 10% FBS; in step 3 of the assay method250 cells per well was used instead; The results were shown in the following table.

Among them, the results of PRMT1 enzyme activity experiments were as follows:

A represents $IC_{50}$ < 50 nM;
B represents 50 nM<= $IC_{50}$ < 500 nM; and
C represents $IC_{50}$>= 500 nM

Results of Toledo and MV-4-11 cell proliferation inhibition assay:

A represents $IC_{50}$ < 1 μM;
B represents 1 μM<= $IC_{50}$ < 5 μM;
C represents $IC_{50}$>= 5 μM

The results of PRMT1 enzyme activity test, and Toledo and MV-4-11 cell proliferation inhibition assay were shown in the table below:

| Patent _ID | PRMT1 $IC_{50}$ | Toledo cell proliferation inhibition $IC_{50}$ | MV-4-11 cell proliferation inhibition ICso | Patent_ ID | PRMT1 $IC_{50}$ | Toledo cell proliferation inhibition $IC_{50}$ | MV-4-11 cell proliferation inhibition ICso |
|---|---|---|---|---|---|---|---|
| P001 | A | A | | P187 | A | A | |
| P002 | A | | | P188 | A | B | |
| P003 | A | A | | P189 | A | A | |
| P005 | A | A | | P190 | A | A | |
| P006 | A | B | | P192 | A | c | |
| P007 | A | A | | P193 | A | B | |
| P008 | A | A | | P194 | A | B | |
| P009 | A | A | | P195 | A | A | |
| P010 | A | B | | P196 | A | A | |
| P011 | A | A | | P197 | C | C | |
| P012 | A | A | | P198 | A | A | |
| P013 | A | B | | P199 | A | A | |
| P014 | A | A | | P200 | A | B | |
| P015 | A | A | | P201 | A | A | |
| P016 | A | A | | P202 | A | A | |
| P017 | A | B | | P203 | B | C | |

(continued)

| Patent _ID | PRMT1 $IC_{50}$ | Toledo cell proliferation inhibition $IC_{50}$ | MV-4-11 cell proliferation inhibition ICso | Patent_ ID | PRMT1 $IC_{50}$ | Toledo cell proliferation inhibition $IC_{50}$ | MV-4-11 cell proliferation inhibition ICso |
|---|---|---|---|---|---|---|---|
| P020 | A | A | | P204 | A | | |
| P021 | A | A | | P205 | B | B | |
| P022 | A | A | | P206 | B | B | |
| P023 | A | A | | P207 | A | A | |
| P024 | A | A | | P208 | A | A | |
| P025 | A | A | | P210 | B | | A |
| P026 | C | | | P211 | B | | |
| P027 | A | B | | P212 | B | C | |
| P028 | A | C | | P214 | A | B | |
| P029 | A | | | P215 | A | B | |
| P030 | A | C | | P216 | A | B | |
| P031 | A | B | | P217 | B | B | |
| P032 | C | | | P218 | A | B | |
| P033 | A | B | | P219 | A | B | |
| P034 | A | A | | P220 | B | | |
| P035 | B | C | | P221 | A | A | |
| P036 | A | A | | P222 | A | c | |
| P037 | A | A | | P224 | A | B | |
| P038 | A | A | | P225 | A | A | |
| P039 | A | A | | P226 | A | A | |
| P040 | A | A | | P227 | A | A | |
| P041 | A | | | P228 | A | A | |
| P042 | A | A | | P230 | A | A | |
| P044 | A | A | | P232 | B | | |
| P046 | A | B | | P233 | A | C | |
| P047 | A | B | | P234 | B | B | |
| P048 | A | A | | P235 | A | | |
| P049 | A | A | | P236 | A | A | |
| P050 | A | A | | P238 | A | A | |
| P051 | A | A | | P239 | A | B | |
| P052 | A | A | | P240 | A | A | |
| P053 | A | A | | P242 | A | A | |
| P054 | A | A | | P243 | A | A | |
| P055 | A | B | | P244 | A | | |
| P056 | A | A | | P245 | A | | |
| P057 | A | A | | P246 | C | | |

(continued)

| Patent _ID | PRMT1 $IC_{50}$ | Toledo cell proliferation inhibition $IC_{50}$ | MV-4-11 cell proliferation inhibition ICso | Patent_ ID | PRMT1 $IC_{50}$ | Toledo cell proliferation inhibition $IC_{50}$ | MV-4-11 cell proliferation inhibition ICso |
|---|---|---|---|---|---|---|---|
| P058 | A | A | | P247 | B | | |
| P059 | A | B | | P248 | B | B | |
| P060 | A | A | | P250 | A | A | |
| P061 | A | A | | P251 | A | A | |
| P062 | A | | | P252 | A | A | |
| P063 | A | A | | P254 | B | c | |
| P064 | A | A | | P255 | A | B | |
| P065 | A | A | | P256 | A | | |
| P066 | A | A | | P257 | A | | |
| P067 | A | A | | P258 | A | A | |
| P068 | A | B | | P259 | A | B | |
| P069 | A | A | | P260 | A | B | |
| P070 | A | A | | P261 | | | A |
| P071 | A | B | | P262 | A | B | |
| P072 | A | A | | P263 | C | C | |
| P073 | A | A | | P265 | A | | |
| P074 | A | A | | P266 | A | A | |
| P075 | A | A | | P267 | A | B | |
| P076 | A | B | | P268 | A | B | |
| P077 | A | B | | P269 | A | B | |
| P078 | A | A | | P270 | A | B | |
| P079 | A | A | | P271 | A | B | |
| P080 | B | A | | P272 | B | B | |
| P081 | A | A | | P273 | A | B | |
| P082 | A | B | | P274 | A | | |
| P083 | A | A | | P275 | C | C | |
| P084 | A | A | | P276 | C | C | |
| P085 | A | A | | P277 | A | A | |
| P086 | A | B | | P278 | A | B | |
| P087 | A | B | | P279 | A | B | |
| P088 | A | A | | P280 | A | C | |
| P089 | A | A | | P281 | B | C | |
| P090 | A | A | | P282 | | | |
| P091 | C | | | P283 | | | |
| P092 | A | A | | P284 | A | B | |
| P093 | A | A | | P285 | C | C | |

(continued)

| Patent _ID | PRMT1 $IC_{50}$ | Toledo cell proliferation inhibition $IC_{50}$ | MV-4-11 cell proliferation inhibition ICso | Patent_ ID | PRMT1 $IC_{50}$ | Toledo cell proliferation inhibition $IC_{50}$ | MV-4-11 cell proliferation inhibition ICso |
|---|---|---|---|---|---|---|---|
| P094 | A | A | | P286 | A | B | |
| P095 | A | A | | P287 | A | A | |
| P096 | A | B | | P288 | A | B | |
| P097 | A | B | | P289 | A | | |
| P098 | A | A | | P290 | A | B | |
| P099 | A | B | | P291 | A | C | |
| P100 | C | C | | P292 | A | A | |
| P101 | B | B | | P293 | B | A | |
| P102 | A | A | | P294 | A | | |
| P103 | A | A | | P295 | A | | |
| P104 | A | A | | P297 | A | C | |
| P105 | A | A | | P298 | A | B | |
| P106 | A | A | | P299 | A | | |
| P107 | A | A | | P300 | B | C | |
| P108 | A | A | | P301 | A | B | |
| P109 | A | A | | P302 | A | B | |
| P110 | A | A | | P303 | A | B | |
| P111 | A | A | | P304 | A | B | |
| P112 | A | A | | P305 | A | A | |
| P113 | A | A | | P306 | B | B | |
| P114 | A | A | | P307 | A | | |
| P115 | A | B | | P313 | A | B | |
| P116 | A | B | | P314 | A | C | |
| P117 | A | A | | P315 | C | C | |
| P118 | A | A | | P318 | A | C | |
| P119 | C | B | | P319 | A | C | |
| P120 | A | B | | P320 | A | C | |
| P122 | B | A | | P321 | A | B | |
| P123 | A | B | | P322 | A | | |
| P124 | C | B | | P323 | A | A | |
| P125 | A | A | | P325 | A | A | |
| P126 | A | B | | P326 | A | B | |
| P127 | A | B | | P327 | A | A | |
| P128 | C | C | | P329 | B | C | |
| P129 | A | B | | P350 | C | | |
| P130 | A | A | | P351 | B | | |

(continued)

| Patent _ID | PRMT1 $IC_{50}$ | Toledo cell proliferation inhibition $IC_{50}$ | MV-4-11 cell proliferation inhibition ICso | Patent_ ID | PRMT1 $IC_{50}$ | Toledo cell proliferation inhibition $IC_{50}$ | MV-4-11 cell proliferation inhibition ICso |
|---|---|---|---|---|---|---|---|
| P131 | A | A | | P352 | B | | |
| P132 | A | A | | P353 | A | | |
| P133 | A | A | | P354 | B | | |
| P134 | A | B | | P355 | A | | |
| P135 | A | A | | P356 | B | | |
| P136 | A | A | | P357 | B | | |
| P137 | A | A | | P358 | A | | |
| P138 | A | | | P359 | B | | |
| P139 | B | C | | P360 | B | | |
| P140 | A | A | | P361 | A | | |
| P141 | A | A | | P362 | B | | |
| P142 | A | A | | P363 | B | | |
| P143 | A | A | | P364 | A | | |
| P144 | A | A | | P365 | B | | |
| P145 | A | B | | P366 | A | | |
| P146 | A | A | | P367 | A | | |
| P147 | A | A | | P368 | B | | |
| P148 | A | A | | P369 | A | | |
| P149 | A | A | | P400 | | | A |
| P151 | A | B | | P401 | A | | A |
| P152 | A | | | P402 | B | | B |
| P153 | A | A | | P403 | C | | B |
| P154 | A | | | P404 | B | | A |
| P155 | A | A | | P405 | A | | A |
| P157 | A | A | | P406 | A | | A |
| P158 | A | A | | P407 | B | | A |
| P159 | C | C | | P408 | | | C |
| P160 | A | A | | P409 | B | | A |
| P161 | A | A | | P410 | | | A |
| P162 | B | A | | P411 | | | |
| P163 | A | A | | P412 | | | |
| P164 | A | B | | P413 | | | A |
| P165 | A | A | | P414 | | | A |
| P166 | A | A | | P415 | | | A |
| P167 | A | A | | P416 | | | A |
| P168 | A | B | | P418 | | | A |

(continued)

| Patent _ID | PRMT1 $IC_{50}$ | Toledo cell proliferation inhibition $IC_{50}$ | MV-4-11 cell proliferation inhibition ICso | Patent_ ID | PRMT1 $IC_{50}$ | Toledo cell proliferation inhibition $IC_{50}$ | MV-4-11 cell proliferation inhibition ICso |
|---|---|---|---|---|---|---|---|
| P169 | A | A | | P419 | | | A |
| P170 | A | A | | P420 | | | A |
| P171 | A | A | | P421 | | | A |
| P172 | A | A | | P422 | | | A |
| P173 | A | A | | P423 | | | A |
| P174 | A | B | | P424 | | | A |
| P176 | A | A | | P425 | | | A |
| P177 | A | B | | P426 | | | A |
| P178 | A | A | | P428 | | | A |
| P179 | A | A | | P429 | | | A |
| P180 | A | A | | P438 | | | |
| P181 | A | B | | P440 | | | A |
| P182 | A | A | | P442 | | | A |
| P183 | A | A | | P446 | | | A |
| P184 | B | A | | P447 | | | A |
| P185 | A | A | | P448 | | | A |
| P186 | A | A | | | | | |

**Test example 3 PRMT panel selectivity test**

PRMT1 was tested as described in Test Example 1, and PRMT5 and PRMT7 tests and curve fitting were performed with optimized parameters with reference to PRMT1 test and data processing methods. PRMT3 was tested as follows:

PRMT3 test method:

This experiment was assayed in a buffer prepared on the same day consisting of 10 mMTirs-HCl (pH = 8.0), 0.01% Tween-20 and 1 mM DTT. The compounds were prepared to the highest inhibitor concentration ultimately required in the 100X reaction with 100% DMSO and then transferred to one well of a 384-well Echo plate (Echo-eligible 384-well polypropylene microplates 2.0, transparent, flat-bottomed) and sequentially diluted in the next well with a 3-fold dilution of 100% DMSO, and so on, with a total of 10 concentrations based on Precision. 100% DMSO was added to two empty wells as control well without compound and control well without enzyme in the same 384-well Echo plate. 100 nL of compound was transferred from each well of the 384-well Echo plate to a 384-well assay plate (OptiPlate (384-well, white)) via the Echo 550 liquid processor. For wells with compounds and control well without compound, a mixture containing PRMT1 enzyme (5 uL) was added to the 384-well assay plate. For control well without enzyme, amixture containing test buffer (5uL) was added instead. The compounds were allowed to incubate with PRMT3 for 15 minutes at room temperature, and then a mixture containing cold SAM and peptide (5uL) was added to start the reaction (final volume = 10uL). The final concentrations of the components were as follows: 0.1 nM for PRMT3, 15 uM for SAM, 0.05 uM for peptide, and 1% DMSO. After 60 min of incubation at room temperature, the reaction was terminated by the addition of donor and acceptor magnetic beads (15 uL) with a final concentration of 10ug/mL diluted with AlphaLISA Epigenetics Buffer. The 384-well assay plate was incubated at room temperature for 60 min and the relative fluorescence units (RFU) were nextly determined using a multi-mode plate reader in Alpha mode (Ex680/Em615).

Curve fitting: the raw data were copied from Reader and the inhibition values were calculated in Excel by equation (1). Equation (1): Inh %=(RFUmax-RFUcmpd)/ (RFUmax-RFUmin)*100, wherein, the Max signal was obtained from the enzyme-substrate interaction and the Min signal was obtained from the substrate. IC50 values were obtained

by fitting the data through equation (2) using XLFit add-in for Excel version 45.4.0.8. Equation (2): Y=Bottom + (Top-Bottom) / (1+ ((IC50/X)*HillSlope)), wherein, Y represents inhibition percentage and X represents compound concentration.

PRMT4, PRMT6, and PRMT8 tests and curve fitting were performed with reference to PRMT3 tests and data processing methods using optimized parameters.

The following table showedthe results of the PRMT panel selectivity tests, wherein, A represents $IC_{50}$ < 50 nM; B represents 50 nM<= $IC_{50}$ < 500 nM; and
C represents $IC_{50}$>= 10 μM

| Compound | PRMT3 | PRMT4 | PRMT5 | PRMT6 | PRMT7 | PRMT8 |
|---|---|---|---|---|---|---|
| P207 | B | A | C | A | C | A |
| P023 | A | A | C | A | C | A |
| P118 | B | B | C | A | C | A |

**Test Example 4 RKO in cell western assay method**

RKO was seeded at a concentration of 20,000 cells/ml into a polylysine-coated 96-well plate (Corning#3599), with 2,000 cells per well and 100 μL of culture mediumper well. 500 nL of compound was added to the 96-well plate. 3) The 96-well plate was incubated in a 37°C, 5% $CO_2$ incubator for 72 hours. After three days, the 96-well plate was takenfrom the incubator, 100 μL of 8% paraformaldehyde solution was added to each well, and the plate was placed at room temperature for 15 min. After 15 min, the liquid in the plate was shaken out and the plate was washed three times with phosphate buffer(1*PBS). The liquid in the plate was shaken out and200 μL of membrane-breaking buffer (0.1% (v/v) Tween-20 in PBS) was added and the plate was incubated on a shaker for 30 min. After half an hour, the liquid in the plate was shaken out, and blocking buffer was added and the plate was incubated for two hours at room temperature with shaking. After that, the liquid in the plate was shaken off, and the blocking solution containing primary antibody (MMA primary antibody, CST#87115, 1:1000 v/v) was added to the 96-well plate and the plate was incubated at 4°C overnight with shaking. The next day, the plate was washed three times with washing solution (0.05% (v/v) Tween-20 in PBS), and a blocking solution containing secondary antibody (goat anti-rabbit IgG (H+L) Invitrogen#31460, 1:10,000 v/v) was added and the palte was incubated for two hours at room temperature. Afterwards, the 96-well plate was washed four times with washing solution. The TMB mixture was added to the 96-well plate and then the plate was incubated for 15 minutes at room temperature with shaking. After that, the reaction terminating solution was added, and the value was read at OD450 nm usinganmicroplate reader. The plate was washed three times with washing solution, and 50 μL of janusgreen staining solution was added, and the plate was incubated for 15 minutes at room temperature with shaking. The reaction was washed ten times with water until no color residue was left. 200 μL of 0.5 M hydrochloric acid solution was added to each well and the plate was incubated for 10 min at room temperature. The value was read at OD595nm using Flexstation.

Calculations:

Firstly, the ratio OD450/OD595 was calculated for each well

Each plate had six DMSO negative control groups (ZPE) and six 10 μM positive control groups (HPE), and the average of the control group ratios was used to determine the percentage of activation for each test well. The compounds to be tested were triple diluted with DMSO for a total of 8 concentration points with a starting concentration of 5 μM. The equation for calculating the activation pencentageof each test well was:

Activation percentage= 100 - [(HPE ratio –ratio of a certain well)/(HPE ratio - ZPE ratio)]*100

**Table 4 Results of RKO ICW assays**

A represents $EC_{30}$<250 nM
B represents 250 nM<= $EC_{30}$<2500 nM
C represents $EC_{30}$>2500 nM

| Patent_ID | RKO ICW_EC30 | Patent_ID | RKO ICW_EC30 |
|---|---|---|---|
| P001 | B | P118 | A |
| P003 | A | P122 | C |
| P004 | B | P130 | B |
| P005 | B | P131 | B |
| P007 | A | P136 | B |
| P008 | B | P137 | B |
| P009 | B | P141 | B |
| P014 | B | P142 | B |
| P015 | B | P143 | B |
| P021 | A | P147 | B |
| P022 | A | P148 | B |
| P023 | A | P149 | B |
| P025 | A | P153 | A |
| P039 | B | P157 | B |
| P044 | B | P158 | A |
| P049 | A | P165 | B |
| P050 | B | P167 | A |
| P051 | B | P170 | B |
| P052 | B | P171 | B |
| P053 | B | P179 | B |
| P054 | A | P184 | C |
| P056 | B | P185 | B |
| P057 | B | P186 | B |
| P069 | B | P187 | A |
| P074 | B | P198 | A |
| P075 | B | P202 | B |
| P078 | A | P206 | B |
| P079 | A | P207 | A |
| P080 | B | P208 | B |
| P081 | A | P225 | A |
| P083 | A | P226 | A |
| P085 | A | P236 | B |
| P088 | B | P240 | B |
| P089 | A | P243 | B |
| P094 | A | P250 | B |

(continued)

| A represents $EC_{30}$<250 nM<br>B represents 250 nM<= $EC_{30}$<2500 nM<br>C represents $EC_{30}$>2500 nM | | | |
|---|---|---|---|
| Patent_ID | RKO ICW_EC30 | Patent_ID | RKO ICW_EC30 |
| P095 | A | P251 | B |
| P106 | B | P252 | B |
| P107 | B | P258 | A |
| P112 | B | P323 | C |
| P114 | A | P325 | B |

All documents referred to in the present invention are incorporated by reference herein as if each document is individually incorporated by reference. Further, it should be understood that upon reading the above teaching of the present invention, various alterationsor modifications may be made to the present invention by those skilled in the art, and those equivalents also fall within the scope defined by the appended claims of the present application.

## Claims

**1.** A compound shown in formula (I), or a pharmaceutically acceptable salt thereof, or a deuterated compound thereof:

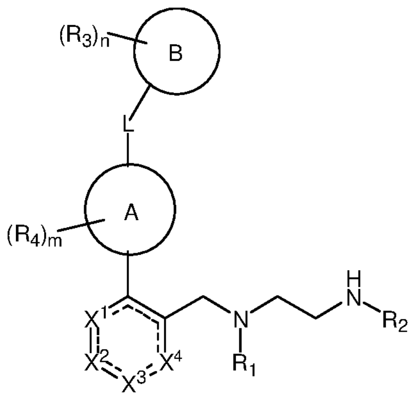

I

$X^1$, $X^2$, $X^3$ and $X^4$ are each independently selected from the group consisting of: CR, NR and N; the dash line is a chemical bond or absent;
ring A is selected from the group consisting of substituted or unsubstituted saturated or partially unsaturated (non-aromatic) 4-8 membered carbocycle, and substituted or unsubstituted saturated or partially unsaturated (non-aromatic) 4-8 membered heterocycle;
ring B is selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_6$-$C_{10}$aryl, substituted or unsubstituted 5-12 membered heteroaromatic ring, substituted or unsubstituted 3-12 membered heterocycle containing 1-3 heteroatoms selected from oxygen, sulfur and nitrogen, substituted or unsubstituted -$C_1$-$C_6$alkyl-6-10 membered aryl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$ amide, substituted or unsubstituted $C_1$-$C_4$ alkyl-$S(O)_2$,and substituted or unsubstituted $C_1$-$C_4$alkyl-SO-;
m and n are each independently selected from the group consisting of 0, 1, 2, 3, 4, 5 and 6;
L is selected from the group consisting of chemical bonds, -O-, -$(CHR^6)_p$-, -$CHR^6$-O-, -$CHR^6$-C(O)-, carbonyl, S, -NH-, -NHC(O)-, -NHS$(O)_2$-, -NHC(O)NH-, -NHC(S)NH-, -COO-, -O-S$(O)_2$-, -COO-$CH_2$-, -C(O)$CH_2$-, and -S$(O)_2$-, or L is absent;

p is selected from the group consisting of 1, 2 and 3;
$R^1$ and $R^2$ are each independently selected from the group consisting of H, substituted or unsubstituted $C_1$-$C_6$ alkyl, and substituted or unsubstituted $C_3$-$C_6$cycloalkyl;
$R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_2$-$C_6$alkenyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-12 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$ amide, substituted or unsubstituted $C_1$-$C_4$alkyl-S(O)$_2$-, and substituted or unsubstituted $C_1$-$C_4$alkyl-SO-;
R is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-12 membered heteroaryl, substituted or unsubstituted 4-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$ amide, substituted or unsubstituted $C_1$-$C_4$alkyl-S(O)$_2$-, and substituted or unsubstituted $C_1$-$C_4$alkyl-SO-;
or two R located on adjacent ring atoms together with the ring atoms to which they are attached form a substituted or unsubstituted 5-11 membered carbocycle or heterocycle, and the ring is partially unsaturated or saturated; preferably, the carbocycle or heterocycle is a 5-9 membered carbocycle or heterocycle, more preferably a 5-7 membered carbocycle or heterocycle (the carbocycle or heterocycle is saturated, partially unsaturated or aromatic);
$R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_2$-$C_6$alkenyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-12 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_{12}$carbocycle, substituted or unsubstituted $C_2$-$C_{10}$acyl, substituted or unsubstituted $C_2$-$C_{10}$ester group, substituted or unsubstituted $C_6$-$C_{10}$aryloxy, substituted or unsubstituted $C_1$-$C_6$ amide, substituted or unsubstituted $C_1$-$C_4$alkyl-S(O)$_2$-, and substituted or unsubstituted $C_1$-$C_4$alkyl-SO-;
or two $R^4$ on the same or adjacent ring atoms together with the ring atoms to which they attached form a substituted or unsubstituted 3-11 membered carbocycle or heterocycle, and the ring is a partially unsaturated ring, saturated ring or aromatic ring;
$R^6$ is a group selected from the group consisting of H, halogen, cyano, amino, nitro, hydroxyl, thiol, aldehyde group, carboxyl, acyl, sulfonyl, substituted or unsubstituted $C_1$-$C_6$alkyl, substituted or unsubstituted $C_1$-$C_6$alkoxy, and substituted or unsubstituted $C_1$-$C_6$alkylamino;
unless otherwise specified, in the above formulas, the "substituted" means that the hydrogen atom on the corresponding group is substituted by one or more substituents selected from the group consisting of: deuterium, tritium, halogen, hydroxyl, carboxyl, thiol, benzyl , oxo (=O), $C_1$-$C_{12}$alkoxycarbonyl, $C_1$-$C_6$aldehyde group, amino, $C_1$-$C_6$ amide, nitro, cyano, unsubstituted or halogenated $C_1$-$C_6$alkyl, $C_2$-$C_{10}$alkenyl, $C_1$-$C_6$alkoxy, $C_1$-$C_6$alkylamino, $C_1$-$C_6$alkyl-sulfonamide, $C_1$-$C_6$alkyl-ureido, $C_1$-$C_6$alkyl-S-, $C_6$-$C_{10}$aryl, five- or six-membered heteroaryl, five- or six-membered non-aromatic heterocyclyl, five- or six-membered non-aromatic heterocyclyl-($CH_2$)-, -O-($C_6$-$C_{10}$aryl), -O-(five or six membered heteroaryl), -NH-($C_6$-$C_{10}$aryl), -NH-(five-membered or six-membered heteroaryl), $C_1$-$C_{12}$alkylaminocarbonyl, unsubstituted or halogenated $C_2$-$C_{10}$acyl, sulfonyl (-$SO_2$-OH), sulfonamide (-SO2-NH2 ), phosphoryl (-$PO_3$-OH), unsubstituted or halogenated $C_1$-$C_4$alkyl-S(O)$_2$-, unsubstituted or halogenated $C_1$-$C_4$alkyl-SO-,

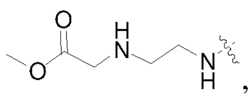 , 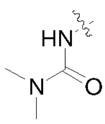 ,

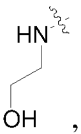 , 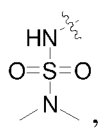 , 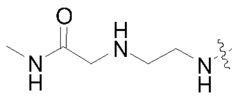 , 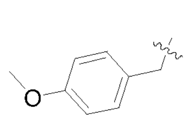 ,

and

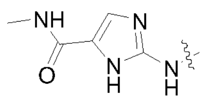 ;

in each formula, the heterocycle or heteroaromatic ring has 1-3 heteroatoms selected from the group consisting of N, S and O; each aryl, heteroaryl, and heterocyclyl can be independently substituted by 1-3 substituents selected from the group consisting of deuterium, tritium, halogen, hydroxyl, carboxyl, thiol, $C_1$-$C_6$alkyl, and $C_1$-$C_6$alkoxy.

**2.** The compound according to claim 1, or a pharmaceutically acceptable salt thereof, or a deuterated compound thereof, wherein $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-10 membered heteroaryl, substituted or unsubstituted 5-7 membered heterocycle containing 1-3 heteroatoms selected from oxygen, sulfur and nitrogen, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_6$-$C_{10}$aryloxy, substituted or unsubstituted $C_1$-$C_6$ amide, substituted or unsubstituted -$C_1$-$C_6$alkyl-phenyl, substituted or unsubstituted $C_1$-$C_4$alkyl-$S(O)_2$-, and substituted or unsubstituted $C_1$-$C_4$alkyl-SO-; and/or
R is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-10 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_6$-$C_{10}$aryloxy, substituted or unsubstituted $C_1$-$C_6$ amide, substituted or unsubstituted -$C_1$-$C_6$alkyl-phenyl, substituted or unsubstituted $C_1$-$C_4$alkyl-$S(O)_2$-, and substituted or unsubstituted $C_1$-$C_4$alkyl-SO-; or two R located on adjacent ring atoms together with the carbon atoms to which they attached form a substituted or unsubstituted 5-9 membered carbocycle or heterocycle, and the ring is partially unsaturated, saturated or aromatic; and/or.

**3.** The compound according to claim 1, or a pharmaceutically acceptable salt thereof, or a deuterated compound thereof, wherein, ring B is selected from the group consisting of: H, or substituted or unsubstituted phenyl, substituted or unsubstituted 5-10 membered heteroaromatic ring, substituted or unsubstituted 3-12 membered heterocycle containing 1-3 heteroatoms selected from oxygen, sulfur and nitrogen, and substituted or unsubstituted $C_3$ -$C_{12}$carbocycle.

**4.** The compound according to claim 1, or a pharmaceutically acceptable salt thereof, or a deuterated compound thereof, wherein $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-10 membered heteroaryl, substituted or unsubstituted 5-7 membered heterocycle containing 1-3 heteroatoms selected from oxygen, sulfur and nitrogen, substituted or unsubstituted $C_2$-$C_6$acyl, substituted or unsubstituted $C_2$-$C_6$ester group, substituted or unsubstituted $C_1$-$C_6$ amide, substituted or unsubstituted $C_1$-$C_4$alkyl-$S(O)_2$-, and substituted or unsubstituted $C_1$-$C_4$alkyl-SO-; and/or

R is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_1$-$C_6$ amide, substituted or unsubstituted $C_1$-$C_4$alkyl-

S(O)$_2$-, and substituted or unsubstituted $C_1$-$C_4$alkyl-SO-; or two R located on adjacent ring atoms together with the ring atoms to which they are attached form a substituted or unsubstituted 5-7 membered carbocycle or heterocycle; and/or

$R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_3$-$C_8$carbocycle, substituted or unsubstituted $C_2$-$C_6$acyl, substituted or unsubstituted $C_2$-$C_{10}$ester group, substituted or unsubstituted $C_1$-$C_6$ amide, substituted or unsubstituted $C_1$-$C_4$alkyl-S(O)$_2$-, and substituted or unsubstituted $C_1$-$C_4$alkyl-SO-; or two $R^4$ on adjacent ring atoms together with the carbon atoms to which they are attached form a substituted or unsubstituted 5-9 membered carbocycle or heterocycle, and the ring is partially unsaturated or saturated.

**5.** The compound according to claim 1, or a pharmaceutically acceptable salt thereof, or a deuterated compound thereof, wherein $R^3$ is a group selected from the group consisting of : the ring

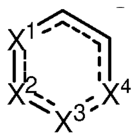

is selected from the group consisting of:

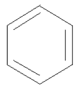, 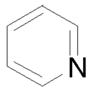, 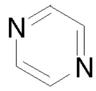, 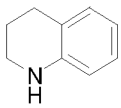,

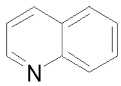, 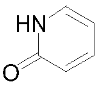, 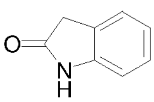, 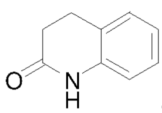, 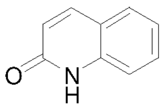, 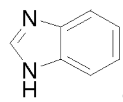, 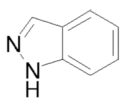, 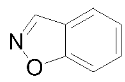,

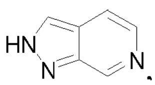, 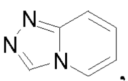, 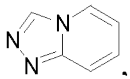, 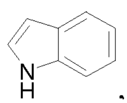, 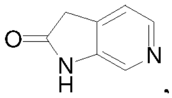, 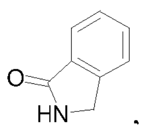, 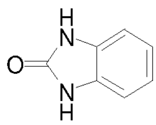, 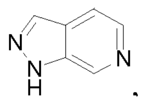,

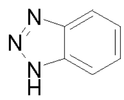,

and

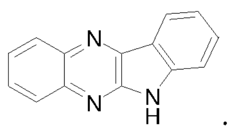.

**6.** The compound according to claim 1, or a pharmaceutically acceptable salt thereof, or a deuterated compound thereof, wherein ring B is selected from the group consisting of substituted or unsubstituted $C_6$-$C_{10}$aryl, substituted or unsubstituted 5-12 membered heteroaryl, substituted or unsubstituted 4-12 membered heterocycle containing 1-3 heteroatoms selected from oxygen, sulfur and nitrogen, and substituted or unsubstituted $C_3$-$C_{12}$carbocycle.

**7.** The compound according to claim 1, or a pharmaceutically acceptable salt thereof, or a deuterated compound thereof, wherein ring A is selected from the group consisting of:

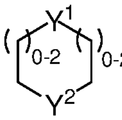,

wherein, $Y^1$ and $Y^2$ are each independently selected from the group consisting of: $CHR^6$ and $NR^6$; 0-2 means that the number of carbon atoms can be 0, 1 or 2; and the L-B structure can be located at $Y^1$ , $Y^2$ or other ring atoms (preferably at $Y^1$ , $Y^2$);
or ring A together with two $R^4$ form a group selected from the group consisting of:

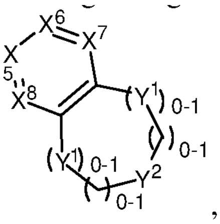,

wherein, $Y^1$ and $Y^2$ are each independently selected from the group consisting of: $CHR^6$, O and $NR^6$; $X^5$, $X^6$, $X^7$ and $X^8$ are each independently selected from the group consisting of: $CR^6$ and N.

**8.** The compound according to claim 1, or a pharmaceutically acceptable salt thereof, or a deuterated compound thereof, wherein $R^3$ is a group selected from the group consisting of: ring A is a substituted or unsubstitutedgroup selected from the group consisting of:

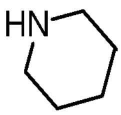, 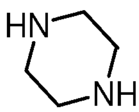, 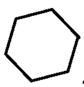, 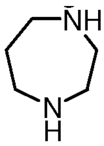, 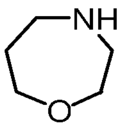

and

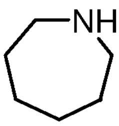

(wherein, when the connection site connected to other structural fragments or substituents is NH, the hydrogen atom on NH is lost to form the connection site);
or ring A together with two $R^4$ form a substituted or unsubstituted group selected from the group consisting of:

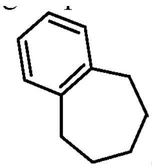, 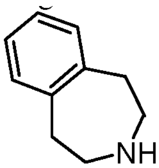, 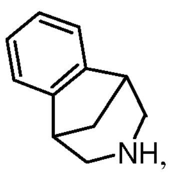, 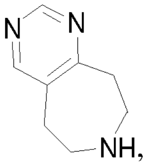, 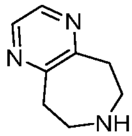, 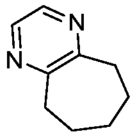, 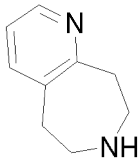,

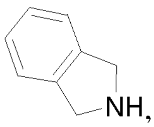, 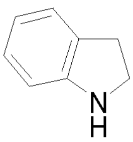, 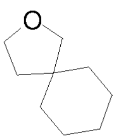, 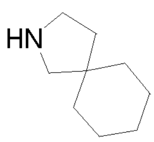, 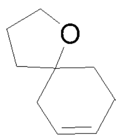, 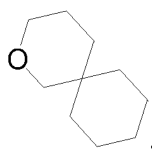, 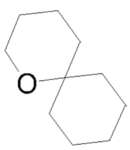, 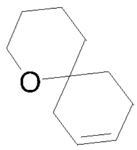,

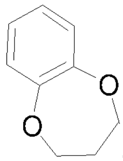, 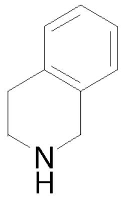, 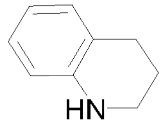

and

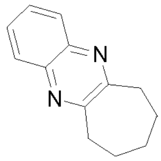

(wherein, when the connection site with other structural fragments or substituents is NH, the hydrogen atom on NH is lost to form the connection site).

**9.** The compound according to claim 1, or a pharmaceutically acceptable salt thereof, or a deuterated compound thereof, wherein the compound has a structure as shown in the following formula II:

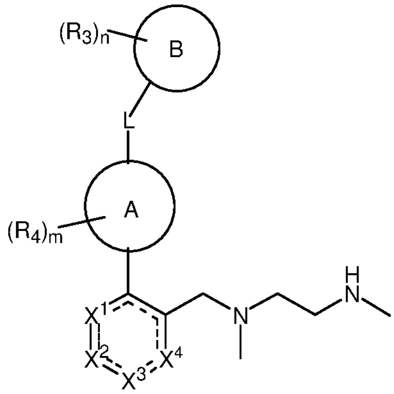

II

.

**10.** The compound according to claim 1, or a pharmaceutically acceptable salt thereof, or a deuterated compound thereof, wherein the compound has a structure as shown in the following formula III:

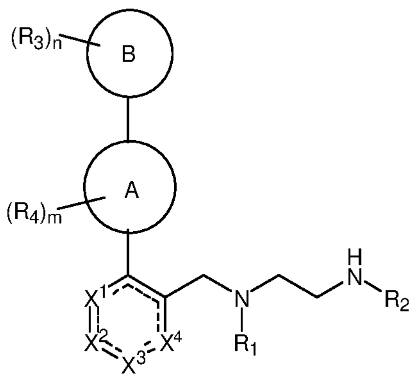

III

wherein,

ring A is selected from the group consisting of substituted or unsubstituted saturated or partially unsaturated (non-aromatic) 4-8 membered carbocycle, and substituted or unsubstituted saturated or partially unsaturated (non-aromatic) 4-8 membered heterocycle;

ring B is selected from the group consisting of substituted or unsubstituted $C_6$-$C_{10}$aryl, substituted or unsubstituted 5-12 membered heteroaryl, substituted or unsubstituted 3-12 membered heterocycle containing 1-3 heteroatoms selected from oxygen, sulfur and nitrogen, and substituted or unsubstituted $C_3$-$C_{12}$heterocycle;

preferably, $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_1$-$C_4$alkyl-$S(O)_2$-, and substituted or unsubstituted $C_1$-$C_4$alkyl-SO-;

preferably, $R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-7 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_1$-$C_4$alkyl-$S(O)_2$-, and substituted or unsubstituted $C_1$-$C_4$alkyl-SO-.

**11.** The compound according to claim 1, or a pharmaceutically acceptable salt thereof, or a deuterated compound thereof, wherein the compound has a structure as shown in the following formula IV:

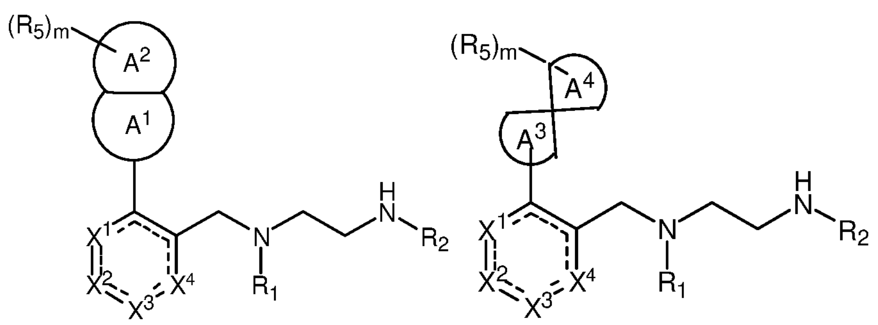

IV IVA

wherein,

ring $A^1$ is selected from the group consisting of substituted or unsubstituted saturated or partially unsaturated (non-aromatic) 5-8 membered carbocycle, and substituted or unsubstituted saturated or partially unsaturated (non-aromatic) 5-8 membered heterocycle;

ring $A^2$ is selected from the group consisting of substituted or unsubstituted $C_6$-$C_{10}$aryl, substituted or unsubstituted 5-12 membered heteroaryl, substituted or unsubstituted 5-7 membered heterocycle containing 1-3 heteroatoms selected from oxygen, sulfur and nitrogen, and substituted or unsubstituted $C_3$-$C_{12}$carbocycle; and ring $A^2$is fused with ring $A^1$;

ring $A^3$and ring $A^4$are each independently selected from the group consisting of substituted or unsubstituted saturated or partially unsaturated (non-aromatic) 5-8 membered carbocycle, and substituted or unsubstituted saturated or partially unsaturated (non-aromatic) 3-8 membered heterocycle;

m is 0, 1, 2, 3 or 4; $R^5$is selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_6$-$C_{10}$aryloxy or heteroaryloxy, substituted or unsubstituted $C_6$-$C_{10}$arylamino or heteroarylamino, substituted or unsubstituted $C_1$-$C_6$ amide, substituted or unsubstituted -$C_1$-$C_6$alkyl-phenyl, substituted or unsubstituted $C_1$-$C_4$alkyl-$S(O)_2$-, and substituted or unsubstituted $C_1$-$C_4$alkyl-SO-; and $R^5$ can be located on ring $A^1$, ring $A^2$, ring $A^3$or ring $A^4$.

**12.** The compound according to claim 1, or a pharmaceutically acceptable salt thereof, or a deuterated compound thereof, wherein the compound has a structure as shown in the following formula V or VI:

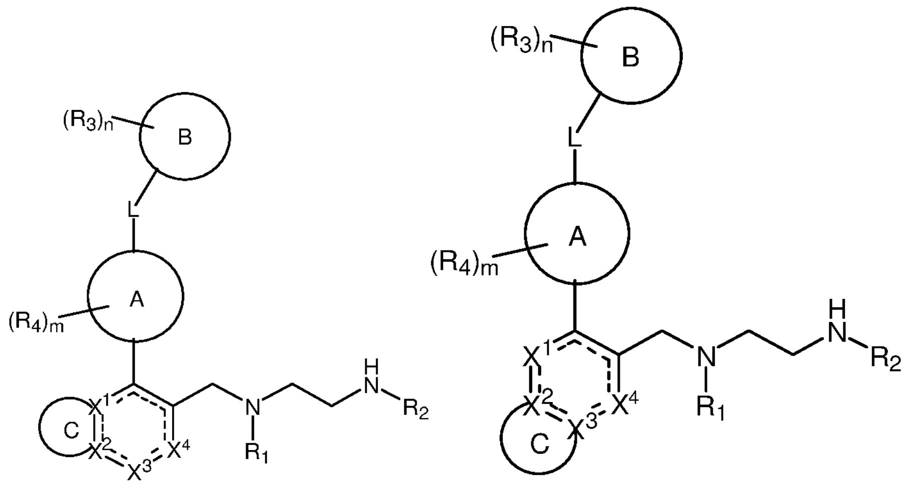

V VI

wherein, ring C is substituted or unsubstituted phenyl, substituted or unsubstituted 5-7 membered heterocyclyl, or substituted or unsubstituted 5-6 membered heteroaryl;
ring A is selected from the group consisting of substituted or unsubstituted saturated or partially unsaturated (non-aromatic) 5-6 membered carbocycle, and substituted or unsubstituted saturated or partially unsaturated (non-aromatic) 4-8 membered heterocycle;
ring B is selected from the group consisting of substituted or unsubstituted $C_6$-$C_{10}$aryl, substituted or unsubstituted 5-12 membered heteroaryl, substituted or unsubstituted 4-12 membered heterocycle containing 1-3 heteroatoms selected from oxygen, sulfur and nitrogen, and substituted or unsubstituted $C_3$-$C_{12}$carbocycle;
preferably, $R^3$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_2$-$C_6$acyl, substituted or unsubstituted $C_2$-$C_6$ester group, substituted or unsubstituted $C_1$-$C_4$alkyl-S(O)$_2$-, and substituted or unsubstituted $C_1$-$C_4$alkyl-SO-;
preferably, $R^4$ is a group selected from the group consisting of H, halogen, cyano, amino, nitro, hydroxyl, thiol, aldehyde group, carboxyl, sulfonyl, substituted or unsubstituted $C_1$-$C_6$alkyl, substituted or unsubstituted $C_1$-$C_6$alkoxy, substituted or unsubstituted $C_1$-$C_6$alkylamino, substituted or unsubstituted $C_6$-$C_{10}$aryl, substituted or unsubstituted 5-7 membered heteroaryl, substituted or unsubstituted 5-7 membered heterocycle containing 1-3 heteroatoms selected from oxygen, sulfur and nitrogen, substituted or unsubstituted -$C_1$-$C_6$alkyl-phenyl, substituted or unsubstituted $C_3$-$C_8$carbocycle, substituted or unsubstituted $C_2$-$C_6$acyl, substituted or unsubstituted $C_2$-$C_6$ester group, substituted or unsubstituted $C_1$-$C_4$alkyl-S(O)$_2$-, and substituted or unsubstituted $C_1$-$C_4$alkyl-SO-.

**13.** The compound according to claim 1, or a pharmaceutically acceptable salt thereof, or a deuterated compound thereof, wherein the compound is any one of the compound of Example P1-P448.

**14.** A pharmaceutical composition, comprising a therapeutically effective amount of the compound of formula I according to claim 1, or a pharmaceutically acceptable salt thereof, and one or morepharmaceutically acceptable carriers, excipients, adjuvants, accessories and/or diluents.

**15.** A use of a compound of formula I according to claim 1, or a pharmaceutically acceptable salt thereof, in the preparation of a pharmaceutical composition for the treatment or prevention of diseases associated with PRMT; preferably the PRMT is type I PRMT.

**INTERNATIONAL SEARCH REPORT**

International application No.
**PCT/CN2022/093688**

**A. CLASSIFICATION OF SUBJECT MATTER**

C07C 211/29(2006.01)i; A61K 31/44(2006.01)i; A61P 35/00(2006.01)i

According to International Patent Classification (IPC) or to both national classification and IPC

**B. FIELDS SEARCHED**

Minimum documentation searched (classification system followed by classification symbols)

C07C; A61K; A61P

Documentation searched other than minimum documentation to the extent that such documents are included in the fields searched

Electronic data base consulted during the international search (name of data base and, where practicable, search terms used)

CNABS: CNTXT; ENTXT; ENTXTC; VEN; CNKI: 精氨酸甲基转移酶, PRMTS, structural formula search

**C. DOCUMENTS CONSIDERED TO BE RELEVANT**

| Category* | Citation of document, with indication, where appropriate, of the relevant passages | Relevant to claim No. |
|---|---|---|
| A | US 2008280925 A1 (METHYLGENE INC.) 13 November 2008 (2008-11-13) entire document | 1-15 |
| X | US 2005192286 A1 (NEUROCRINE BIOSCIENCES INC.) 01 September 2005 (2005-09-01) entire document, specifically description, table 6, and paragraphs [0100]-[0101] | 1-8, 14 |
| X | WO 2016044585 A1 (EPIZYME INC.) 24 March 2016 (2016-03-24) entire document, specifically claims 1-393 | 1-15 |
| X | CN 112533904 A (CRINETICS PHARMACEUTICALS INC.) 19 March 2021 (2021-03-19) entire document, specifically description, table 2, and paragraphs [0177]-[0178] | 1-8, 14 |
| X | JOE, A. T. et al. "Pyrrolidines as Potent Functional Agonists of the Human Melanocortin-4 Receptor" *Bioorganic & Medicinal Chemistry Letters,* 04 July 2007 (2007-07-04), page 5168, table 1 | 1-8, 14 |
| X | "STN Search Results" *STN REGISTRY database,* 06 January 2020 (2020-01-06), | 1-5, 7, 9 |
| X | CN 110845474 A (SICHUAN UNIVERSITY) 28 February 2020 (2020-02-28) entire document, specifically claims 1-10 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 September 2022** | **23 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/093688**

**C. DOCUMENTS CONSIDERED TO BE RELEVANT**

| Category* | Citation of document, with indication, where appropriate, of the relevant passages | Relevant to claim No. |
|---|---|---|
| A | CN 108947879 A (SHANGHAI INSTITUTE OF MATERIA MEDICA, CHINESE ACADEMY OF SCIENCES) 07 December 2018 (2018-12-07)<br>entire document | 1-15 |
| A | US 2004053933 A1 (NEUROCRINE BIOSCIENCES INC.) 18 March 2004 (2004-03-18)<br>entire document | 1-15 |
| A | US 2014323537 A1 (EPIZYME INC.) 30 October 2014 (2014-10-30)<br>entire document | 1-15 |
| A | US 2017280720 A1 (EPIZYME INC.) 05 October 2017 (2017-10-05)<br>entire document | 1-15 |

Form PCT/ISA/210 (second sheet) (January 2015)

**INTERNATIONAL SEARCH REPORT**
**Information on patent family members**

International application No.
**PCT/CN2022/093688**

| Patent document cited in search report | Publication date (day/month/year) | Patent family member(s) | Publication date (day/month/year) |
|---|---|---|---|
| US 2008280925 A1 | 13 November 2008 | WO 2008104077 A1 | 04 September 2008 |
| | | EP 2137158 A1 | 30 December 2009 |
| US 2005192286 A1 | 01 September 2005 | WO 2005040109 A1 | 06 May 2005 |
| WO 2016044585 A1 | 24 March 2016 | US 2017280720 A1 | 05 October 2017 |
| CN 112533904 A | 19 March 2021 | EP 3802500 A1 | 14 April 2021 |
| | | AU 2019280689 A1 | 21 January 2021 |
| | | US 2021238164 A1 | 05 August 2021 |
| | | US 2021002254 A1 | 07 January 2021 |
| | | US 2020010452 A1 | 09 January 2020 |
| | | CA 3101644 A1 | 12 December 2019 |
| | | US 2019367481 A1 | 05 December 2019 |
| | | JP 2021528375 A | 21 October 2021 |
| | | TW 202016089 A | 01 May 2020 |
| | | IL 279152 A | 31 January 2021 |
| | | WO 2019236699 A1 | 12 December 2019 |
| | | US 2020216415 A1 | 09 July 2020 |
| | | MA 52806 A | 14 April 2021 |
| | | BR 112020024577 A2 | 09 March 2021 |
| | | SG 11202012071 T A | 28 January 2021 |
| | | KR 20210005995 A | 15 January 2021 |
| CN 110845474 A | 28 February 2020 | WO 2021088885 A1 | 14 May 2021 |
| CN 108947879 A | 07 December 2018 | None | |
| US 2004053933 A1 | 18 March 2004 | EP 1503761 A1 | 09 February 2005 |
| | | JP 2005534632 A | 17 November 2005 |
| | | CA 2484968 A1 | 20 November 2003 |
| | | WO 03094918 A1 | 20 November 2003 |
| | | MX PA04011093 A | 14 February 2005 |
| | | AU 2003230367 A1 | 11 November 2003 |
| US 2014323537 A1 | 30 October 2014 | US 2017057926 A1 | 02 March 2017 |
| | | CA 2903264 A1 | 06 November 2014 |
| | | AU 2014260433 A1 | 10 September 2015 |
| | | JP 2016514164 A | 19 May 2016 |
| | | WO 2014178954 A1 | 06 November 2014 |
| | | US 2018237397 A1 | 23 August 2018 |
| | | EP 2970137 A1 | 20 January 2016 |
| | | US 2016137609 A1 | 19 May 2016 |
| US 2017280720 A1 | 05 October 2017 | WO 2016044585 A1 | 24 March 2016 |

Form PCT/ISA/210 (patent family annex) (January 2015)